# EUROPEAN PATENT SPECIFICATION

(11) **EP 4 247 800 B1**
(45) Date of publication and mention of the grant of the patent: **13.11.2024**
(21) Application number: 21815188.4
(22) Date of filing: 19.11.2021
(51) Int. Cl.: C07D 263/57, C07D 413/12, C07D 498/04, A61K 31/423, A61P 31/12

(54) **N-SUBSTITUTED 4-(1,3-ARYLOXAZOLO-2-YL)PHENYL COMPOUNDS FOR THE TREATMENT AND PROPHYLAXIS OF HEPATITIS B VIRUS INFECTION**
N-SUBSTITUIERTE 4-(1,3-ARYLOXAZOLO-2-YL)PHENYL-VERBINDUNGEN ZUR BEHANDLUNG UND PROPHYLAXE VON HEPATITIS-B-VIRUSINFEKTIONEN
N-COMPOSÉS DE 4-(1,3-ARYLOXAZOLO-2-YL)PHÉNYLE-SUBSTITUÉS POUR LE TRAITEMENT ET LA PROPHYLAXIE D'UNE INFECTION PAR LE VIRUS DE L'HÉPATITE B

(30) Priority: 20.11.2020 WO PCT/CN2020/130578
(43) Date of publication of application: 27.09.2023
(73) Proprietor: F. Hoffmann-La Roche AG, 4070 Basel (CH)
(72) Inventor: LIN, Xianfeng, Shanghai 201203 (CN); YUN, Hongying, Shanghai 201203 (CN); ZHANG, Bo, Shanghai 201203 (CN); ZHENG, Xiufang, Shanghai 201203 (CN)
(74) Representative: Schirlin, Julien
(86) International application number: PCT/EP2021/082237
(87) International publication number: WO 2022/106589

(56) References cited:
- WO-A1-2006/122546
- WO-A1-2020/043880
- WO-A1-2020/077024
- WO-A1-96/26932
- JP-A- 2018 087 173
- CHEE WEE PHOON ET AL: "Biological evaluation of hepatitis c virus helicase inhibitors", BIOORGANIC & MEDICINAL CHEMISTRY LETTERS, 1 January 2001 (2001-01-01), England, pages 1647 - 1650, XP055086365, Retrieved from the Internet <URL:http://www.ncbi.nlm.nih.gov/pubmed/11425528> DOI: 10.1016/S0960-894X(01)00263-3

## Description

The present invention relates to organic compounds useful for therapy and/or prophylaxis of HBV infection in a mammal, and in particular to HBsAg (HBV Surface antigen) and HBeAg (HBV e antigen) inhibitors useful for treating HBV infection.

### FIELD OF THE INVENTION

The present invention relates to N-substituted 4-(1,3-aryloxazolo-2-yl)phenyl compounds and their corresponding derivatives that have anti-virus activity, as well as their manufacture, pharmaceutical compositions containing them and their potential use as medicaments.

The present invention relates to compounds of formula (I), 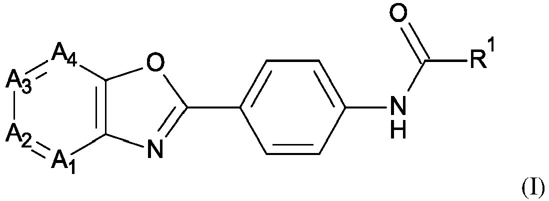 wherein A₁ to A₄ and R¹ are as described below, or a pharmaceutically acceptable salt thereof.

Hepatitis B virus (HBV) is one of the most dangerous human pathogens. A safe and effective vaccine has been available for longer than two decades; however, WHO estimated that approximately 257 million people are chronically infected with HBV. Chronic Hepatitis B (CHB) infection predisposes its host to severe liver disease, including liver cirrhosis and hepatocellular carcinoma, if left untreated. HBV infection is ranked among the top unmet medical need worldwide. The currently approved drugs have contributed to substantial progress in CHB treatment; however, the cure rate remains less than 10%.

The control of viral infection needs an effective immune surveillance. Upon recognition of viral infection, the host innate immune system could respond within minutes to impede viral replication and limits the development of a chronic and persistent infection. The secretion of antiviral cytokines from infected hepatocytes and intra-hepatic immune cells is critically important for the clearance of viral infection. However, chronically infected patients only display a weak immune response due to various escape strategies adopted by the virus to counteract the host cell recognition systems and the subsequent antiviral responses.

Many observations showed that several HBV viral proteins could counteract the initial host cellular response by interfering with the viral recognition signaling system and subsequently the interferon (IFN) antiviral activity. Among these, the excessive secretion of HBV empty subviral particles (SVPs, HBsAg) may contribute to immune tolerant state observed in CHB patients. The persistent exposure to HBsAg and other viral antigens can lead to HBV-specific T-cell functional impairment and depletion (Kondo et al. Journal of Immunology (1993), 150, 4659-4671; Kondo et al. Journal of Medical Virology (2004), 74, 425-433; Fisicaro et al. Gastroenterology, (2010), 138, 682-693;). Moreover, HBsAg has been reported to suppress immune cell functions, including monocytes, dendritic cells (DCs) and natural killer (NK) cells (Op den Brouw et al. Immunology, (2009b), 126, 280-289; Woltman et al. PLoS One, (2011), 6, e15324; Shi et al. J Viral Hepat. (2012), 19, e26-33; Kondo et al. ISRN Gasteroenterology, (2013), Article ID 935295).

HBsAg is an important biomarker for prognosis and treatment response in CHB. However, the achievement of HBsAg loss and seroconversion is rarely achieved in CHB patients. HBsAg loss with or without anti-HBsAg seroconversion remains the ideal clinical treatment endpoints. Current therapies, such as nucleos(t)ide analogues, are effective in supressing HBV DNA, but are not effective in reducing HBsAg level. Nucleos(t)ide analogs, even with prolonged therapy, have demonstrated HBsAg clearance rates comparable to those observed naturally (Janssen et al. Lancet, (2005), 365, 123-129; Marcellin et al. N. Engl. J. Med., (2004), 351, 1206-1217; Buster et al. Hepatology, (2007), 46, 388-394). Therefore, there is an urgent need for the development of novel therapeutic agents that could efficiently reduce HBsAg. (Wieland, S. F. & F. V. Chisari. J Virol, (2005), 79, 9369-9380; Kumar et al. J Virol, (2011), 85, 987-995; Woltman et al. PLoS One, (2011), 6, e15324; Op den Brouw et al. Immunology, (2009b), 126, 280-289).

WO2006/122546 and Phoon et al., Bioorg. Med. Chem. Lett, 2001, 1647-1650 disclose structurally related compounds.

### SUMMARY OF THE INVENTION

Objects of the present invention are novel compounds of formula (I), their manufacture, medicaments based on a compound in accordance with the invention and their production as well as the use of compounds of formula (I) as HBV inhibitors and for the treatment or prophylaxis of HBV infection. The compounds of formula (I) show superior anti-HBV activity. In addition, the compounds of formula (I) also show good safety and good PK profiles.

The present invention relates to a compound of formula (I) 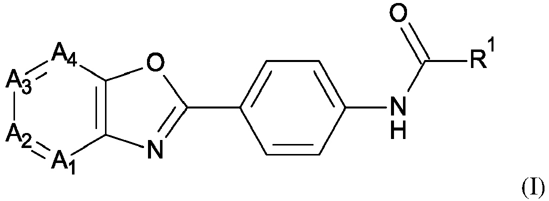 wherein
R¹ is tetrahydrofuran-3-yl, 1-hydroxyethyl, tert-butyl, tetrahydrothiopyran-4-yl, 2-methyltetrahydrofuran-2-yl, propyl, 1,1-dioxothiolan-3-yl, 3-furyl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothian-4-yl, cyclopropyl, 3-methyltetrahydrofuran-3-yl, 8-oxabicyclo[3.2.1]octan-3-yl, 1,4-dioxan-2-yl, cyclobutyl, 4-acetoxytetrahydropyran-4-yl, 2,2-difluorocyclopropyl, tetrahydropyran-4-yl, oxetan-3-yl, 1,1-dioxothietan-3-yl, 1-methylcyclopropyl, 3-oxabicyclo[3.1.0]hexan-6-yl, 1-methylcyclobutyl, 1-acetoxycyclopropyl, tetrahydrofuran-3-yloxy, 3-methoxycarbonyl-1-bicyclo[1.1.1]pentanyl, 3-ethyloxetan-3-yl, oxazol-2-yl, 5-methyloxazol-2-yl, 2-pyridyl, 4-methyloxazol-2-yl, thiazol-2-yl, 3-pyridyl, 3-methyloxetan-3-yl, 1-methyl-3-oxo-cyclobutyl, methoxy, ethoxy, isopropoxy, 1-methoxyethyl or 5-methyl-1,3,4-oxadiazol-2-yl;
A₁ is N or CR²; wherein R² is H or halogen; wherein with the proviso that when R¹ is 3-pyridyl, A₁ is CF;
A₂ is N or CR³; wherein R³ is H or halogen;
A₃ is N or CR⁴; wherein R⁴ is H, halogen, C₁₋₆alkyl, or C₁₋₆alkoxy;
A₄ is N or CR⁵; wherein R⁵ is H or halogen; wherein with the proviso that when R⁴ is C₁₋₆alkyl or C₁₋₆alkoxy, A₄ is N;
wherein with the proviso that A₁, A₂, A₃ and A₄ are not CH simultaneously;
or a pharmaceutically acceptable salt thereof.

### DETAILED DESCRIPTION OF THE INVENTION

### DEFINITIONS

As used herein, the term "C₁₋₆alkyl" alone or in combination signifies a saturated, linear- or branched chain alkyl group containing 1 to 6, particularly 2 to 6 or 1 to 4 carbon atoms, for example methyl, ethyl, propyl, isopropyl, butyl, isobutyl, *tert*-butyl and the like. Particular "C₁₋₆alkyl" groups are methyl, ethyl, propyl, isopropyl, isobutyl and *tert*-butyl.

The term "C₁₋₆alkoxy" alone or in combination signifies a group C₁₋₆alkyl-O-, wherein the "C₁₋₆alkyl" is as defined above; for example methoxy, ethoxy, propoxy, *iso*-propoxy, *n*-butoxy, *iso*-butoxy, 2-butoxy, *tert*-butoxy, pentoxy, hexyloxy and the like. Particular "C₁₋₆alkoxy" groups are methoxy, ethoxy, and propoxy.

The term "halogen" denotes fluoro, chloro, bromo, or iodo.

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of formula (I) and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as *p*-toluenesulfonic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et al., Organic Process Research & Development 2000, 4, 427-435. Particular are the sodium salts of the compounds of formula (I).

### HBV INHIBITORS

The present invention provides (i) a compound having the general formula (I): 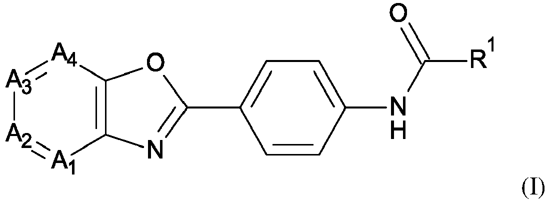 wherein
- R¹: is tetrahydrofuran-3-yl, 1-hydroxyethyl, tert-butyl, tetrahydrothiopyran-4-yl, 2-methyltetrahydrofuran-2-yl, propyl, 1,1-dioxothiolan-3-yl, 3-furyl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothian-4-yl, cyclopropyl, 3-methyltetrahydrofuran-3-yl, 8-oxabicyclo[3.2.1]octan-3-yl, 1,4-dioxan-2-yl, cyclobutyl, 4-acetoxytetrahydropyran-4-yl, 2,2-difluorocyclopropyl, tetrahydropyran-4-yl, oxetan-3-yl, 1,1-dioxothietan-3-yl, 1-methylcyclopropyl, 3-oxabicyclo[3.1.0]hexan-6-yl, 1-methylcyclobutyl, 1-acetoxycyclopropyl, tetrahydrofuran-3-yloxy, 3-methoxycarbonyl-1-bicyclo[1.1.1]pentanyl, 3-ethyloxetan-3-yl, oxazol-2-yl, 5-methyloxazol-2-yl, 2-pyridyl, 4-methyloxazol-2-yl, thiazol-2-yl, 3-pyridyl, 3-methyloxetan-3-yl, 1-methyl-3-oxo-cyclobutyl, methoxy, ethoxy, isopropoxy, 1-methoxyethyl or 5-methyl-1,3,4-oxadiazol-2-yl;
- A₁: is N or CR²; wherein R² is H or halogen; wherein with the proviso that when R¹ is 3-pyridyl, A₁ is CF;
- A₂: is N or CR³; wherein R³ is H or halogen;
- A₃: is N or CR⁴; wherein R⁴ is H, halogen, C₁₋₆alkyl, or C₁₋₆alkoxy;
- A₄: is N or CR⁵; wherein R⁵ is H or halogen; wherein with the proviso that when R⁴ is C₁₋₆alkyl or C₁₋₆alkoxy, A₄ is N;

wherein with the proviso that A₁, A₂, A₃ and A₄ are not CH simultaneously;
or a pharmaceutically acceptable salt thereof.

A further embodiment of the present invention is (ii) a compound of formula (I) according to (i), wherein
- R¹: is tetrahydrofuran-3-yl, 1-hydroxyethyl, tert-butyl, tetrahydrothiopyran-4-yl, 2-methyltetrahydrofuran-2-yl, propyl, 1,1-dioxothiolan-3-yl, 3-furyl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothian-4-yl, cyclopropyl, 3-methyltetrahydrofuran-3-yl, 8-oxabicyclo[3.2.1]octan-3-yl, 1,4-dioxan-2-yl, cyclobutyl, 4-acetoxytetrahydropyran-4-yl, 2,2-difluorocyclopropyl, tetrahydropyran-4-yl, oxetan-3-yl, 1,1-dioxothietan-3-yl, 1-methylcyclopropyl, 3-oxabicyclo[3.1.0]hexan-6-yl, 1-methylcyclobutyl, 1-acetoxycyclopropyl, tetrahydrofuran-3-yloxy, 3-methoxycarbonyl-1-bicyclo[1.1.1]pentanyl, 3-ethyloxetan-3-yl, oxazol-2-yl, 5-methyloxazol-2-yl, 2-pyridyl, 4-methyloxazol-2-yl, thiazol-2-yl, 3-pyridyl, 3-methyloxetan-3-yl, 1-methyl-3-oxo-cyclobutyl, methoxy, ethoxy, isopropoxy, 1-methoxyethyl or 5-methyl-1,3,4-oxadiazol-2-yl;
- A₁: is CR²; wherein R² is H or F; wherein with the proviso that when R¹ is 3-pyridyl, A₁ is CF;
- A₂: is CR³; wherein R³ is H, For Cl;
- A₃: is CR⁴; wherein R⁴ is H, F, Cl, methyl or methoxy;
- A₄: is N or CR⁵; wherein R⁵ is H, For Cl; wherein with the proviso that when R⁴ is methyl or methoxy, A₄ is N;

wherein with the proviso that A₁, A₂, A₃ and A₄ are not CH simultaneously;
or a pharmaceutically acceptable salt thereof.

A further embodiment of the present invention is (iii) a compound of formula (I) according to (i), or a pharmaceutically acceptable salt thereof, wherein A₂ is CH.

A further embodiment of the present invention is (iv) a compound of formula (I) according to (i), or a pharmaceutically acceptable salt thereof, wherein A₃ is CR⁴; wherein R⁴ is H or halogen.

A further embodiment of the present invention is (v) a compound of formula (I) according to (i), or a pharmaceutically acceptable salt thereof, wherein A₃ is CR⁴; wherein R⁴ is H, For Cl.

A further embodiment of the present invention is (vi) a compound of formula (I) according to (i), or a pharmaceutically acceptable salt thereof, wherein A₄ is CR⁵; wherein R⁵ is H or halogen.

A further embodiment of the present invention is (vii) a compound of formula (I) according to (i), or a pharmaceutically acceptable salt thereof, wherein A₄ is CR⁵; wherein R⁵ is H or F.

A further embodiment of the present invention is (viii) a compound of formula (I) according to (i), or a pharmaceutically acceptable salt thereof, wherein R¹ is tetrahydrofuran-3-yl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothiolan-3-yl, 1,1-dioxothian-4-yl, tert-butyl, cyclopropyl, 3-methyltetrahydrofuran-3-yl, 8-oxabicyclo[3.2.1]octan-3-yl, 1,4-dioxan-2-yl, 1-hydroxyethyl, cyclobutyl, tetrahydropyran-4-yl, oxazol-2-yl, 5-methyloxazol-2-yl, 2-pyridyl or 4-methyloxazol-2-yl.

A further embodiment of the present invention is (ix) a compound of formula (I) according to (i), or a pharmaceutically acceptable salt thereof, wherein
- R¹: is tetrahydrofuran-3-yl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothiolan-3-yl, 1,1-dioxothian-4-yl, tert-butyl, cyclopropyl, 3-methyltetrahydrofuran-3-yl, 8-oxabicyclo[3.2.1]octan-3-yl, 1,4-dioxan-2-yl, 1-hydroxyethyl, cyclobutyl, tetrahydropyran-4-yl, oxazol-2-yl, 5-methyloxazol-2-yl, 2-pyridyl or 4-methyloxazol-2-yl;
- A₁: is CR²; wherein R² is H or halogen;
- A₂: is CH;
- A₃: is CR⁴; wherein R⁴ is H or halogen;
- A₄: is CR⁵; wherein R⁵ is H or halogen;
wherein with the proviso that A₁, A₂, A₃ and A₄ are not CH simultaneously.

A further embodiment of the present invention is (x) a compound of formula (I) according to (i), or a pharmaceutically acceptable salt thereof, wherein
- R¹: is tetrahydrofuran-3-yl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothiolan-3-yl, 1,1-dioxothian-4-yl, tert-butyl, cyclopropyl, 3-methyltetrahydrofuran-3-yl, 8-oxabicyclo[3.2.1]octan-3-yl, 1,4-dioxan-2-yl, 1-hydroxyethyl, cyclobutyl, tetrahydropyran-4-yl, oxazol-2-yl, 5-methyloxazol-2-yl, 2-pyridyl or 4-methyloxazol-2-yl;
- A₁: is CH or CF;
- A₂: is CH;
- A₃: is CH, CF or CCl;
- A₄: is CH or CF;
wherein with the proviso that A₁, A₂, A₃ and A₄ are not CH simultaneously.

In another embodiment (xi) of the present invention, particular compounds of the present invention are selected from:
N-[4-(6-fluoro-1,3-benzoxazol-2-yl)phenyl]tetrahydrofuran-3-carboxamide;
N-[4-(6-fluoro-1,3-benzoxazol-2-yl)phenyl]-2-hydroxy-propanamide;
N-[4-(6-fluoro-1,3-benzoxazol-2-yl)phenyl]-2,2-dimethyl-propanamide;
N-[4-(6-fluoro-1,3-benzoxazol-2-yl)phenyl]tetrahydrothiopyran-4-carboxamide;
N-[4-(6-fluoro-1,3-benzoxazol-2-yl)phenyl]-2-methyl-tetrahydrofuran-2-carboxamide;
N-[4-(5-fluoro-1,3-benzoxazol-2-yl)phenyl]-2,2-dimethyl-propanamide;
N-[4-(5-fluoro-1,3-benzoxazol-2-yl)phenyl]tetrahydrothiopyran-4-carboxamide;
N-[4-(6-chloro-1,3-benzoxazol-2-yl)phenyl]tetrahydrofuran-3-carboxamide;
N-[4-(6-chloro-1,3-benzoxazol-2-yl)phenyl]butanamide;
N-[4-(6-chloro-1,3-benzoxazol-2-yl)phenyl]-1,1-dioxo-thiolane-3-carboxamide;
N-[4-(7-fluoro-1,3-benzoxazol-2-yl)phenyl]furan-3-carboxamide;
N-[4-(7-fluoro-1,3-benzoxazol-2-yl)phenyl]-3-methyl-oxetane-3-carboxamide;
N-[4-(7-fluoro-1,3-benzoxazol-2-yl)phenyl]-2-hydroxy-2-methyl-propanamide;
N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]tetrahydrofuran-3-carboxamide;
3-fluoro-N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]-1,1-dioxo-thiolane-3-carboxamide;
N-[4-(7-fluoro-1,3-benzoxazol-2-yl)phenyl]-1,1-dioxo-thiane-4-carboxamide;
N-[4-(7-fluoro-1,3-benzoxazol-2-yl)phenyl]-2,2-dimethyl-propanamide;
N-[4-(7-fluoro-1,3-benzoxazol-2-yl)phenyl]cyclopropanecarboxamide;
N-[4-(7-fluoro-1,3-benzoxazol-2-yl)phenyl]-3-methyl-tetrahydrofuran-3-carboxamide;
N-[4-(7-fluoro-1,3-benzoxazol-2-yl)phenyl]-8-oxabicyclo[3.2.1]octane-3-carboxamide;
N-[4-(7-fluoro-1,3-benzoxazol-2-yl)phenyl]-1,4-dioxane-2-carboxamide;
N-[4-(7-fluoro-1,3-benzoxazol-2-yl)phenyl]-2-hydroxy-propanamide;
N-[4-(7-fluoro-1,3-benzoxazol-2-yl)phenyl]cyclobutanecarboxamide;
[4-[[4-(7-fluoro-1,3-benzoxazol-2-yl)phenyl]carbamoyl]tetrahydropyran-4-yl] acetate;
2,2-difluoro-N-[4-(7-fluoro-1,3-benzoxazol-2-yl)phenyl]cyclopropanecarboxamide;
N-[4-(7-fluoro-1,3-benzoxazol-2-yl)phenyl]tetrahydropyran-4-carboxamide;
N-[4-(7-fluoro-1,3-benzoxazol-2-yl)phenyl]oxetane-3-carboxamide;
N-[4-(7-fluoro-1,3-benzoxazol-2-yl)phenyl]tetrahydrothiopyran-4-carboxamide;
N-[4-(7-fluoro-1,3-benzoxazol-2-yl)phenyl]-1,1-dioxo-thietane-3-carboxamide;
N-[4-(7-fluoro-1,3-benzoxazol-2-yl)phenyl]-1-methyl-cyclopropanecarboxamide;
N-[4-(7-fluoro-1,3-benzoxazol-2-yl)phenyl]-3-oxabicyclo[3.1.0]hexane-6-carboxamide;
N-[4-(7-fluoro-1,3-benzoxazol-2-yl)phenyl]-1-methyl-cyclobutanecarboxamide;
N-[4-(7-fluoro-1,3-benzoxazol-2-yl)phenyl]-2-methyl-tetrahydrofuran-2-carboxamide;
[1-[[4-(7-fluoro-1,3-benzoxazol-2-yl)phenyl]carbamoyl]cyclopropyl] acetate;
tetrahydrofuran-3-yl N-[4-(7-fluoro-1,3-benzoxazol-2-yl)phenyl]carbamate;
methyl 3-[[4-(7-fluoro-1,3-benzoxazol-2-yl)phenyl]carbamoyl]bicyclo[1.1.1]pentane-1-carboxylate;
3-ethyl-N-[4-(7-fluoro-1,3-benzoxazol-2-yl)phenyl]oxetane-3-carboxamide;
N-[4-(4-fluoro-1,3-benzoxazol-2-yl)phenyl]oxazole-2-carboxamide;
N-[4-(4-fluoro-1,3-benzoxazol-2-yl)phenyl]-5-methyl-oxazole-2-carboxamide;
N-[4-(4-fluoro-1,3-benzoxazol-2-yl)phenyl]pyridine-2-carboxamide;
N-[4-(4-fluoro-1,3-benzoxazol-2-yl)phenyl]-4-methyl-oxazole-2-carboxamide;
N-[4-(4-fluoro-1,3-benzoxazol-2-yl)phenyl]thiazole-2-carboxamide;
N-[4-(4-fluoro-1,3-benzoxazol-2-yl)phenyl]-2-methyl-oxazole-5-carboxamide;
N-[4-(4-fluoro-1,3-benzoxazol-2-yl)phenyl]pyridine-3-carboxamide;
N-[4-(4-fluoro-1,3-benzoxazol-2-yl)phenyl]bicyclo[1.1.1]pentane-1-carboxamide;
3-fluoro-N-[4-(4-fluoro-1,3-benzoxazol-2-yl)phenyl]bicyclo[1.1.1]pentane-1-carboxamide;
N-[4-(4-fluoro-1,3-benzoxazol-2-yl)phenyl]-2-hydroxy-2-methyl-propanamide;
N-[4-(4-fluoro-1,3-benzoxazol-2-yl)phenyl]-1,1-dioxo-thiolane-3-carboxamide;
N-[4-(4-fluoro-1,3-benzoxazol-2-yl)phenyl]-3-methyl-oxetane-3-carboxamide;
N-[4-(4-fluoro-1,3-benzoxazol-2-yl)phenyl]-2-hydroxy-propanamide;
N-[4-(4-fluoro-1,3-benzoxazol-2-yl)phenyl]-2,2-dimethyl-propanamide;
N-[4-(4-fluoro-1,3-benzoxazol-2-yl)phenyl]tetrahydrofuran-3-carboxamide;
N-[4-(4-fluoro-1,3-benzoxazol-2-yl)phenyl]-1,1-dioxo-thiane-4-carboxamide;
N-[4-(4-fluoro-1,3-benzoxazol-2-yl)phenyl]-1-methyl-3-oxo-cyclobutanecarboxamide;
N-[4-(4-fluoro-1,3-benzoxazol-2-yl)phenyl]-1,1-dioxo-thietane-3-carboxamide;
methyl N-[4-(4-fluoro-1,3-benzoxazol-2-yl)phenyl]carbamate;
ethyl N-[4-(4-fluoro-1,3-benzoxazol-2-yl)phenyl]carbamate;
isopropyl N-[4-(4-fluoro-1,3-benzoxazol-2-yl)phenyl]carbamate;
2,2-dimethyl-N-(4-oxazolo[5,4-b]pyridin-2-ylphenyl)propanamide;
2-methoxy-N-(4-oxazolo[5,4-b]pyridin-2-ylphenyl)propanamide;
N-(4-oxazolo[5,4-b]pyridin-2-ylphenyl)-1,1-dioxo-thiolane-3-carboxamide;
3-methyl-N-(4-oxazolo [5,4-b]pyridin-2-ylphenyl)tetrahydrofuran-3-carboxamide;
3-methyl-N-(4-oxazolo[5,4-b]pyridin-2-ylphenyl)oxetane-3-carboxamide;
N-(4-oxazolo[5,4-b]pyridin-2-ylphenyl)-1,1-dioxo-thiane-4-carboxamide;
N-(4-oxazolo[5,4-b]pyridin-2-ylphenyl)tetrahydrofuran-3-carboxamide;
N-(4-oxazolo [5,4-b]pyridin-2-ylphenyl)oxetane-3-carboxamide;
N-[4-(4,5-difluoro-1,3-benzoxazol-2-yl)phenyl]tetrahydrofuran-3-carboxamide;
N-[4-(5,7-difluoro-1,3-benzoxazol-2-yl)phenyl]tetrahydrofuran-3-carboxamide;
N-[4-(5-methyloxazolo[5,4-b]pyridin-2-yl)phenyl]tetrahydrofuran-3-carboxamide;
N-[4-(5-methoxyoxazolo[5,4-b]pyridin-2-yl)phenyl]tetrahydrofuran-3-carboxamide;
N-[4-(5-chloro-1,3-benzoxazol-2-yl)phenyl]oxazole-2-carboxamide;
N-[4-(5-chloro-1,3-benzoxazol-2-yl)phenyl]pyridine-2-carboxamide;
N-[4-(5-chloro-1,3-benzoxazol-2-yl)phenyl]tetrahydrofuran-3-carboxamide;
N-[4-(5-chloro-1,3-benzoxazol-2-yl)phenyl]bicyclo[1.1.1]pentane-1-carboxamide;
N-[4-(5-chloro-1,3-benzoxazol-2-yl)phenyl]-2-hydroxy-propanamide;
N-[4-(6,7-difluoro-1,3-benzoxazol-2-yl)phenyl]-5-methyl-oxazole-2-carboxamide;
N-[4-(6,7-difluoro-1,3-benzoxazol-2-yl)phenyl]-4-methyl-oxazole-2-carboxamide;
N-[4-(6,7-difluoro-1,3-benzoxazol-2-yl)phenyl]-5-methyl-1,3,4-oxadiazole-2-carboxamide;
N-[4-(6,7-difluoro-1,3-benzoxazol-2-yl)phenyl]-2-methyl-oxazole-5-carboxamide;
N-[4-(6,7-difluoro-1,3-benzoxazol-2-yl)phenyl]tetrahydrofuran-3-carboxamide;
N-[4-(6,7-difluoro-1,3-benzoxazol-2-yl)phenyl]-1,1-dioxo-thiolane-3-carboxamide;
N-[4-(4,6-difluoro-1,3-benzoxazol-2-yl)phenyl]tetrahydrofuran-3-carboxamide;
N-[4-(7-chloro-1,3-benzoxazol-2-yl)phenyl]-1,1-dioxo-thiolane-3-carboxamide;
(3R)-N-(4-oxazolo[5,4-b]pyridin-2-ylphenyl)-1,1-dioxo-thiolane-3-carboxamide;
(3S)-N-(4-oxazolo[5,4-b]pyridin-2-ylphenyl)-1,1-dioxo-thiolane-3-carboxamide;
(3R)-N-[4-(5-chloro-1,3-benzoxazol-2-yl)phenyl]tetrahydrofuran-3-carboxamide; and
(3S)-N-[4-(5-chloro-1,3-benzoxazol-2-yl)phenyl]tetrahydrofuran-3-carboxamide;
or a pharmaceutically acceptable salt thereof.

In another embodiment (xii) of the present invention, particular compounds of the present invention are selected from:
N-[4-(6-fluoro-1,3-benzoxazol-2-yl)phenyl]tetrahydrofuran-3-carboxamide;
N-[4-(6-chloro-1,3-benzoxazol-2-yl)phenyl]tetrahydrofuran-3-carboxamide;
N-[4-(7-fluoro-1,3-benzoxazol-2-yl)phenyl]-3-methyl-oxetane-3-carboxamide;
N-[4-(7-fluoro-1,3-benzoxazol-2-yl)phenyl] -2-hydroxy-2-methyl-propanamide;
N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]tetrahydrofuran-3-carboxamide;
3-fluoro-N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]-1,1-dioxo-thiolane-3-carboxamide;
N-[4-(7-fluoro-1,3-benzoxazol-2-yl)phenyl]-1,1-dioxo-thiane-4-carboxamide;
N-[4-(7-fluoro-1,3-benzoxazol-2-yl)phenyl] -2,2-dimethyl-propanamide;
N-[4-(7-fluoro-1,3-benzoxazol-2-yl)phenyl]cyclopropanecarboxamide;
N-[4-(7-fluoro-1,3-benzoxazol-2-yl)phenyl]-3-methyl-tetrahydrofuran-3-carboxamide;
N-[4-(7-fluoro-1,3-benzoxazol-2-yl)phenyl]-8-oxabicyclo[3.2.1]octane-3-carboxamide;
N-[4-(7-fluoro-1,3-benzoxazol-2-yl)phenyl]-1,4-dioxane-2-carboxamide;
N-[4-(7-fluoro-1,3-benzoxazol-2-yl)phenyl]-2-hydroxy-propanamide;
N-[4-(7-fluoro-1,3-benzoxazol-2-yl)phenyl]cyclobutanecarboxamide;
N-[4-(7-fluoro-1,3-benzoxazol-2-yl)phenyl]tetrahydropyran-4-carboxamide;
N-[4-(4-fluoro-1,3-benzoxazol-2-yl)phenyl]oxazole-2-carboxamide;
N-[4-(4-fluoro-1,3-benzoxazol-2-yl)phenyl]-5-methyl-oxazole-2-carboxamide;
N-[4-(4-fluoro-1,3-benzoxazol-2-yl)phenyl]pyridine-2-carboxamide;
3-fluoro-N-[4-(4-fluoro-1,3-benzoxazol-2-yl)phenyl]bicyclo[1.1.1]pentane-1-carboxamide;
N-[4-(4-fluoro-1,3-benzoxazol-2-yl)phenyl]-2-hydroxy-2-methyl-propanamide;
N-[4-(6,7-difluoro-1,3-benzoxazol-2-yl)phenyl]-5-methyl-oxazole-2-carboxamide;
N-[4-(6,7-difluoro-1,3-benzoxazol-2-yl)phenyl]-4-methyl-oxazole-2-carboxamide; and
N-[4-(6,7-difluoro-1,3-benzoxazol-2-yl)phenyl]tetrahydrofuran-3-carboxamide;
or a pharmaceutically acceptable salt thereof.

### SYNTHESIS

The compounds of the present invention can be prepared by any conventional means. Suitable processes for synthesizing these compounds as well as their starting materials are provided in the schemes below and in the examples. All substituents, in particular, A₁ to A₄ and R¹ are as defined above unless otherwise indicated. Furthermore, and unless explicitly otherwise stated, all reactions, reaction conditions, abbreviations and symbols have the meanings well known to a person of ordinary skill in organic chemistry. 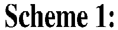 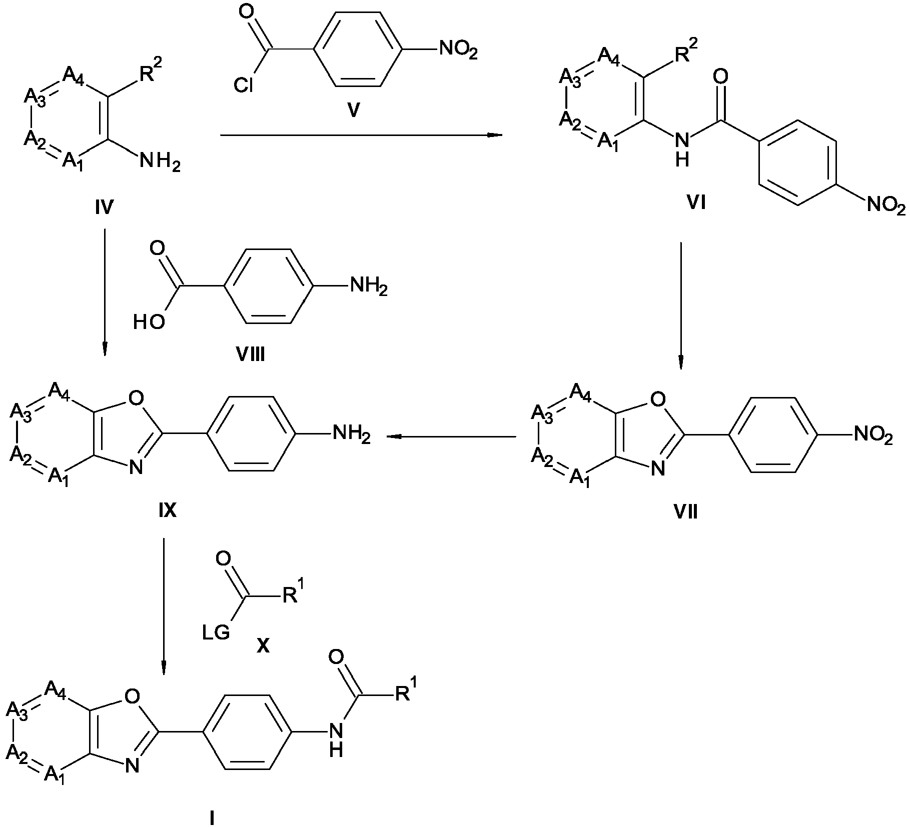 wherein R² is OH or halogen; LG is OH or halogen.

A compound of formula **IV** reacts with an acyl chloride **V** in the presence of a base, such as DIPEA, in a solvent, such as THF, to give a compound of formula **VI.** The compound of formula **VI** can also be formed with heating in the presence of a solvent such as pyridine. Cyclization of a compound of formula **VI** with POCl₃, affords a compound of formula **VII.** The compound of formula **VII** can also be formed in the presence of CuI, *N,N'-*dimethylethylenediamine and a base, such as Cs₂CO₃, in a suitable solvent such as 1,4-dioxane. Hydrogenation of a compound of formula **VII** in the presence of a catalyst, such as palladium on carbon, under a hydrogen atmosphere, in a suitable solvent, such as MeOH or a mixed solution of THF and EtOH, affords a compound of formula **IX. The** compound of formula **IX** can also be prepared by reaction of a compound of formula **IV** and a carboxylic acid **VIII** in the presence of an acid, such as polyphosphoric acid. Then a compound of formula **IX** reacts with a compound of formula **X** in the presence of a coupling reagent, such as HATU, EDCI or T₃P, and a base, such as TEA or DIPEA, in a solvent such as DMF or DCM, to afford a compound of formula **I.** The compound of formula **I** can also be prepared by reaction of a compound of formula **IX** and a compound of formula **X**in the presence of a base, such as TEA or DIPEA, in a suitable solvent, such as DCM or pyridine. The compound of formula **I** can also be prepared by reaction of a compound of formula **IX** and a compound of formula **X** by heating under a neat condition.

This invention also relates to a process for the preparation of a compound of formula (I) comprising one of the following steps:
(a) reaction of a compound of formula (IX), 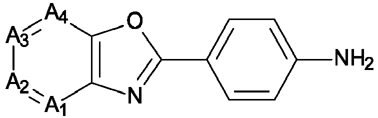 (IX), with a compound of formula (X), 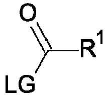 (X), in the presence of a base and optionally in the presence of a coupling reagent;
(b) reaction of a compound of formula (IX) with a compound of formula (X) by heating; wherein LG is OH or halogen.

The base in step (a) can be for example TEA or DIPEA;

The coupling reagent in step (a) can be for example HATU, EDCI or T₃P;

A compound of formula (I) when manufactured according to the above process is also an object of the invention.

The compound of this invention also shows good safety and PK profile.

### PHARMACEUTICAL COMPOSITIONS AND ADMINISTRATION

The invention also relates to a compound of formula (I) for use as therapeutically active substance. Another embodiment provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to reduction of HBsAg and HBeAg in HBV patients. For example, such amount may be below the amount that is toxic to normal cells, or the mammal as a whole.

In one example, the pharmaceutically effective amount of the compound of the invention administered parenterally per dose will be in the range of about 0.1 to 100 mg/kg, alternatively about 0.1 to 50 mg/kg of patient body weight per day, with the typical initial range of compound used being 0.3 to 15 mg/kg/day. In another embodiment, oral unit dosage forms, such as tablets and capsules, preferably contain from about 25 to about 1000 mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, e.g., tablets, powders, capsules, solutions, dispersions, suspensions, syrups, sprays, suppositories, gels, emulsions, patches, etc. Such compositions may contain components conventional in pharmaceutical preparations, e.g., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, *e.g.,* Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 25 to 500 mg of the compound of the invention compounded with about 90 to 30 mg anhydrous lactose, about 5 to 40 mg sodium croscarmellose, about 5 to 30 mg polyvinylpyrrolidone (PVP) K30, and about 1 to 10 mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 5 to 400 mg, of the invention in a suitable buffer solution, *e.g.* a phosphate buffer, adding a tonicifier, *e.g.* a salt such sodium chloride, if desired. The solution may be filtered, *e.g.,* using a 0.2 micron filter, to remove impurities and contaminants.

An embodiment, therefore, includes a pharmaceutical composition comprising a compound of formula (I), or a pharmaceutically acceptable salt thereof.

In a further embodiment includes a pharmaceutical composition comprising a compound of formula (I), or a pharmaceutically acceptable salt thereof, together with a pharmaceutically acceptable carrier or excipient.

Another embodiment includes a pharmaceutical composition comprising a compound of formula (I), or a pharmaceutically acceptable salt thereof for use in the treatment of HBV infection.

The references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### INDICATIONS AND METHODS OF TREATMENT

The compounds of the invention have anti-HBV activity. Accordingly, the compounds of the invention are useful for the treatment or prophylaxis of HBV infection.

The invention also relates to the use of a compound of formula (I) for the inhibition of HBeAg.

The invention further relates to the use of a compound of formula (I) for the inhibition of HBsAg.

The invention relates to the use of a compound of formula (I) for the inhibition of HBV DNA.

The invention relates to the use of a compound of formula (I) for use in the treatment or prophylaxis of HBV infection.

The use of a compound of formula (I) for the preparation of medicaments useful in the treatment or prophylaxis diseases that are related to HBV infection is an object of the invention.

The invention relates in particular to the use of a compound of formula (I) for the preparation of a medicament for the treatment or prophylaxis of HBV infection.

Another embodiment includes a method for the treatment or prophylaxis of HBV infection, which method comprises administering an effective amount of a compound of formula (I), or a pharmaceutically acceptable salt thereof.

The invention relates in particular to a compound of formula (I) for use in the treatment or prophylaxis of HBV infection.

### EXAMPLES

The invention will be more fully understood by reference to the following examples. They should not, however, be construed as limiting the scope of the invention.

### ABBREVIATIONS

- ACN:: acetonitrile
- DIPEA:: *N*,*N*-diisopropylethylamine
- DMAP:: 4-dimethylaminopyridine
- DMSO-*d₆*:: deuterated dimethylsulfoxide
- DCM:: dichloromethane
- EC₅₀:: the molar concentration of an agonist, which produces 50% of the maximum possible response for that agonist.
- EDCI:: *N*-ethyl-*N'*-(3-dimethylaminopropyl)carbodiimide hydrochloride
- EtOAc:: ethyl acetate
- FBS:: fetal bovine serum
- h:: hour
- HATU: *O*-(7-aza-1H-benzotriazole-1-yl)-*N*,*N*,*N'*,*N'*-tetramethyluronium hexafluorophosphate
- HPLC:: high performance liquid chromatography
- IC₅₀:: the half maximal inhibitory concentration
- LC/MS:: liquid chromatography/mass spectrometry
- LDA:: lithium diisopropylamide
- MeOH:: methanol
- MHz:: megahertz
- min:: minute
- mL:: milliliter
- mmol:: millimole
- MMTrCl:: 4-methoxytriphenylmethyl chloride
- MS (ESI):: mass spectroscopy (electron spray ionization)
- MTBE:: methyl *tert*-butyl ether
- N:: mol/L
- NaHCO₃:: sodium bicarbonate
- Na₂SO₄:: sodium sulfate
- NMR:: nuclear magnetic resonance
- obsd.:: observed
- Pd/C:: palladium on carbon
- PE:: petroleum ether
- psi:: pounds per square inch
- SFC:: supercritical fluid chromatography
- TBAF:: tetrabutylammonium fluoride
- TMSOTf:: trimethylsilyl trifluoromethanesulfonate
- THF:: tetrahydrofuran
- TLC:: thin layer chromatography
- T₃P:: 2,4,6-tripropyl-1,3,5,2,4,6-trioxatriphosphinane 2,4,6-trioxide
- µL:: microliter
- v/v:: volume ratio
- δ:: chemical shift

### GENERAL EXPERIMENTAL CONDITIONS

Intermediates and final compounds were purified by flash chromatography using one of the following instruments: i) Biotage SP1 system and the Quad 12/25 Cartridge module, ii) ISCO combi-flash chromatography instrument. Silica gel Brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using X Bridge^{™} Perp C₁₈ (5 µm, OBD^{™} 30 × 100 mm) column or SunFire^{™} Perp C₁₈ (5 µm, OBD^{™} 30 × 100 mm) column.

Chiral Separation was conducted on Thar 350 preparative SFC using ChiralPak AD-10µ (200 × 50 mm I.D.) with mobile phase A for CO₂ and B for ethanol.LC/MS spectra were obtained using a Waters UPLC-SQD Mass. Standard LC/MS conditions were as follows (running time: 3 minutes):
Acidic condition: A: 0.1% formic acid and 1% acetonitrile in H₂O; B: 0.1% formic acid in acetonitrile;
Basic condition: A: 0.05% NH₃·H₂O in H₂O; B: acetonitrile.
Mass spectra (MS): generally only ions which indicate the parent mass are reported, and unless otherwise stated the mass ion quoted is the positive mass ion (M+H)⁺.

NMR Spectra were obtained using Bruker Avance 400MHz.

All reactions involving air-sensitive reagents were performed under an argon atmosphere. Reagents were used as received from commercial suppliers without further purification unless otherwise noted.

### PREPARATIVE EXAMPLES

### Intermediate 1

### 4-(6-Fluoro-1,3-benzoxazol-2-yl)aniline

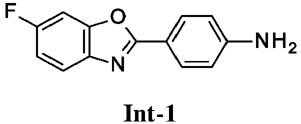

### Preparation of 4-(6-fluoro-1,3-benzoxazol-2-yl)aniline

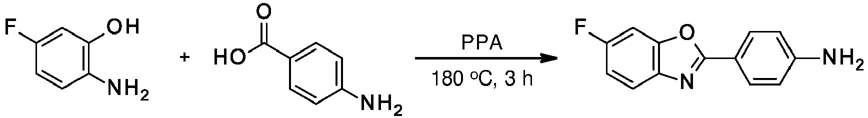

To PPA (10 g, 3.15 mmol) at 110°C were added simultaneously 2-amino-5-fluorophenol (0.40 g, 3.15 mmol) and 4-aminobenzoic acid (0.43 g, 3.15 mmol). The resulting mixture was then heated to 180 °C for 3h. The solution was then poured into water. The resulting precipitate was collected by filtration and washed with water (20 mL), a mix solution of methanol and water (40 mL, methanol/water=1/3) and saturated aqueous NaHCO₃ (40 mL). The filter cake was collected and purified by silica gel column eluted with DCM to DCM/MeOH=40/1 to give 4-(6-fluoro-1,3-benzoxazol-2-yl)aniline (496 mg) as a light yellow solid. MS obsd. (ESI⁺) [(M+H)⁺]: 229.3. ¹H NMR (400 MHz, DMSO-*d₆*) δ: 7.80 - 7.86 (m, 2 H), 7.64 -7.69 (m, 2 H), 7.19 (ddd, *J* = 10.0, 8.8, 2.5 Hz, 1 H), 6.66 - 6.72 (m, 2 H), 6.01 (s, 2 H).

### Intermediate 2

### 4-(5-Fluoro-1,3-benzoxazol-2-yl)aniline

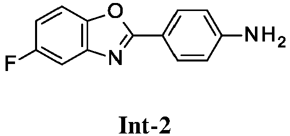

The title compound was prepared in analogy to the procedure described for the preparation of **Int-1,** by using 2-amino-4-fluorophenol instead of 2-amino-5-fluorophenol. The product was purified by preparative HPLC to afford **Int-2** as a light yellow solid.
MS obsd. (ESI⁺) [(M+H)⁺]: 229.6. ¹H NMR (400 MHz, DMSO-*d₆*) δ: 7.85 (d, *J* = 7.6 Hz, 2 H), 7.62 - 7.77 (m, 1 H), 7.52 (d, *J* = 7.6 Hz, 1 H), 7.15 (s, 1 H), 6.69 (d, *J* = 7.3 Hz, 2 H), 6.06 (br, 2 H).

### Intermediate 3

### 4-(6-Chloro-1,3-benzoxazol-2-yl)aniline

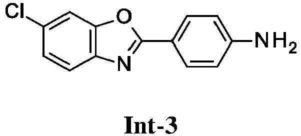

The title compound was prepared in analogy to the procedure described for the preparation of **Int-1,** by using 2-amino-5-chlorophenol instead of 2-amino-5-fluorophenol. The product was purified by preparative HPLC to afford Int-3 as a light yellow solid.
MS obsd. (ESI⁺) [(M+H)⁺]: 245.9. ¹H NMR (400 MHz, DMSO-*d₆*) δ: 7.82 - 7.87 (m, 3 H), 7.66 (d, *J* = 8.4 Hz, 1 H), 7.37 (dd, *J* = 8.4, 2.1 Hz, 1 H), 6.69 (d, *J* = 8.1 Hz, 2 H), 6.06 (s, 2 H).

### Intermediate 4

### 4-(7-Fluoro-1,3-benzoxazol-2-yl)aniline

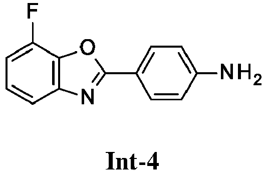

The title compound was prepared in analogy to the procedure described for the preparation of **Int-1,** by using 2-amino-6-fluorophenol instead of 2-amino-5-fluorophenol. The product was purified by silica gel column to afford **Int-4** as a light yellow solid.
MS obsd. (ESI⁺) [(M+H)⁺]: 229.3. ¹H NMR (400 MHz, DMSO-*d₆*) δ: 7.86 - 7.91 (m, 2 H), 7.51 (dd, *J* = 7.9, 0.9 Hz, 1 H), 7.21 - 7.36 (m, 2 H), 6.68 - 6.73 (m, 2 H), 6.10 (s, 2 H).

### Intermediate 5

### 4-(4-Fluoro-1,3-benzoxazol-2-yl)aniline

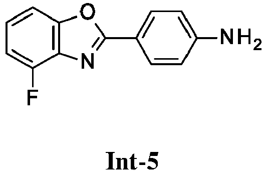

The title compound was prepared in analogy to the procedure described for the preparation of **Int-1,** by using 2-amino-3-fluorophenol instead of 2-amino-5-fluorophenol. The product was purified by silica gel column to afford **Int-5** as a pink solid.
MS obsd. (ESI⁺) [(M+H)⁺]: 229.3. ¹H NMR (400 MHz, DMSO-*d₆*) δ: 7.85 - 7.91 (m, 2 H), 7.55 (dd, *J* = 8.1, 0.7 Hz, 1 H), 7.32 (td, *J* = 8.2, 5.1 Hz, 1 H), 7.21 (t, *J* = 9.3 Hz, 1 H), 6.68 - 6.73 (m, 2 H), 6.07 (s, 2 H).

### Intermediate 6

### 4-Oxazolo[5,4-b]pyridin-2-ylaniline

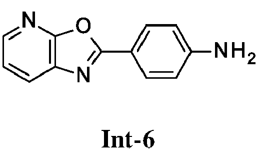

### Preparation of N-(2-hydroxy-3-pyridyl)-4-nitro-benzamide

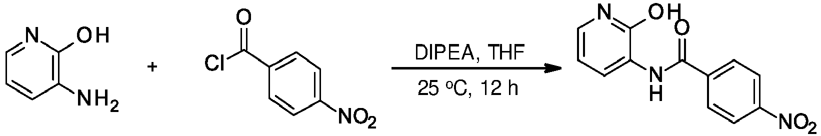

To a solution of 4-nitrobenzoic acid chloride (3.71 g, 20.0 mmol) and DIPEA (7.04 g, 54.5 mmol) in THF (30 mL) was added 3-amino-2-hydroxypyridine (2.0 g, 18.2 mmol). The mixture was stirred at 25 °C for 12 h. Then the resulting mixture was filtered and washed with MTBE (50 mL) to give N-(2-hydroxy-3-pyridyl)-4-nitro-benzamide (1.50 g) as a light grey solid.
MS obsd. (ESI⁺) [(M+H)⁺]:260.1

### Preparation of 2-(4-nitrophenyl)oxazolo[5,4-b]pyridine

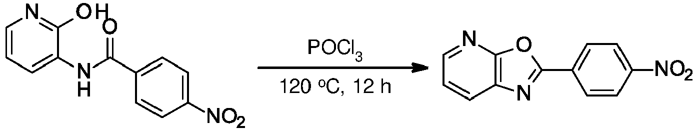

A mixture of N-(2-hydroxy-3-pyridyl)-4-nitro-benzamide (600.0 mg, 2.31 mmol) in POCl₃ (10.0 mL) was heated to 120 °C for 12 h. The mixture was concentrated and then poured into ice-cold saturated aqueous NaHCO₃. The resulting precipitate was collected by filtration and washed with ACN (40 mL). The solid was collected to afford 2-(4-nitrophenyl)oxazolo[5,4-b]pyridine (320 mg) as a light grey solid.
MS obsd (ESI⁺) [(M+H)⁺]: 242.2

### Preparation of 4-oxazolo [5, 4-b]pyridin-2-ylaniline

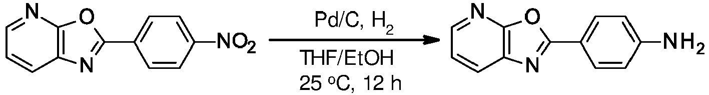

To a solution of 2-(4-nitrophenyl)oxazolo[5,4-b]pyridine (150.0 mg, 0.620 mmol) in THF (10 mL) and ethanol (10 mL) was added Pd/C (6.6 mg, 0.060 mmol) under nitrogen atmosphere. The suspension was degassed under vacuum and purged with H₂ several times. The mixture was stirred under H₂ balloon at 25 °C for 12 h. The mixture was then filtered to remove Pd/C and concentrated to afford the crude product. The crude product was purified by preparative HPLC to give 4-oxazolo[5,4-b]pyridin-2-ylaniline (Int-6) (29.6 mg) as a white solid.
MS obsd (ESI⁺) [(M+H)⁺]: 211.9. ¹H NMR (400 MHz, DMSO-*d₆*) δ: 8.24 (dd, *J* = 4.9, 1.4 Hz, 1 H), 8.08 (dd, *J* = 7.8, 1.4 Hz, 1 H), 7.89 (d, *J* = 8.7 Hz, 2 H), 7.41 (dd, *J* = 7.7, 4.9 Hz, 1 H), 6.71 (d, *J* = 8.7 Hz, 2 H), 6.14 (s, 1 H), 6.21 - 6.06 (m, 1 H).

### Intermediate 7

### 4-(4,5-Difluoro-1,3-benzoxazol-2-yl)aniline

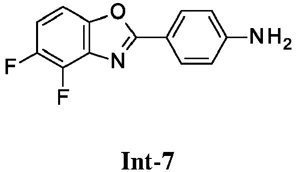

### Preparation of N-(6-bromo-2,3-difluoro-phenyl)-4-nitro-benzamide

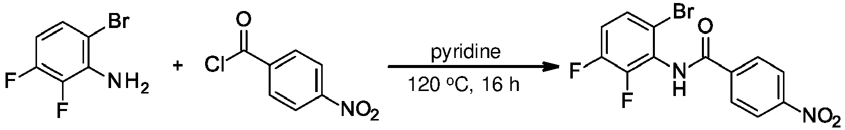

To a solution of 4-nitrobenzoic acid chloride (1.78 g, 9.62 mmol) in pyridine (20 mL) was added 6-bromo-2,3-difluoro-aniline (1.0 g, 4.81 mmol) in one portion. The reaction mixture was stirred at 120 °C for 16 h and concentrated. The residue was diluted with DCM (50 mL) and was washed with saturated aqueous NaHCO₃ (10 mL × 2). The organic layer was concentrated to give the crude product. The crude product was purified by silica gel column (PE/EtOAc= 5/1) to give N-(6-bromo-2,3-difluoro-phenyl)-4-nitro-benzamide (540 mg) as a white solid.
MS obsd. (ESI⁺)[(M+H)+]: 367.0.

### Preparation of 4,5-difluoro-2-(4-nitrophenyl)-1,3-benzoxazole

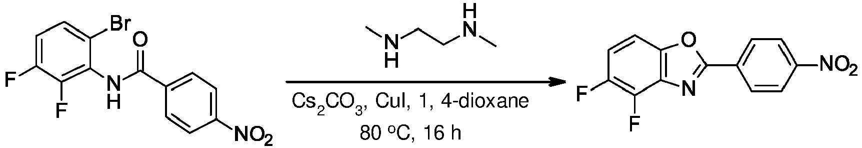

To a solution of N-(6-bromo-2,3-difluoro-phenyl)-4-nitro-benzamide (300.0 mg, 0.840 mmol), N,N'-dimethylethylenediamine (14.8 mg, 0.170 mmol) and Cs₂CO₃ (547.4 mg, 1.68 mmol) in 1,4-dioxane (60 mL) was added copper(I) iodide (32.0 mg, 0.170 mmol) in one portion under nitrogen atmosphere. The reaction mixture was stirred at 80 °C for 16 h and filtered. The filtrate was concentrated to give the crude product. The crude product was purified by silica gel column (PE/EtOAc= 1511) to give 4,5-difluoro-2-(4-nitrophenyl)-1,3-benzoxazole (240 mg) as a white solid.
MS obsd. (ESI⁺)[(M+H)+]: 277.1.

### Preparation of 4-(4,5-difluoro-1,3-benzoxazol-2-yl)aniline

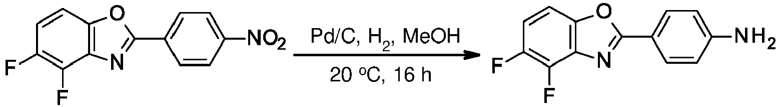

To a solution of 4,5-difluoro-2-(4-nitrophenyl)-1,3-benzoxazole (480.0 mg, 1.74 mmol) in methanol (50 mL) was added palladium on carbon (184.9 mg, 0.170 mmol) in one portion under nitrogen atmosphere. The reaction system was degassed and purged with H₂ (15 psi) and stirred at 20 °C for 16 h. The reaction mixture was filtered, and the filtrate was concentrated to give the crude product. The crude product was purified by preparative HPLC to give 4-(4,5-difluoro-1,3-benzoxazol-2-yl)aniline **(Int-7)** (185 mg) as a white solid.

MS obsd. (ESI⁺)[(M+H)+]: 247.1. ¹H NMR (400 MHz, DMSO-*d₆*) δ: 7.87 (d, *J* = 8.7 Hz, 2 H), 7.53 (ddd, *J* = 1.1, 3.4, 8.9 Hz, 1 H), 7.36 (ddd, *J* = 7.8, 8.8, 11.5 Hz, 1 H), 6.70 (d, *J* = 8.8 Hz, 2 H), 6.14 (s, 2 H).

### Intermediate 8

### 4-(5,7-Difluoro-1,3-benzoxazol-2-yl)aniline

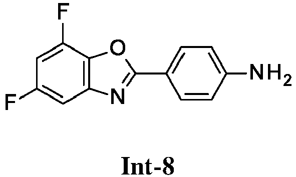

### Preparation of 2, 4-difluoro-6-nitro-phenol

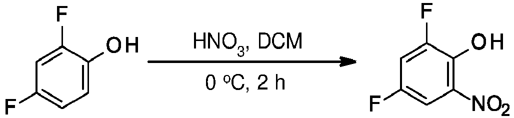

To a solution of 2, 4-difluorophenol (5.0 g, 38.4 mmol) in DCM (20 mL) was added nitric acid (5.0 mL) dropwise under nitrogen atmosphere at 0 °C. After addition, the resulting reaction was stirred at 0 °C for 2 h. The mixture was poured into ice-water (20 mL), extracted with DCM (30 mL × 3). The combined organic layer was washed with brine (30 mL), dried over Na₂SO₄, filtered and concentrated. The residue was purified by silica gel column to give 2, 4-difluoro-6-nitro-phenol (3.4 g) as a yellow solid.

### Preparation of 2-amino-4, 6-difluoro-phenol

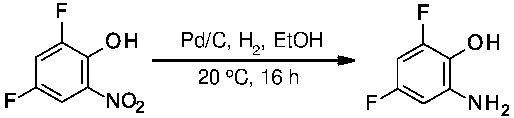

A mixture of 2, 4-difluoro-6-nitro-phenol (3.4 g, 19.4 mmol) and Pd/C (0.34 g) in ethanol (50 mL) was stirred at 20 °C for 16 h under H₂ atmosphere (1520 mmHg). The mixture was filtered and the filtrate was concentrated to give 2-amino-4, 6-difluoro-phenol (2.7 g) as a light yellow solid.

### Preparation of 4-(5,7-difluoro-1,3-benzoxazol-2-yl)aniline

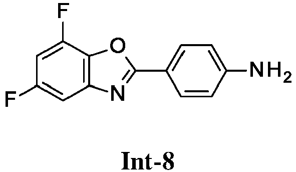

The title compound was prepared in analogy to the procedure described for the preparation of **Int-1,** by using 2-amino-4, 6-difluoro-phenol instead of 2-amino-5-fluorophenol. The product was purified by preparative HPLC to afford **Int-8** as an off-white solid.
MS obsd. (ESI⁺) [(M+H)⁺]: 247.0. ¹H NMR (400 MHz, DMSO-*d₆*) δ: 7.87 (d, *J* = 8.6 Hz, 2 H), 7.44 (dd, *J* = 8.6, 2.07 Hz, 1 H), 7.31 (td, *J* = 10.3, 2.26 Hz, 1 H), 6.70 (d, *J* = 8.8 Hz, 2 H), 6.16 (s, 2 H).

### Intermediate 9

### 4-(5-Methyloxazolo[5,4-b]pyridin-2-yl)aniline

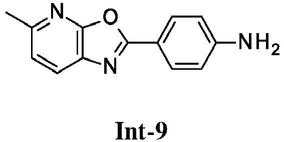

The title compound was prepared in analogy to the procedure described for the preparation of **Int-7,** by using 3-amino-2-chloro-6-picoline instead of 6-bromo-2,3-difluoro-aniline. The product was purified by silica gel column to afford **Int-9** as a yellow solid.
MS obsd. (ESI⁺) [(M+H)⁺]: 226.0. ¹H NMR (400 MHz, DMSO-*d₆*) δ: 7.95 (d, *J* = 7.9 Hz, 1 H), 7.85 (d, *J* = 8.7 Hz, 2 H), 7.26 (d, *J* = 8.0 Hz, 1 H), 6.70 (d, *J* = 8.7 Hz, 2 H), 6.07 (s, 2 H), 2.55 (s, 3 H).

### Intermediate 10

### 4-(5-Methoxyoxazolo[5,4-b]pyridin-2-yl)aniline

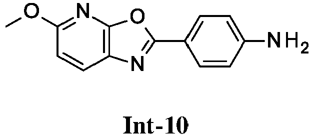

### Preparation of 2-chloro-6-methoxy-pyridin-3-amine

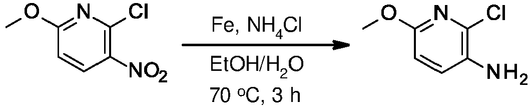

To a solution of 2-chloro-6-methoxy-3-nitropyridine (4.0 g, 21.2 mmol) and ammonium chloride (5.67 g, 106.1mmol) in ethanol (100 mL) and water (20 mL) was added iron (5.92 g, 106.1 mmol) in one portion under nitrogen atmosphere. The reaction mixture was stirred at 70 °C for 3 h. The reaction mixture was filtered, and the filtrate was extracted with DCM (100 mL × 3). The combined organic layers were dried over Na₂SO₄ and concentrated to give the crude product. The crude product was purified by silica gel column to give 2-chloro-6-methoxy-pyridin-3-amine (2.95 g) as yellow oil.
MS obsd. (ESI⁺)[(M+H)+]: 159.1.

### Preparation of 4-(5-methoxyoxazolo[5,4-b]pyridin-2-yl)aniline

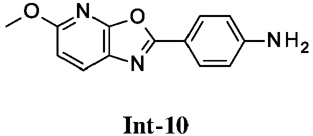

The title compound was prepared in analogy to the procedure described for the preparation of **Int-7,** by using 2-chloro-6-methoxy-pyridin-3-amine instead of 6-bromo-2,3-difluoro-aniline. The product was purified by preparative HPLC to afford **Int-10** as a yellow solid.
MS obsd. (ESI⁺) [(M+H)⁺]: 242.1. ¹H NMR (400 MHz, DMSO-*d₆*) δ: 8.02 (d, *J* = 8.5 Hz, 1 H), 7.81 (d, *J* = 8.7 Hz, 2 H), 6.83 (d, *J* = 8.5 Hz, 1 H), 6.69 (d, *J* = 8.7 Hz, 2 H), 5.99 (s, 2 H), 3.91 (s, 3 H).

### Intermediate 11

### 4-(5-Chloro-1,3-benzoxazol-2-yl)aniline

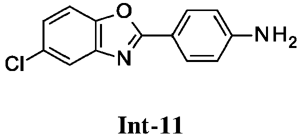

The title compound was prepared in analogy to the procedure described for the preparation of **Int-1,** by using 2-amino-4-chlorophenol instead of 2-amino-5-fluorophenol. The product was purified by trituration to afford **Int-11** as a light brown solid.
MS obsd. (ESI⁺) [(M+H)⁺]: 245.9. ¹H NMR (400 MHz, DMSO-*d₆*) δ: 7.82 - 7.90 (m, *J* = 8.7 Hz, 2 H), 7.74 (d, *J* = 2.1 Hz, 1 H), 7.70 (d, *J* = 8.6 Hz, 1 H), 7.34 (dd, *J* = 8.6, 2.1 Hz, 1 H), 6.66 - 6.74 (m, *J* = 8.7 Hz, 2 H), 6.07 (s, 2 H).

### Intermediate 12

### 4-(6,7-Difluoro-1,3-benzoxazol-2-yl)aniline

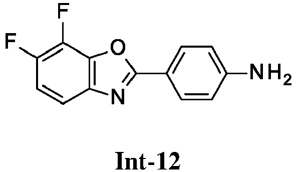

The title compound was prepared in analogy to the procedure described for the preparation of **Int-1,** by using 6-amino-2,3-difluoro-phenol instead of 2-amino-5-fluorophenolThe product was collected by filtration to afford **Int-12** as a black solid.
MS obsd. (ESI⁺) [(M+H)⁺]: 247.0. ¹H NMR (400 MHz, DMSO-*d₆*) δ: 7.84 (d, *J* = 8.7 Hz, 2 H), 7.35 - 7.50 (m, 2 H), 6.69 (d, *J* = 8.7 Hz, 2 H), 6.10 (s, 2 H).

### Intermediate 13

### 4-(5-Methyloxazolo[5,4-b]pyridin-2-yl)aniline

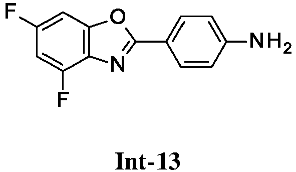

The title compound was prepared in analogy to the procedure described for the preparation of **Int-7,** by using 2-bromo-4,6-difluoro-aniline instead of 6-bromo-2,3-difluoro-aniline. The product was purified by silica gel column to afford **Int-13** as a yellow solid.
MS obsd. (ESI⁺) [(M+H)⁺]: 247.0. ¹H NMR (400 MHz, DMSO-*d₆*) δ: 7.81 - 7.87 (m, 2 H), 7.59 (dd, *J* = 8.2, 2.0 Hz, 1 H), 7.29 (td, *J* = 10.5, 2.3 Hz, 1 H), 6.67 - 6.73 (m, 2 H), 6.10 (s, 2 H).

### Intermediate 14

### 4-(5-Methyloxazolo[5,4-b]pyridin-2-yl)aniline

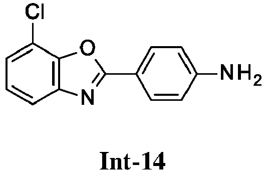

The title compound was prepared in analogy to the procedure described for the preparation of **Int-1,** by using 2-amino-6-chlorophenol instead of 2-amino-5-fluorophenol. The product was purified by silica gel column to afford **Int-14** as a yellow solid.
MS obsd. (ESI⁺) [(M+H)⁺]: 246.0. ¹H NMR (400 MHz, DMSO-*d₆*) δ: 7.84 - 7.90 (m, 2 H), 7.64 (dd, *J* = 7.7, 1.1 Hz, 1 H), 7.31 - 7.42 (m, 2 H), 6.68 - 6.73 (m, 2 H), 6.10 (s, 2 H).

### Intermediate 15

### 4-Acetoxytetrahydropyran-4-carboxylic acid

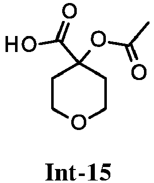

### Preparation of 4-acetoxytetrahydropyran-4-carboxylic acid

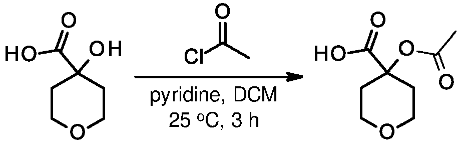

A solution of 4-hydroxytetrahydro-2H-pyran-4-carboxylic acid (300.0 mg, 2.05 mmol) was mixed with pyridine (195.0 mg, 199 µL, 2.46 mmol) and DCM (10 mL). After cooling to 0 °C, acetyl chloride (177.0 mg, 161 µL, 2.26 mmol) was added dropwise to the mixture. The resulting mixture was stirred at room temperature for 3 h. The reaction was diluted with DCM (15 mL) and washed with saturated aqueous NH₄Cl (5 mL) and brine (5 mL). The organic phase was dried over Na₂SO₄ and concentrated in vacuum. The crude product (**Int-15**)was used for next step without further purification.
MS obsd. (ESI⁺) [(M-H)⁺]: 187.0.

### Intermediate 16

### 1-Acetoxycyclopropanecarboxylic acid

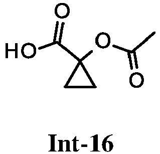

The title compound was prepared in analogy to the procedure described for the preparation of **Int-15,** by using 1-hydroxycyclopropanecarboxylic acid instead of 4-hydroxytetrahydro-2H-pyran-4-carboxylic acid. The product **Int-16** was used for next step without further purification.

### Intermediate 17

### Tetrahydrofuran-3-yl carbonochloridate

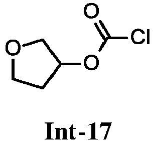

### Preparation of tetrahydrofuran-3-yl carbonochloridate

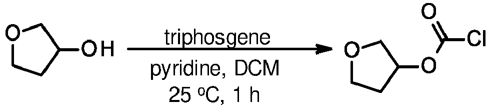

To a solution of triphosgene (842.0 mg, 2.84 mmol) in DCM (2.5 mL) was added a solution of tetrahydrofuran-3-ol (500.0 mg, 5.68 mmol) and pyridine (978.0 mg, 1 mL, 12.4 mmol) in DCM (2.5 mL) dropwise at 0 °C. The mixture became orange and a yellow precipitate appeared. The mixture was allowed to warm to room temperature and stirred for 1 h. Aqueous HCl (0.1N, 10 mL) was added and the organic layer was separated. The organic layer was washed with 0.1 N HCl (3 × 5 mL), brine (5 mL), dried over Na₂SO₄ and concentrated in vacuum to give light brown oil which became solid upon standing in the freezer for 24 h. The crude product **Int-17** was used for next step without further purification.

### Example 1

### N-[4-(6-fluoro-1,3-benzoxazol-2-yl)phenyl]tetrahydrofuran-3-carboxamide

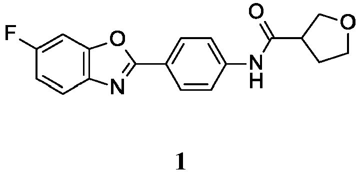

### Preparation of N-[4-(6-fluoro-1,3-benzoxazol-2-yl)phenyl]tetrahydrofuran-3-carboxamide

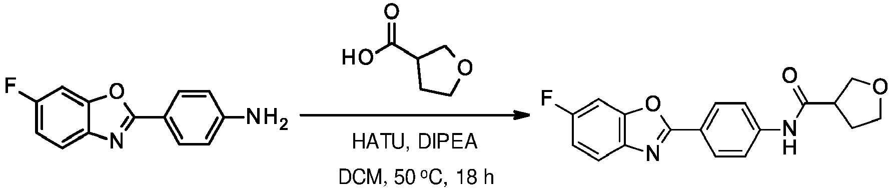

To a solution of 4-(6-fluorobenzo[d]oxazol-2-yl)aniline (**Int-1,** as the **"AMINE"** in Table 1) (50.0 mg, 0.219 mmol) and tetrahydrofuran-3-carboxylic acid (30.5 mg, 0.263 mmol, as the **"ACID"** or **"ACYL CHLORIDE"** in Table 1) in DCM (2 mL) was added HATU (100.0 mg, 0.263 mmol) and DIPEA (56.6 mg, 76.5 µL, 0.438 mmol). Then the mixture was stirred at 50 °C for 18 h. The reaction mixture was concentrated in vacuum and the residue was triturated in water (5 mL). The resulting mixture was filtered and the filter cake was washed with a mixed solution of water (10 mL) and methanol (10 mL). The cake was collected and dried in vacuum to give *N*-[4-(6-fluoro-1,3-benzoxazol-2-yl)phenyl]tetrahydrofuran-3-carboxamide (**Example 1**)(69 mg) as a pink solid.
MS obsd. (ESI⁺) [(M+H)⁺]: 327.3. ¹H NMR (400 MHz, DMSO-*d₆*) δ: 10.40 (s, 1 H), 8.12 (d, *J* = 8.2 Hz, 2 H), 7.75 - 7.87 (m, 4 H), 7.28 (ddd, *J* = 10.0, 8.7, 2.6 Hz, 1 H), 3.96 (t, *J* = 8.2 Hz, 1 H), 3.70 - 3.83 (m, 3 H), 3.16 - 3.30 (m, 1 H), 2.05 - 2.17 (m, 2 H).

The following **Example 2** to **Example 85** were prepared in analogy to the procedure described for the preparation of **Example 1,** replacing 4-(6-fluorobenzo[d]oxazol-2-yl)aniline (**Int-1**) with **"AMINE",** tetrahydrofuran-3-carboxylic acid with **"ACID" or "ACYL CHLORIDE".** The **"AMINE", "ACID"** and **"ACYL CHLORIDE"** are the reagents indicated in Table 1.

**Table 1: Compounds synthesis and characterization**

| Example No. | Compounds Name and Structure | **AMINE, ACID** and **ACYL CHLORIDE** | ¹H NMR and (ESI⁺) |
|---|---|---|---|
| **2** | ***N*-[4-(6-fluoro-1,3-benzoxazol-2-yl)phenyl]-2-hydroxy-propanamide** 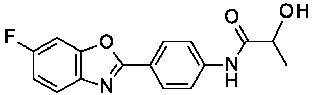 | **AMINE: Int-1** | ¹H NMR (400 MHz, DMSO-*d₆*) δ: 10.06 (s, 1 H), 8.07 - 8.16 (m, 2 H), 7.94 - 8.03 (m, 2 H), 7.73 - 7.85 (m, 2 H), 7.28 (t, *J* = 9.2 Hz, 1 H), 5.84 (br d, *J* = 4.2 Hz, 1 H), 4.15 - 4.24 (m, 1 H), 1.33 (d, *J* = 6.8 Hz, 3 H). |
| | | **ACID:** 2-hydroxypropan oic acid | |
| | | | MS obsd. (ESI⁺) [(M+H)⁺]: 301.2. |
| **3** | ***N*-[4-(6-fluoro-1,3-benzoxazol-2-yl)phenyl]-2,2-dimethyl-propanamide** 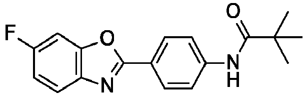 | **AMINE: Int-1** | ¹H NMR (400 MHz, DMSO-*d₆*) δ: 9.55 1 8.08 8.14 2 (s, H), - (m, H), 7.90 - 7.96 (m, 2 H), 7.74 - 7.82 (m, 2 H), 7.28 (ddd, *J* = 10.0, 8.7, 2.4 Hz, 1 H), 1.26 (s, 9 H). |
| | | **ACYL CHLORIDE:** 2,2-dimethylpropa noyl chloride | |
| | | | MS obsd. (ESI⁺) [(M+H)⁺]: 313.4. |
| **4** | ***N*-[4-(6-fluoro-1,3-benzoxazol-2-yl)phenyl]tetrahydrothiopyran-4-carboxamide** 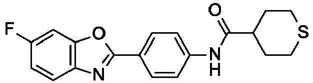 | **AMINE: Int-1** | ¹H NMR (DMSO-*d₆*, 400MHz)δ: 10.26 (s, 1 H), 8.19 - 8.06 (m, 2 H), 7.93 - 7.66 (m, 4 H), 7.27 (ddd, *J* = 2.4, 8.7, 10.0 Hz, 1 H), 2.73 - 2.59 (m, 5 H), 2.12 (br dd, *J* = 3.3, 13.3 Hz, 2 H), 1.83 - 1.66 (m, 2 H). |
| | | **ACID:** tetrahydrothiop yran-4-carboxylic acid | |
| | | | MS obsd. (ESI⁺) [(M+H)⁺]: 357.1 |
| **5** | ***N*-[4-(6-fluoro-1,3-benzoxazol-2-yl)phenyl]-2-methyl-tetrahydrofuran-2-carboxamide** | **AMINE: Int-1** | ¹H NMR (DMSO-*d₆*, 400MHz)δ: 9.96 (s, 1 H), 8.08 - 8.14 (m, 2 H), 7.99 - 8.04 (m, 2 H), 7.76 - 7.82 (m, 2 H), 7.28 (ddd, *J* = 10.0, 8.7, 2.4 Hz, 1 H), 3.92 - 4.00 (m, 2 H), 2.27 - 2.48 (m, 1 H), 1.77 - 1.98 (m, 3 H), 1.43 (s, 3 H). |
| | | **ACID:** 2-methyltetrahyd rofuran-2-carboxylic acid | |
| | 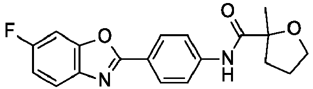 | | |
| | | | MS obsd. (ESI⁺) [(M+H)⁺]: 341.2 |
| **6** | ***N*-[4-(5-fluoro-1,3-benzoxazol-2-yl)phenyl] -2,2-dimethyl-propanamide** | **AMINE: Int-2** | ¹H NMR (DMSO-*d₆*, 400MHz) δ: 9.56 (s, 1 H), 8.08 - 8.18 (m, 2 H), 7.91 - 7.97 (m, 2 H), 7.80 (dd, *J* = 8.9, 4.3 Hz, 1 H), 7.65 (dd, *J* = 8.8, 2.6 Hz, 1 H), 7.27 (td, *J* = 9.3, 2.7 Hz, 1 H), 1.26 (s, 9 H). |
| | | **ACYL CHLORIDE:** 2,2-dimethylpropa noyl chloride | |
| | 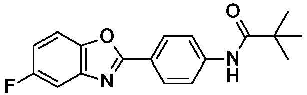 | | |
| | | | MS obsd. (ESI⁺) [(M+H)⁺]: 313.3 |
| **7** | ***N*-[4-(5-fluoro-1,3-benzoxazol-2-yl)phenyl]tetrahydrothiopyran-4-carboxamide** 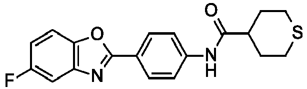 | **AMINE: Int-2** | ¹H NMR (DMSO-*d₆*, 400MHz) δ: 10.29 (s, 1 H), 8.28 - 8.10 (m, 2 H), 7.95 - 7.79 (m, 2 H), 7.63 (dd, *J* = 1.0, 7.8 Hz, 1 H), 7.44 - 7.24 (m, 2 H), 2.81 - 2.59 (m, 4 H), 2.13 (br dd, *J* = 3.2, 13.3 Hz, 2H), 1.85 - 1.60 (m, 2 H). |
| | | **ACID:** tetrahydrothiop yran-4-carboxylic acid | |
| | | | MS obsd. (ESI⁺) [(M+H)⁺]: 357.1. |
| **8** | ***N*-[4-(6-chloro-1,3-benzoxazol-2-yl)phenyl]tetrahydrofuran-3-carboxamide** 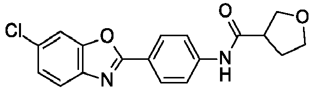 | **AMINE: Int-3** | ¹H NMR (DMSO-*d₆*, 400MHz) δ: 10.43 (s, 1 H), 8.14 (d, *J* = 8.7 Hz, 2 H), 7.97 (d, *J* = 2.0 Hz, 1 H), 7.77 - 7.88 (m, 3 H), 7.45 (dd, *J* = 8.4, 2.0 Hz, 1 H), 3.96 (t, *J* = 8.2 Hz, 1 H), 3.69 - 3.84 (m, 3 H), 3.21 (quin, *J* = 7.5 Hz, 1 H), 2.06 - 2.17 (m, 2 H). |
| | | **ACID:** tetrahydrofura n-3-carboxylic acid | |
| | | | MS obsd. (ESI⁺) [(M+H)⁺]: 343.1 |
| **9** | ***N*-[4-(6-chloro-1,3-benzoxazol-2-yl)phenyl]butanamide** 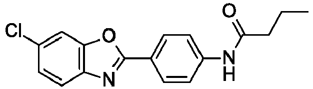 | **AMINE: Int-3** | ¹H NMR (DMSO-*d₆*, 400MHz) δ: 10.28 (s, 1 H), 8.12 (d, *J* = 8.0 Hz, 2 H), 7.97 (d, *J* = 2.0 Hz, 1 H), 7.77 - 7.88 (m, 3 H), 7.45 (dd, *J* = 8.5, 2.0 Hz, 1 H), 2.35 (t, *J* = 7.3 Hz, 2 H), 1.64 (sxt, *J* = 7.4 Hz, 2 H), 0.93 (t, *J* = 7.4 Hz, 3 H). |
| | | **ACYL CHLORIDE:** butanoyl chloride | |
| | | | MS obsd. (ESI⁺) [(M+H)⁺]: 315.1. |
| **10** | ***N*-[4-(6-chloro-1,3-benzoxazol-2-yl)phenyl]-1,1-dioxo-thiolane-3-carboxamide** 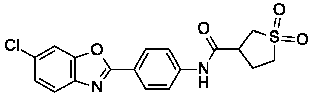 | **AMINE: Int-3** | ¹H NMR (DMSO-*d₆*, 400MHz) δ: 10.58 (s, 1 H), 8.16 (d, *J* = 8.1 Hz, 2 H), 7.98 (d, *J* = 1.8 Hz, 1 H), 7.78 - 7.87 (m, 3 H), 7.45 (dd, *J* = 8.5, 2.0 Hz, 1 H), 3.35 - 3.51 (m, 2 H), 3.11 - 3.31 (m, 3 H), 2.43 - 2.47 (m, 1 H), 2.19-2.29 (m, 1 H). |
| | | **ACID:** 1,1-dioxothiolane-3-carboxylic acid | |
| | | | MS obsd. (ESI⁺) [(M+H)⁺]: 391.1. |
| **11** | ***N*-[4-(7-fluoro-1,3-benzoxazol-2-yl)phenyl]furan-3-carboxamide** 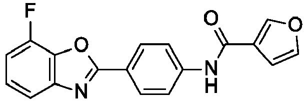 | **AMINE: Int-4** | ¹H NMR (DMSO-*d₆*, 400MHz) δ: 10.29 (s, 1 H), 8.46 (s, 1 H), 8.19 - 8.25 (m, 2 H), 7.98 - 8.04 (m, 2 H), 7.84 (t, *J* = 1.7 Hz, 1 H), 7.64 (dd, *J* = 7.7, 1.0 Hz, 1 H), 7.32 - 7.44 (m, 2 H), 7.04 (dd, *J* = 1.8, 0.7 Hz, 1H). |
| | | **ACID:** furan-3-carboxylic acid | |
| | | | MS obsd. (ESI⁺) [(M+H)⁺]: 323.1. |
| **12** | ***N*-[4-(7-fluoro-1,3-benzoxazol-2-yl)phenyl]-3-methyl-oxetane-3-carboxamide** 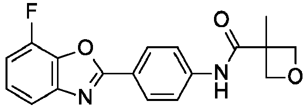 | **AMINE: Int-4** | ¹H NMR (DMSO-*d₆*, 400MHz) δ: 10.15 (s, 1 H), 8.17 - 8.22 (m, 2 H), 7.87 - 7.93 (m, 2 H), 7.64 (dd, *J* = 7.8, 1.0 Hz, 1 H), 7.32 - 7.44 (m, 2 H), 4.86 (d, *J* = 6.1 Hz, 2 H), 4.38 (d, *J* = 6.1 Hz, 2 H), 1.64 (s, 3 H). |
| | | **ACID:** 3-methyloxetane -3-carboxylic acid | |
| | | | MS obsd. (ESI+) [(M+H)+]: 327.5. |
| **13** | ***N*-[4-(7-fluoro-1,3-benzoxazol-2-yl)phenyl] -2-hydroxy-2-methyl-propanamide** 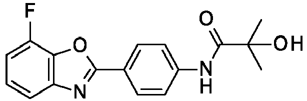 | **AMINE: Int-4** | ¹H NMR (DMSO-*d₆*, 400MHz) δ: 9.98 (s, 1 H), 8.13 - 8.19 (m, 2 H), 8.00 - 8.07 (m, 2 H), 7.63 (dd, *J* = 7.8, 1.0 Hz, 1 H), 7.31 - 7.43 (m, 2 H), 5.82 (s, 1 H), 1.38 (s, 6 H). |
| | | **ACID:** 2-hydroxy-2-methyl-propanoic acid | |
| | | | MS obsd. (ESI+) [(M+H)+]: 315.2. |
| **14** | ***N*-[4-(7-fluoro-1,3-benzoxazol-2-yl)phenyl]bicyclo[1.1.1]penta ne-1-carboxamide** 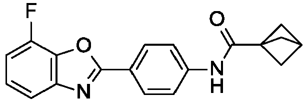 | **AMINE: Int-4** | ¹H NMR (DMSO-*d₆*, 400MHz) δ: 9.89 (s, 1 H), 8.13 - 8.22 (m, *J* = 8.7 Hz, 2 H), 7.88 - 7.98 (m, *J* = 8.7 Hz, 2 H), 7.63 (d, *J* = 7.7 Hz, 1 H), 7.31 - 7.44 (m, 2 H), 2.46 - 2.49 (m, 1 H), 2.12 (s, 6 H). |
| | | **ACID:** bicyclo[1.1.1]p entane-1-carboxylic acid | |
| | | | MS obsd. (ESI+) [(M+H)+]: 323.3. |
| **15** | ***N*-[4-(7-fluoro-1,3-benzoxazol-2-yl)phenyl]tetrahydrofuran-3-carboxamide** 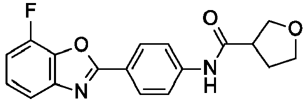 | **AMINE: Int-4** | ¹H NMR (DMSO-*d₆*, 400MHz) δ: 10.44 (s, 1 H), 8.14 - 8.20 (m, *J* = 8.8 Hz, 2 H), 7.83 - 7.89 (m, *J* = 8.8 Hz, 2H), 7.63 (dd, *J* = 7.8, 0.9 Hz, 1 H), 7.31 - 7.43 (m, 2 H), 3.96 (t, *J* = 8.3 Hz, 1 H), 3.70 - 3.84 (m, 3 H), 3.21 (quin, *J* = 7.5 Hz, 1 H), 2.06 - 2.18 (m, 2 H). |
| | | **ACID:** tetrahydrofura n-3-carboxylic acid | |
| | | | MS obsd. (ESI+) [(M+H)+]: 327.3. |
| **16** | **3-fluoro-*N*-[4-(7-fluoro-1,3-benzoxazol-2-yl)phenyl]bicyclo[1.1.1]penta ne-1-carboxamide** 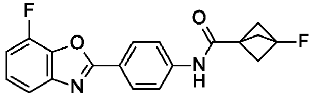 | **AMINE: Int-4** | ¹H NMR (DMSO-*d₆*, 400MHz) δ: 10.07 (s, 1 H), 8.16 - 8.22 (m, 2 H), 7.88 - 7.94 (m, 2 H), 7.64 (dd, *J* = 7.8, 0.9 Hz, 1 H), 7.32 - 7.44 (m, 2 H), 2.44 (d, *J* = 2.6 Hz, 6 H). |
| | | **ACID:** 3-fluorobicyclo[ 1.1.1]pentane-1-carboxylic acid | |
| | | | MS obsd. (ESI+) [(M+H)+]: 341.2. |
| **17** | ***N*-[4-(7-fluoro-1,3-benzoxazol-2-yl)phenyl]-1,1-dioxo-thiolane-3-carboxamide** 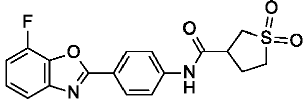 | **AMINE: Int-4** | ¹H NMR (DMSO-*d₆*, 400MHz) δ: 10.60 (s, 1 H), 8.17 - 8.22 (m, 2 H), 7.86 (d, *J* = 8.8 Hz, 2 H), 7.64 (dd, *J* = 7.8, 1.0 Hz, 1 H), 7.32 - 7.44 (m, 2 H), 3.35-3.51 (m, 2 H), 3.12 - 3.32 (m, 3 H), 2.35 - 2.48 (m, 1 H), 2.12 - 2.32 (m, 1 H). |
| | | **ACID:** 1,1-dioxothiolane-3-carboxylic acid | |
| | | | MS obsd. (ESI+) [(M+H)+]: 375.1. |
| **18** | ***N*-[4-(7-fluoro-1,3-benzoxazol-2-yl)phenyl]-1,1-dioxo-thiane-4-carboxamide** 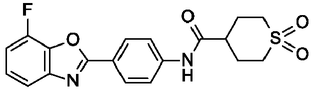 | **AMINE: Int-4** | ¹H NMR (DMSO-*d₆*, 400MHz) δ: 10.47 (s, 1 H), 8.15 - 8.20 (m, 2 H), 7.84 - 7.90 (m, *J* = 8.9 Hz, 2 H), 7.63 (dd, *J* = 7.8, 0.9 Hz, 1 H), 7.32 - 7.43 (m, 2 H), 3.14 - 3.31 (m, 4 H), 2.67 - 2.78 (m, 1 H), 2.07 - 2.28 (m, 4 H). |
| | | **ACID:** 1,1-dioxothiane-4-carboxylic acid | |
| | | | MS obsd. (ESI+) [(M+H)+]: 389.1. |
| **19** | ***N*-[4-(7-fluoro-1,3-benzoxazol-2-yl)phenyl] -2,2-dimethyl-propanamide** 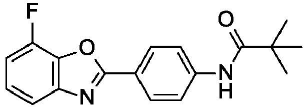 | **AMINE: Int-4** | ¹H NMR (DMSO-*d₆*, 400MHz) δ: 9.58 (s, 1 H), 8.13 - 8.19 (m, *J* = 8.8 Hz, 2 H), 7.92 - 7.98 (m, *J* = 8.8 Hz, 2 H), 7.63 (dd, *J* = 7.8, 1.0 Hz, 1 H), 7.31 - 7.44 (m, 2 H), 1.26 (s, 9 H). |
| | | **ACYL CHLORIDE:** 2,2-dimethylpropa noyl chloride | |
| | | | MS obsd. (ESI+) [(M+H)+]: 313.3. |
| **20** | ***N*-[4-(7-fluoro-1,3-benzoxazol-2-yl)phenyl]cyclopropanecarbo xamide** 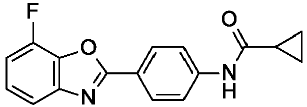 | **AMINE: Int-4** | ¹H NMR (DMSO-*d₆*, 400MHz) δ: 10.61 (s, 1 H), 8.13 - 8.19 (m, 2 H), 7.83 - 7.88 (m, 2 H), 7.63 (dd, *J* = 7.8, 1.0 Hz, 1 H), 7.31 - 7.43 (m, 2 H), 1.84 (quin, *J* = 6.2 Hz, 1 H), 0.82 - 0.88 (m, 4 H). |
| | | **ACYL CHLORIDE:** cyclopropanec arbonyl chloride | |
| | | | MS obsd. (ESI+) [(M+H)+]: 297.2. |
| **21** | ***N*-[4-(7-fluoro-1,3-benzoxazol-2-yl)phenyl]-3-methyl-tetrahydrofuran-3-carboxamide** 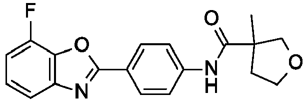 | **AMINE: Int-4** | ¹H NMR (DMSO-*d₆*, 400MHz) δ: 9.88 (s, 1 H), 8.15 - 8.21 (m, 2 H), 7.90 - 7.96 (m, 2 H), 7.63 (dd, *J* = 7.7, 1.0 Hz, 1 H), 7.32 - 7.44 (m, 2H), 4.12 (d, *J* = 8.7 Hz, 1 H), 3.75 - 3.85 (m, 2 H), 3.52 (d, *J* = 8.7 Hz, 1 H), 2.44 - 2.49 (m, 1 H), 1.83 (ddd, *J* = 12.5, 7.6, 6.2 Hz, 1 H), 1.42 (s, 3 H). |
| | | **ACID:** 2-methyltetrahyd rofuran-2-carboxylic acid | |
| | | | MS obsd. (ESI+) [(M+H)+]: 340.8. |
| **22** | ***N*-[4-(7-fluoro-1,3-benzoxazol-2-yl)phenyl]-8-oxabicyclo[3.2.1]octane-3-carboxamide** 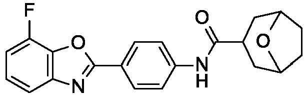 | **AMINE: Int-4** | ¹H NMR (DMSO-*d₆*, 400MHz) δ: 10.24 (s, 1 H), 8.13 - 8.19 (m, 2 H), 7.82 - 7.88 (m, 2 H), 7.63 (d, *J* = 8.0 Hz, 1 H), 7.30 - 7.43 (m, 2 H), 4.36 (br s, 2 H), 2.84 - 2.94 (m, 1 H), 1.73 - 1.92 (m, 6 H), 1.60 (br dd, *J* = 12.9, 5.0 Hz, 2 H). |
| | | **ACID:** 8-oxabicyclo [3.2 .1]octane-3-carboxylic acid | |
| | | | MS obsd. (ESI+) [(M+H)+]: 367.1. |
| **23** | ***N*-[4-(7-fluoro-1,3-benzoxazol-2-yl)phenyl]-1,4-dioxane-2-carboxamide** 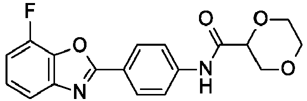 | **AMINE: Int-4** | ¹H NMR (DMSO-*d₆*, 400MHz) δ: 10.20 (s, 1 H), 8.15 - 8.20 (m, 2 H), 7.94 - 7.99 (m, *J* = 8.8 Hz, 2 H), 7.64 (dd, *J* = 7.8, 0.9 Hz, 1 H), 7.32 - 7.44 (m, 2 H), 4.30 (dd, *J* = 9.2, 3.1 Hz, 1 H), 3.88 - 3.99 (m, 2 H), 3.69 - 3.78 (m, 2 H), 3.54 - 3.64 (m, 2 H). |
| | | **ACID:** 1,4-dioxane-2-carboxylic acid | |
| | | | MS obsd. (ESI+) [(M+H)+]: 343.1. |
| **24** | ***N*-[4-(7-fluoro-1,3-benzoxazol-2-yl)phenyl]-2-hydroxy-propanamide** 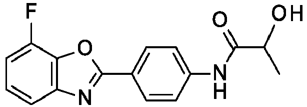 | **AMINE: Int-4** | ¹H NMR (DMSO-*d₆*, 400MHz) δ: 10.09 (s, 1 H), 8.14 - 8.19 (m, 2 H), 7.98 - 8.03 (m, 2 H), 7.64 (dd, *J* = 7.8, 1.0 Hz, 1 H), 7.32 - 7.43 (m, 2 H), 5.84 (br d, *J* = 5.1 Hz, 1 H), 4.17 - 4.24 (m, 1 H), 1.34 (d, *J* = 6.8 Hz, 3 H). |
| | | **ACID:** 2-hydroxypropan oic acid | |
| | | | MS obsd. (ESI+) [(M+H)+]: 301.2. |
| **25** | ***N*-[4-(7-fluoro-1,3-benzoxazol-2-yl)phenyl]cyclobutanecarbox amide** 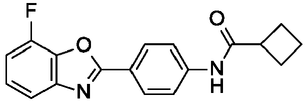 | **AMINE: Int-4** | ¹H NMR (DMSO-*d₆*, 400MHz) δ: 10.13 (s, 1 H), 8.13 - 8.18 (m, 2 H), 7.84 - 7.90 (m, *J* = 8.8 Hz, 2 H), 7.63 (dd, *J* = 7.8, 0.9 Hz, 1 H), 7.31 - 7.43 (m, 2 H), 3.24 - 3.32 (m, 1 H), 2.09 - 2.30 (m, 4 H), 1.78 - 2.02 (m, 2 H). |
| | | **ACID:** cyclobutanecar boxylic acid | |
| | | | MS obsd. (ESI+) [(M+H)+]: 311.3. |
| **26** | **[4-[[4-(7-fluoro-1,3-benzoxazol-2-yl)phenyl] carbamoyl] tetrahy dropyran-4-yl] acetate** 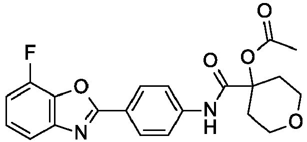 | **AMINE: Int-4** | ¹H NMR (DMSO-*d₆*, 400MHz) δ: 10.04 (s, 1 H), 8.13 - 8.20 (m, 2 H), 7.87 - 7.93 (m, 2 H), 7.63 (dd, *J* = 7.8, 1.0 Hz, 1 H), 7.32 - 7.44 (m, 2 H), 3.75 - 3.86 (m, 2 H), 3.63 (td, *J* = 11.0, 2.8 Hz, 2 H), 2.01 - 2.19 (m, 7 H). |
| | | **ACID: Int-15** | |
| | | | MS obsd. (ESI+) [(M+H)+]: 399.4. |
| **27** | **2,2-difluoro-*N*-[4-(7-fluoro-1,3-benzoxazol-2-yl)phenyl]cyclopropanecarbo xamide** 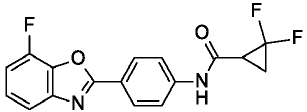 | **AMINE: Int-4** | ¹H NMR (DMSO-*d₆*, 400MHz) δ: 10.84 (s, 1 H), 8.16 - 8.23 (m, *J* = 8.8 Hz, 2H), 7.81 - 7.89 (m, *J* = 8.7 Hz, 2 H), 7.64 (d, *J* = 7.8 Hz, 1 H), 7.31 - 7.45 (m, 2 H), 2.81 - 2.95 (m, 1 H), 1.97 - 2.12 (m, 2 H). |
| | | **ACID:** 2,2-difluorocyclop ropanecarboxy lic acid | |
| | | | MS obsd. (ESI+) [(M+H)+]: 333.3. |
| **28** | ***N*-[4-(7-fluoro-1,3-benzoxazol-2-yl)phenyl]tetrahydropyran-4-carboxamide** 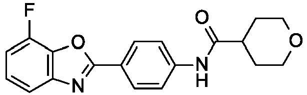 | **AMINE: Int-4** | ¹H NMR (DMSO-*d₆*, 400MHz) δ: 10.31 (s, 1 H), 8.14 - 8.19 (m, 2 H), 7.84 - 7.90 (m, 2 H), 7.63 (dd, *J* = 7.8, 1.0 Hz, 1 H), 7.31 - 7.43 (m, 2 H), 3.88 - 3.97 (m, 2 H), 3.34 - 3.41 (m, 2 H), 2.53 - 2.70 (m, 1 H), 1.63 - 1.77 (m, 4 H). |
| | | **ACID:** tetrahydropyra n-4-carboxylic acid | |
| | | | MS obsd. (ESI+) [(M+H)+]: 341.2. |
| **29** | ***N*-[4-(7-fluoro-1,3-benzoxazol-2-yl)phenyl]oxetane-3-carboxamide** 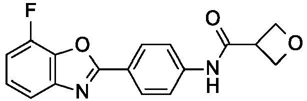 | **AMINE: Int-4** | ¹H NMR (DMSO-*d₆*, 400MHz) δ: 10.35 (s, 1 H), 8.16 - 8.21 (m, *J* = 8.8 Hz, 2 H), 7.85 - 7.91 (m, *J* = 8.8 Hz, 2 H), 7.64 (dd, *J* = 7.7, 1.0 Hz, 1 H), 7.32 - 7.44 (m, 2 H), 4.69 - 4.76 (m, 4 H), 3.97 - 4.06 (m, 1 H). |
| | | **ACID:** oxetane-3-carboxylic acid | |
| | | | MS obsd. (ESI+) [(M+H)+]: 313.2. |
| **30** | ***N*-[4-(7-fluoro-1,3-benzoxazol-2-yl)phenyl]tetrahydrothiopyra n-4-carboxamide** 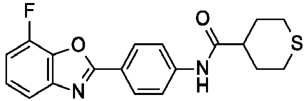 | **AMINE: Int-4** | ¹H NMR (DMSO-*d₆*, 400MHz) δ: 10.29 (s, 1 H), 8.12 - 8.19 (m, *J* = 8.8 Hz, 2 H), 7.83 - 7.90 (m, *J* = 8.8 Hz, 2H), 7.63 (dd, *J* = 7.8, 0.9 Hz, 1 H), 7.30 - 7.43 (m, 2 H), 2.57 - 2.74 (m, 4 H), 2.44 - 2.49 (m, 1 H), 2.13 (br dd, *J* = 13.3, 3.0 Hz, 2 H), 1.66 - 1.82 (m, 2 H). |
| | | **ACID:** tetrahydrothiop yran-4-carboxylic acid | |
| | | | MS obsd. (ESI+) [(M+H)+]: 357.1. |
| **31** | ***N*-[4-(7-fluoro-1,3-benzoxazol-2-yl)phenyl]-1,1-dioxo-thietane-3-carboxamide** 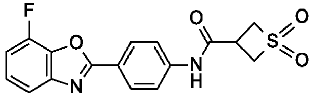 | **AMINE: Int-4** | ¹H NMR (DMSO-*d₆*, 400MHz) δ: 10.68 (s, 1 H), 8.18 - 8.24 (m, 2 H), 7.83 - 7.89 (m, *J* = 8.8 Hz, 2 H), 7.64 (dd, *J* = 7.7, 1.0 Hz, 1 H), 7.33 - 7.44 (m, 2 H), 4.36 - 4.48 (m, 4 H), 3.52 - 3.61 (m, 1 H). |
| | | **ACID:** 1,1-dioxothietane-3-carboxylic acid | |
| | | | MS obsd. (ESI+) [(M+H)+]: 361.1. |
| **32** | ***N*-[4-(7-fluoro-1,3-benzoxazol-2-yl)phenyl]-1-methyl-cyclopropanecarboxamide** 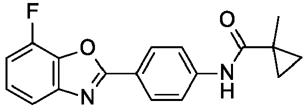 | **AMINE: Int-4** | ¹H NMR (DMSO-*d₆*, 400MHz) δ: 9.53 (s, 1 H), 8.08 - 8.23 (m, *J* = 8.6 Hz, 2 H), 7.85 - 8.01 (m, *J* = 8.6 Hz, 2 H), 7.63 (br d, *J* = 7.7 Hz, 1 H), 7.28 - 7.46 (m, 2 H), 1.44 (s, 3 H), 1.14 (br s, 2 H), 0.62 - 0.76 (m, 2 H). |
| | | **ACID:** 1-methylcyclopr opanecarboxyli c acid | |
| | | | MS obsd. (ESI+) [(M+H)+]: 310.8. |
| **33** | ***N*-[4-(7-fluoro-1,3-benzoxazol-2-yl)phenyl]-3-oxabicyclo[3.1.0]hexane-6-carboxamide** 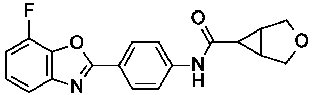 | **AMINE: Int-4** | ¹H NMR (DMSO-*d₆*, 400MHz) δ: 10.57 (s, 1 H), 8.13 - 8.19 (m, 2 H), 7.80 - 7.86 (m, *J* = 8.8 Hz, 2 H), 7.63 (dd, *J* = 7.8, 1.0 Hz, 1 H), 7.31 - 7.43 (m, 2 H), 3.87 (d, *J* = 8.7 Hz, 2 H), 3.68 (d, *J* = 8.4 Hz, 2H), 2.11 - 2.17 (m, 2 H), 1.76 (t, *J* = 3.1 Hz, 1 H). |
| | | **ACID:** 3-oxabicyclo[3.1 .0]hexane-6-carboxylic acid | |
| | | | MS obsd. (ESI+) [(M+H)+]: 339.2. |
| **34** | ***N*-[4-(7-fluoro-1,3-benzoxazol-2-yl)phenyl]-1-methyl-cyclobutanecarboxamide** 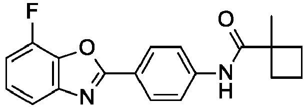 | **AMINE: Int-4** | ¹H NMR (DMSO-*d₆*, 400MHz) δ: 9.75 (s, 1 H), 8.13 - 8.19 (m, *J* = 8.8 Hz, 2 H), 7.92 - 7.97 (m, 2 H), 7.63 (dd, *J* = 7.8, 0.9 Hz, 1 H), 7.31 - 7.43 (m, 2H), 2.41 - 2.48 (m, 2 H), 1.82 - 2.01 (m, 3 H), 1.63 - 1.79 (m, 1 H), 1.47 (s, 3 H). |
| | | **ACID:** 1-methylcyclobu tanecarboxylic acid | |
| | | | MS obsd. (ESI+) [(M+H)+]: 325.3. |
| **35** | ***N*-[4-(7-fluoro-1,3-benzoxazol-2-yl)phenyl]-2-methyl-tetrahydrofuran-2-carboxamide** 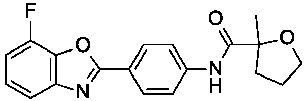 | **AMINE: Int-4** | ¹H NMR (DMSO-*d₆*, 400MHz) δ: 9.99 (s, 1 H), 8.14 - 8.19 (m, *J* = 8.8 Hz, 2 H), 7.99 - 8.06 (m, *J* = 8.8 Hz, 2 H), 7.64 (dd, *J* = 7.8, 0.9 Hz, 1 H), 7.32 - 7.44 (m, 2 H), 3.90 - 4.02 (m, 2 H), 2.27 - 2.38 (m, 1 H), 1.77 - 1.99 (m, 3 H), 1.44 (s, 3 H). |
| | | **ACID:** 2-methyltetrahyd rofuran-2-carboxylic acid | |
| | | | MS obsd. (ESI+) [(M+H)+]: 341.3. |
| **36** | **[1-[[4-(7-fluoro-1,3-benzoxazol-2-yl)phenyl]carbamoyl]cyclopr opyl] acetate** 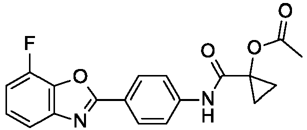 | **AMINE: Int-4** | ¹H NMR (DMSO-*d₆*, 400MHz) δ: 10.03 (s, 1 H), 8.14 - 8.23 (m, *J* = 8.7 Hz, 2 H), 7.85 - 7.96 (m, *J* = 8.7 Hz, 2 H), 7.64 (d, *J* = 7.7 Hz, 1 H), 7.32 - 7.45 (m, 2 H), 2.14 (s, 3 H), 1.41 - 1.57 (m, 2 H), 1.14 - 1.29 (m, 2 H). |
| | | **ACID: Int-16** | |
| | | | MS obsd. (ESI+) [(M+H)+]: 355.3. |
| **37** | **tetrahydrofuran-3-yl *N*-[4-(7-fluoro-1,3-benzoxazol-2-yl)phenyl]carbamate** 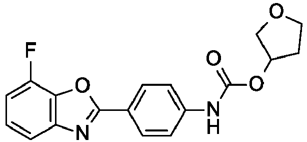 | **AMINE: Int-4** | ¹H NMR (DMSO-*d₆*, 400MHz) δ: 10.24 (s, 1 H), 8.12 - 8.17 (m, 2 H), 7.69 - 7.75 (m, *J* = 8.8 Hz, 2 H), 7.63 (dd, *J* = 7.7, 1.0 Hz, 1 H), 7.31 - 7.43 (m, 2 H), 5.31 (br dd, *J* = 5.5, 2.3 Hz, 1 H), 3.74 - 3.89 (m, 4 H), 2.16 - 2.27 (m, 1 H), 2.00 (ddd, *J* = 11.8, 6.9, 5.1 Hz, 1 H). |
| | | **ACID: Int-17** | |
| | | | MS obsd. (ESI+) [(M+H)+]: 343.3. |
| **38** | **methyl 3-[[4-(7-fluoro-1,3-benzoxazol-2-yl)phenyl]carbamoyl]bicyclo[ 1.1.1]pentane-1-carboxylate** 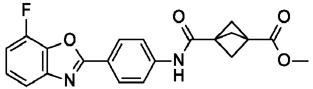 | **AMINE: Int-4** | ¹H NMR (DMSO-*d₆*, 400MHz) δ: 10.04 (s, 1 H), 8.15 - 8.21 (m, 2 H), 7.89 - 7.95 (m, 2 H), 7.63 (dd, *J* = 7.7, 1.0 Hz, 1 H), 7.32 - 7.43 (m, 2 H), 3.65 (s, 3 H), 2.34 (s, 6 H). |
| | | **ACID:** 3-methoxycarbo nylbicyclo[1.1. 1]pentane-1-carboxylic acid | |
| | | | MS obsd. (ESI+) [(M+H)+]: 381.2. |
| **39** | **3-ethyl-*N*-[4-(7-fluoro-1,3-benzoxazol-2-yl)phenyl]oxetane-3-carboxamide** 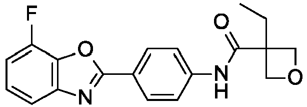 | **AMINE: Int-4** | ¹H NMR (DMSO-*d₆*, 400MHz) δ: 10.11 (s, 1 H), 8.17 - 8.22 (m, 2 H), 7.87 - 7.93 (m, 2 H), 7.64 (dd, *J* = 7.7, 1.0 Hz, 1 H), 7.32 - 7.44 (m, 2 H), 4.82 (d, *J* = 6.2 Hz, 2H), 4.41 (d, *J* = 6.2 Hz, 2 H), 2.09 (q, *J* = 7.3 Hz, 2 H), 0.85 (t, *J* = 7.5 Hz, 3 H). |
| | | **ACID:** 3-ethyloxetane-3-carboxylic acid | |
| | | | MS obsd. (ESI+) [(M+H)+]: 340.9. |
| **40** | ***N*-[4-(4-fluoro-1,3-benzoxazol-2-yl)phenyl]oxazole-2-carboxamide** 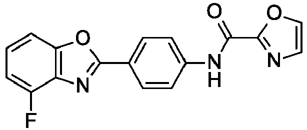 | **AMINE: Int-5** | ¹H NMR (DMSO-*d₆*, 400MHz) |
| | | **ACID:** oxazole-2-carboxylic acid | δ: 11.19 - 11.30 (m, 1 H), 8.44 - 8.48 (m, 1 H), 8.23 (d, *J* = 8.8 Hz, 2 H), 8.11 (d, *J* = 8.8 Hz, 2 H), 7.67 (d, *J* = 8.3 Hz, 1 H), 7.60 (s, 1 H), 7.45 (dt, *J* = 5.1, 8.3 Hz, 1 H), 7.23 - 7.33 (m, 1 H). |
| | | | MS obsd. (ESI+) [(M+H)+]: 324.0. |
| **41** | ***N*-[4-(4-fluoro-1,3-benzoxazol-2-yl)phenyl]-5-methyl-oxazole-2-carboxamide** 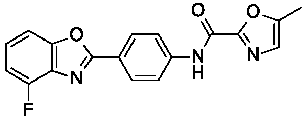 | **AMINE: Int-5** | ¹H NMR (DMSO-*d₆*, 400MHz) δ: 11.13 (s, 1 H), 8.18 - 8.25 (m, 2H), 8.07-8.13 (m, 2 H), 7.63 - 7.70 (m, 1 H), 7.44 (dt, *J* = 5.1, 8.3 Hz, 1 H), 7.29 (dd, *J* = 8.4, 10.2 Hz, 1 H), 7.22 (d, *J* = 1.1 Hz, 1 H), 2.45 (d, *J* = 1.0 Hz, 3 H). |
| | | **ACID:** 5-methyloxazole -2-carboxylic acid | |
| | | | MS obsd. (ESI+) [(M+H)+]: 338.0. |
| **42** | ***N*-[4-(4-fluoro-1,3-benzoxazol-2-yl)phenyl]pyridine-2-carboxamide** 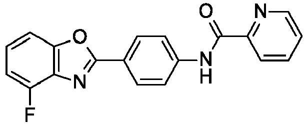 | **AMINE: Int-5** | ¹H NMR (DMSO-*d₆*, 400MHz) δ: 11.04 (s, 1 H), 8.79 (d, *J* = 4.5 Hz, 1 H), 8.17 - 8.29 (m, 5 H), 7.06 - 7.15 (m, 1 H), 7.72 (dd, *J* = 5.1, 6.8 Hz, 1 H), 7.67 (d, *J* = 8.2 Hz, 1 H), 7.44 (dt, *J* = 5.1, 8.2 Hz, 1 H), 7.29 (t, *J* = 9.3 Hz, 1 H). |
| | | **ACID:** pyridine-2-carboxylic acid | |
| | | | MS obsd. (ESI+) [(M+H)+]: 333.9. |
| **43** | ***N*-[4-(4-fluoro-1,3-benzoxazol-2-yl)phenyl]-4-methyl-oxazole-2-carboxamide** 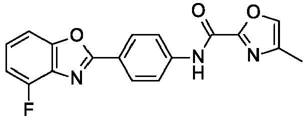 | **AMINE: Int-5** | ¹H NMR (DMSO-*d₆*, 400MHz) δ: 11.14 - 11.27 (m, 1 H), 8.17 - 8.25 (m, 2 H), 8.07 - 8.16 (m, 3 H), 7.66 (d, *J* = 8.2 Hz, 1 H), 7.44 (dt, *J* = 5.1, 8.2 Hz, 1 H), 7.22 - 7.33 (m, 1 H), 2.24 (d, *J* = 0.9 Hz, 3 H). |
| | | **ACID:** 4-methyloxazole -2-carboxylic acid | |
| | | | MS obsd. (ESI+) [(M+H)+]: 338.0. |
| **44** | ***N*-[4-(4-fluoro-1,3-benzoxazol-2-yl)phenyl]thiazole-2-carboxamide** 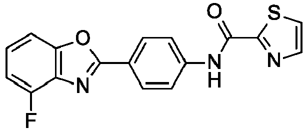 | **AMINE: Int-5** | ¹H NMR (DMSO-*d₆*, 400MHz) δ: 11.11 - 11.28 (m, 1 H), 8.72 - 9.31 (m, 1 H), 8.18 - 8.27 (m, 3 H), 8.11-8.17 (m, 3 H), 7.60-7.69 (m, 1 H), 7.39 - 7.51 (m, 1 H), 7.29 (br t, *J* = 7.8 Hz, 1 H). |
| | | **ACID:** thiazole-2-carboxylic acid | |
| | | | MS obsd. (ESI+) [(M+H)+]: 339.9. |
| **45** | ***N*-[4-(4-fluoro-1,3-benzoxazol-2-yl)phenyl]-2-methyl-oxazole-5-carboxamide** 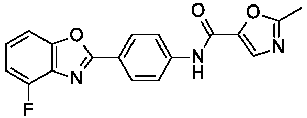 | **AMINE: Int-5** | ¹H NMR (DMSO-*d₆*, 400MHz) δ: 10.68 (s, 1 H), 8.23 (d, *J* = 8.8 Hz, 2 H), 8.01 (d, *J* = 8.8 Hz, 2 H), 7.94 (s, 1 H), 7.67 (d, *J* = 8.2 Hz, 1 H), 7.45 (dt, *J* = 5.1, 8.2 Hz, 1 H), 7.25 - 7.33 (m, 1 H), 2.56 (s, 3 H). |
| | | **ACID:** 2-methyloxazole -5-carboxylic acid | |
| | | | MS obsd. (ESI+) [(M+H)+]: 337.9. |
| **46** | ***N*-[4-(4-fluoro-1,3-benzoxazol-2-yl)phenyl]pyridine-3-carboxamide** 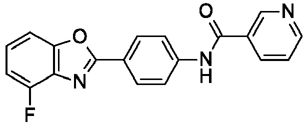 | **AMINE: Int-5** | ¹H NMR (DMSO-*d₆*, 400MHz) δ: 10.80 (s, 1 H), 9.14 (d, *J* = 1.8 Hz, 1 H), 8.80 (dd, *J* = 1.4, 4.8 Hz, 1 H), 8.33 (td, *J* = 1.8, 7.9 Hz, 1 H), 8.23 (d, *J* = 8.8 Hz, 2H), 8.06 (d, *J* = 8.8 Hz, 2 H), 7.65 (d, *J* = 8.2 Hz, 1 H), 7.60 (dd, *J* = 4.9, 7.9 Hz, 1 H), 7.44 (dt, *J* = 5.1, 8.2 Hz, 1 H), 7.23 - 7.33 (m, 1 H). |
| | | **ACID:** nicotinic acid | |
| | | | MS obsd. (ESI+) [(M+H)+]: 334.0. |
| **47** | ***N*-[4-(4-fluoro-1,3-benzoxazol-2-yl)phenyl]bicyclo[1.1.1]penta ne-1-carboxamide** 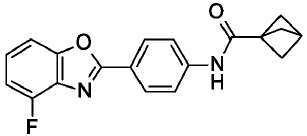 | **AMINE: Int-5** | ¹H NMR (DMSO-*d₆*, 400MHz) δ: 9.88 (s, 1 H), 8.14 - 8.20 (m, 2 H), 7.90 - 7.96 (m, *J* = 8.8 Hz, 2 H), 7.64 (d, *J* = 8.2 Hz, 1 H), 7.43 (td, *J* = 8.3, 5.1 Hz, 1 H), 7.28 (dd, *J* = 9.8, 8.4 Hz, 1 H), 2.12 (s, 6 H). |
| | | **ACID:** bicyclo[1.1.1]p entane-1-carboxylic acid | |
| | | | MS obsd. (ESI+) [(M+H)+]: 323.3. |
| **48** | **3-fluoro-*N*-[4-(4-fluoro-1,3-benzoxazol-2-yl)phenyl]bicyclo[1.1.1]pentane-1-carboxamide** 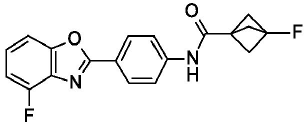 | **AMINE: Int-5** | ¹H NMR (DMSO-*d₆*, 400MHz) δ: 10.07 (s, 1 H), 8.16 - 8.22 (m, 2 H), 7.88 - 7.94 (m, 2 H), 7.65 (d, *J* = 7.9 Hz, 1 H), 7.43 (td, *J* = 8.3, 5.1 Hz, 1 H), 7.28 (dd, *J* = 9.9, 8.6 Hz, 1 H), 2.44 (d, *J* = 2.6 Hz, 6 H). |
| | | **ACID:** 3-fluorobicyclo[ 1.1.1]pentane-1-carboxylic acid | |
| | | | MS obsd. (ESI+) [(M+H)+]: 341.0. |
| **49** | ***N*-[4-(4-fluoro-1,3-benzoxazol-2-yl)phenyl]-2-hydroxy-2-methyl-propanamide** 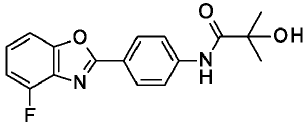 | **AMINE: Int-5** | ¹H NMR (DMSO-*d₆*, 400MHz) δ: 9.99 (s, 1 H), 8.13 - 8.18 (m, 2 H), 8.01 - 8.06 (m, 2 H), 7.65 (dd, *J* = 8.2, 0.7 Hz, 1 H), 7.43 (td, *J* = 8.2, 5.1 Hz, 1 H), 7.28 (dd, *J* = 9.7, 8.4 Hz, 1 H), 5.83 (s, 1 H), 1.38 (s, 6 H). |
| | | **ACID:** 2-hydroxy-2-methyl-propanoic acid | |
| | | | MS obsd. (ESI+) [(M+H)+]: 314.9. |
| **50** | **N-[4-(4-fluoro-1,3-benzoxazol-2-yl)phenyl]-1,1-dioxo-thiolane-3-carboxamide** 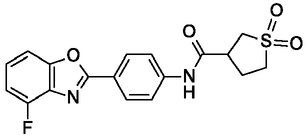 | **AMINE: Int-5** | ¹H NMR (DMSO-*d₆*, 400MHz) δ: 10.59 (s, 1 H), 8.16 - 8.22 (m, 2 H), 7.83 - 7.89 (m, 2 H), 7.65 (d, *J* = 8.2 Hz, 1 H), 7.43 (td, *J* = 8.3, 5.1 Hz, 1 H), 7.28 (dd, *J* = 9.8, 8.5 Hz, 1 H), 3.39 - 3.52 (m, 2 H), 3.12-3.32 (m, 3 H), 2.43 - 2.48 (m, 1 H), 2.20 - 2.30 (m, 1 H). |
| | | **ACID:** 1,1-dioxothiolane-3-carboxylic acid | |
| | | | MS obsd. (ESI+) [(M+H)+]: 375.2. |
| **51** | ***N*-[4-(4-fluoro-1,3-benzoxazol-2-yl)phenyl]-3-methyl-oxetane-3-carboxamide** 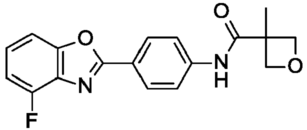 | **AMINE: Int-5** | ¹H NMR (DMSO-*d₆*, 400MHz) δ: 10.14 (s, 1 H), 8.16 - 8.22 (m, 2 H), 7.88 - 7.93 (m, 2 H), 7.65 (d, *J* = 8.2 Hz, 1 H), 7.43 (td, *J* = 8.2, 5.1 Hz, 1 H), 7.28 (dd, *J* = 10.0, 8.5 Hz, 1 H), 4.86 (d, *J* = 6.1 Hz, 2 H), 4.38 (d, *J* = 6.1 Hz, 2 H), 1.64 (s, 3 H). |
| | | **ACID:** 3-methyloxetane -3-carboxylic acid | |
| | | | MS obsd. (ESI+) [(M+H)+]: 327.2. |
| **52** | ***N*-[4-(4-fluoro-1,3-benzoxazol-2-yl)phenyl] -2-hydroxy-propanamide** 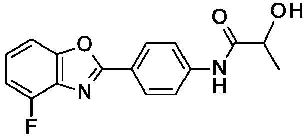 | **AMINE: Int-5** | ¹H NMR (DMSO-*d₆*, 400MHz) δ: 10.07 (s, 1 H), 8.14 - 8.19 (m, 2 H), 7.97 - 8.02 (m, 2 H), 7.65 (dd, *J* = 8.2, 0.7 Hz, 1 H), 7.43 (td, *J* = 8.3, 5.1 Hz, 1 H), 7.28 (dd, *J* = 9.7, 8.4 Hz, 1 H), 5.83 (d, *J* = 5.1 Hz, 1 H), 4.16 - 4.24 (m, 1 H), 1.34 (d, *J* = 6.7 Hz, 3 H). |
| | | **ACID:** 2-hydroxypropan oic acid | |
| | | | MS obsd. (ESI+) [(M+H)+]: 301.2. |
| **53** | ***N*-[4-(4-fluoro-1,3-benzoxazol-2-yl)phenyl] -2,2-dimethyl-propanamide** 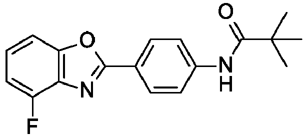 | **AMINE: Int-5** | ¹H NMR (DMSO-*d₆*, 400MHz) δ: 9.57 (s, 1 H), 8.13 - 8.19 (m, 2 H), 7.92 - 7.98 (m, 2 H), 7.64 (dd, *J* = 8.2, 0.6 Hz, 1 H), 7.43 (td, *J* = 8.3, 5.1 Hz, 1 H), 7.29 (t, *J* = 9.2 Hz, 1 H), 1.26 (s, 9 H). |
| | | **ACYL CHLORIDE:** 2,2-dimethylpropa noyl chloride | |
| | | | MS obsd. (ESI+) [(M+H)+]: 313.3. |
| **54** | ***N*-[4-(4-fluoro-1,3-benzoxazol-2-yl)phenyl]tetrahydrofuran-3-carboxamide** 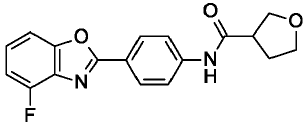 | **AMINE: Int-5** | ¹H NMR (DMSO-*d₆*, 400MHz) δ: 10.43 (s, 1 H), 8.13 - 8.21 (m, 2 H), 7.83 - 7.89 (m, 2 H), 7.65 (d, *J* = 7.9 Hz, 1 H), 7.43 (td, *J* = 8.3, 5.1 Hz, 1 H), 7.28 (dd, *J* = 9.8, 8.4 Hz, 1 H), 3.96 (t, *J* = 8.2 Hz, 1 H), 3.70 - 3.84 (m, 3 H), 3.18 - 3.25 (m, 1 H), 2.06 - 2.18 (m, 2 H). |
| | | **ACID:** tetrahydrofura n-3-carboxylic acid | |
| | | | MS obsd. (ESI+) [(M+H)+]: 327.4. |
| **55** | ***N*-[4-(4-fluoro-1,3-benzoxazol-2-yl)phenyl]-1,1-dioxo-thiane-4-carboxamide** 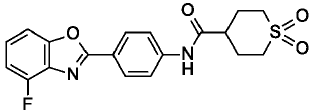 | **AMINE: Int-5** | ¹H NMR (DMSO-*d₆*, 400MHz) δ: 10.46 (s, 1 H), 8.14 - 8.20 (m, 2 H), 7.83 - 7.89 (m, 2 H), 7.65 (d, *J* = 8.3 Hz, 1 H), 7.43 (td, *J* = 8.3, 5.1 Hz, 1 H), 7.28 (dd, *J* = 9.9, 8.6 Hz, 1 H), 3.14 - 3.31 (m, 4 H), 2.70 - 2.77 (m, 1 H), 2.07 - 2.28 (m, 4 H). |
| | | **ACID:** 1,1-dioxothiane-4-carboxylic acid | |
| | | | MS obsd. (ESI+) [(M+H)+]: 389.3. |
| **56** | ***N*-[4-(4-fluoro-1,3-benzoxazol-2-yl)phenyl]-1-methyl-3-oxo-cyclobutanecarboxamide** 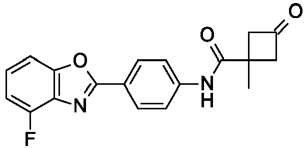 | **AMINE: Int-5** | ¹H NMR (DMSO-*d₆*, 400MHz) δ: 10.16 (s, 1 H), 8.15 - 8.23 (m, *J* = 8.8 Hz, 2 H), 7.91 - 7.99 (m, *J* = 8.8 Hz, 2 H), 7.65 (d, *J* = 8.1 Hz, 1 H), 7.43 (td, *J* = 8.2, 5.1 Hz, 1 H), 7.29 (t, *J* = 9.2 Hz, 1 H), 3.56 - 3.68 (m, 2 H), 2.90 - 3.00 (m, 2 H), 1.65 (s, 3 H). |
| | | **ACID:** 1-methyl-3-oxo-cyclobutanecar boxylic acid | |
| | | | MS obsd. (ESI+) [(M+H)+]: 339.2. |
| **57** | ***N*-[4-(4-fluoro-1,3-benzoxazol-2-yl)phenyl]-1,1-dioxo-thietane-3-carboxamide** 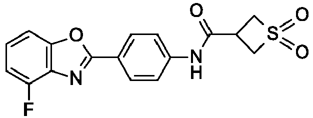 | **AMINE: Int-5** | ¹H NMR (DMSO-*d₆*, 400MHz) δ: 10.67 (s, 1 H), 8.16 - 8.25 (m, 2 H), 7.83 - 7.89 (m, *J* = 8.8 Hz, 2 H), 7.65 (dd, *J* = 8.2, 0.6 Hz, 1 H), 7.44 (td, *J* = 8.3, 5.1 Hz, 1 H), 7.29 (dd, *J* = 9.9, 8.6 Hz, 1 H), 4.36 - 4.49 (m, 4 H), 3.52 - 3.62 (m, 1 H). |
| | | **ACID:** 1,1-dioxothietane-3-carboxylic acid | |
| | | | MS obsd. (ESI+) [(M+H)+]: 361.1. |
| **58** | **methyl *N*-[4-(4-fluoro-1,3-benzoxazol-2-yl)phenyl]carbamate** 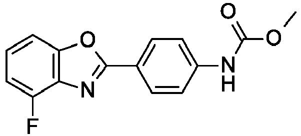 | **AMINE: Int-5** | ¹H NMR (DMSO-*d₆*, 400MHz) δ: 10.16 (s, 1 H), 8.15 (d, *J* = 8.8 Hz, 2 H), 7.64 - 7.73 (m, 3 H), 7.43 (t, *J* = 8.8 Hz, 1 H), 7.27 (t, *J* = 9.2 Hz, 1 H), 3.34 (s, 3 H). |
| | | **ACYL CHLORIDE:** methyl carbonochlorid | |
| | | ate | MS obsd. (ESI+) [(M+H)+]: 287.0. |
| **59** | **ethyl *N*-[4-(4-fluoro-1,3-benzoxazol-2-yl)phenyl]carbamate** 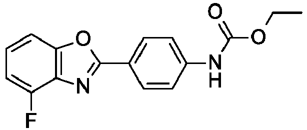 | **AMINE: Int-5** | ¹H NMR (DMSO-*d₆*, 400MHz) δ: 10.11 (s, 1 H), 8.08 - 8.19 (m, 2 H), 7.70 (d, *J* = 8.5 Hz, 2 H), 7.60 - 7.66 (m, 1 H), 7.37 - 7.45 (m, 1 H), 7.22 - 7.30 (m, 1 H), 4.17 (q, *J* = 7.1 Hz, 2H), 1.27 (t, *J* = 7.0 Hz, 3 H). |
| | | **ACYL CHLORIDE:** ethyl carbonochlorid ate | |
| | | | MS obsd. (ESI+) [(M+H)+]: 301.1. |
| **60** | **isopropyl *N*-[4-(4-fluoro-1,3-benzoxazol-2-yl)phenyl]carbamate** 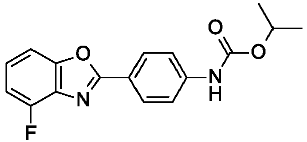 | **AMINE: Int-5** | ¹H NMR (DMSO-*d₆*, 400MHz) δ: 8.17 - 8.32 (m, 2 H), 7.59 (d, *J* = 8.8 Hz, 2 H), 7.40 (dd, *J* = 0.8, 8.2 Hz, 1 H), 7.29 - 7.34 (m, 1 H), 7.09 (dt, *J* = 0.7, 9.0 Hz, 1 H), 6.77 (s, 1 H), 4.95 - 5.16 (m, 1 H), 1.35 (d, *J* = 6.3 Hz, 6 H). |
| | | **ACYL CHLORIDE:** isopropyl carbonochlorid ate | |
| | | | MS obsd. (ESI+) [(M+H)+]: 315.1. |
| **61** | **2,2-dimethyl-*N*-(4-oxazolo[5,4-b]pyridin-2-ylphenyl)propanamide** 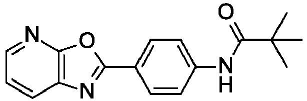 | **AMINE: Int-6** | ¹H NMR (DMSO-*d₆*, 400MHz) δ: 9.59 (s, 1 H), 8.36 (dd, *J* = 4.9, 1.5 Hz, 1 H), 8.15 - 8.25 (m, 3 H), 7.96 (d, *J* = 8.1 Hz, 2 H), 7.49 (dd, *J* = 7.8, 5.0 Hz, 1 H), 1.26 (s, 9 H). |
| | | **ACYL CHLORIDE:** 2,2-dimethylpropa noyl chloride | |
| | | | MS obsd. (ESI+) [(M+H)+]: 295.8. |
| **62** | **2-methoxy-*N*-(4-oxazolo[5,4-b]pyridin-2-ylphenyl)propanamide** 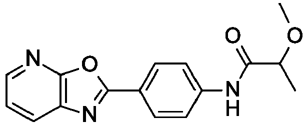 | **AMINE:Int-6** | ¹H NMR (DMSO-*d₆*, 400MHz) δ: 10.25 (s, 1 H), 8.36 (dd, *J* = 5.0, 1.5 Hz, 1 H), 8.17 - 8.26 (m, 3 H), 7.98 (d, *J* = 8.0 Hz, 2 H), 7.50 (dd, *J* = 7.8, 4.9 Hz, 1 H), 3.93 (q, *J* = 6.6 Hz, 1 H), 3.34 (s, 3 H), 1.35 (d, *J* = 6.7 Hz, 3 H). |
| | | **ACID:** 2-methoxypropa noic acid | |
| | | | MS obsd. (ESI+) [(M+H)+]: 298.2. |
| **63** | ***N*-(4-oxazolo[5,4-b]pyridin-2-ylphenyl)-1,1-dioxo-thiolane-3-carboxamide** 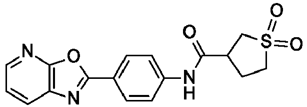 | **AMINE: Int-6** | ¹H NMR (DMSO-*d₆*, 400MHz) δ: 10.61 (s, 1 H), 8.36 (dd, *J* = 4.9, 1.6 Hz, 1 H), 8.19 - 8.26 (m, 3 H), 7.87 (d, *J* = 8.8 Hz, 2 H), 7.50 (dd, *J* = 7.8, 4.9 Hz, 1 H), 3.39 - 3.52 (m, 2 H), 3.12 - 3.31 (m, 3 H), 2.44 - 2.48 (m, 1 H), 2.19 - 2.32 (m, 1 H). |
| | | **ACID:** 1,1-dioxothiolane-3-carboxylic acid | |
| | | | MS obsd. (ESI+) [(M+H)+]: 358.1. |
| **64** | **3-methyl-*N*-(4-oxazolo[5,4-b]pyridin-2-ylphenyl)tetrahydrofuran-3-carboxamide** 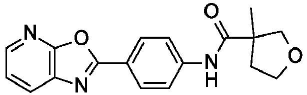 | **AMINE: Int-6** | ¹H NMR (DMSO-*d₆*, 400MHz) δ: 9.87 (s, 1 H), 8.36 (dd, *J* = 5.0, 1.5 Hz, 1 H), 8.16 - 8.25 (m, 3 H), 7.93 (d, *J* = 8.8 Hz, 2 H), 7.49 (dd, *J* = 7.8, 4.9 Hz, 1 H), 4.12 (d, *J* = 8.7 Hz, 1 H), 3.76 - 3.85 (m, 2 H), 3.52 (d, *J* = 8.6 Hz, 1 H), 2.44 - 2.49 (m, 1 H), 1.76 - 1.87 (m, 1 H), 1.43 (s, 3 H). |
| | | **ACID:** 3-methyltetrahyd rofuran-3-carboxylic acid | |
| | | | MS obsd. (ESI+) [(M+H)+]: 324.2. |
| **65** | **3-methyl-*N*-(4-oxazolo[5,4-b]pyridin-2-ylphenyl)oxetane-3-carboxamide** 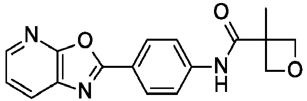 | **AMINE: Int-6** | ¹H NMR (DMSO-*d₆*, 400MHz) δ: 10.15 (s, 1 H), 8.36 (dd, *J* = 5.0, 1.5 Hz, 1 H), 8.18 - 8.25 (m, 3 H), 7.91 (d, *J* = 8.2 Hz, 2 H), 7.50 (dd, *J* = 7.8, 4.9 Hz, 1 H), 4.87 (d, *J* = 6.0 Hz, 2 H), 4.38 (d, *J* = 6.1 Hz, 2 H), 1.64 (s, 3 H). |
| | | **ACID:** 3-methyloxetane -3-carboxylic acid | |
| | | | MS obsd. (ESI+) [(M+H)+]: 310.3. |
| **66** | ***N*-(4-oxazolo[5,4-b]pyridin-2-ylphenyl)-1,1-dioxo-thiane-4-carboxamide** 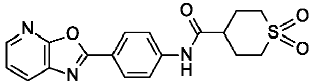 | **AMINE: Int-6** | ¹H NMR (DMSO-*d₆*, 400MHz) δ: 10.47 (s, 1 H), 8.36 (dd, *J* = 4.9, 1.6 Hz, 1 H), 8.16 - 8.25 (m, 3 H), 7.87 (d, *J* = 8.9 Hz, 2 H), 7.49 (dd, *J* = 7.8, 4.9 Hz, 1 H), 3.14 - 3.30 (m, 4 H), 2.68 - 2.79 (m, 1 H), 2.07 - 2.28 (m, 4 H). |
| | | **ACID:** 1,1-dioxothiane-4-carboxylic acid | |
| | | | MS obsd. (ESI+) [(M+H)+]: 372.1. |
| **67** | ***N*-(4-oxazolo[5,4-b]pyridin-2-ylphenyl)tetrahydrofuran-3-carboxamide** 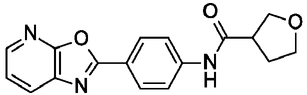 | **AMINE: Int-6** | ¹H NMR (DMSO-*d₆*, 400MHz) δ: 10.45 (s, 1 H), 8.36 (dd, *J* = 4.9, 1.6 Hz, 1 H), 8.16 - 8.25 (m, 3 H), 7.87 (d, *J* = 8.1 Hz, 2 H), 7.49 (dd, *J* = 7.8, 5.0 Hz, 1 H), 3.96 (t, *J* = 8.2 Hz, 1 H), 3.70 - 3.83 (m, 3 H), 3.17 - 3.26 (m, 1 H), 2.05 - 2.18 (m, 2 H). |
| | | **ACID:** tetrahydrofura n-3-carboxylic acid | |
| | | | MS obsd. (ESI+) [(M+H)+]: 310.2. |
| **68** | ***N*-(4-oxazolo[5,4-b]pyridin-2-ylphenyl)oxetane-3-carboxamide** 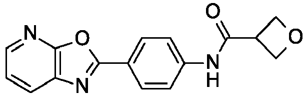 | **AMINE: Int-6** | ¹H NMR (DMSO-*d₆*, 400MHz) δ: 10.36 (s, 1 H), 8.36 (dd, *J* = 5.0, 1.5 Hz, 1 H), 8.18 - 8.25 (m, 3 H), 7.89 (d, *J* = 8.8 Hz, 2 H), 7.49 (dd, *J* = 7.8, 4.9 Hz, 1 H), 4.69 - 4.76 (m, 4 H), 3.98 - 4.11 (m, 1 H). |
| | | **ACID:** oxetane-3-carboxylic acid | |
| | | | MS obsd. (ESI+) [(M+H)+]: 296.2. |
| **69** | ***N*-[4-(4,5-difluoro-1,3-benzoxazol-2-yl)phenyl]tetrahydrofuran-3-carboxamide** 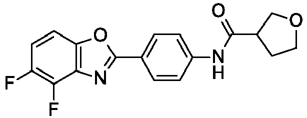 | **AMINE: Int-7** | ¹H NMR (DMSO-*d₆*, 400MHz) δ: 10.43 (s, 1 H), 8.14 - 8.21 (m, 2 H), 7.83 - 7.89 (m, 2 H), 7.66 (ddd, *J* = 9.0, 3.4, 1.3 Hz, 1 H), 7.50 (ddd, *J* = 11.5, 8.9, 7.6 Hz, 1 H), 3.92 - 3.99 (m, 1 H), 3.70 - 3.83 (m, 3 H), 3.16 - 3.26 (m, 1 H), 2.05 - 2.18 (m, 2 H). |
| | | **ACID:** tetrahydrofura n-3-carboxylic acid | |
| | | | MS obsd. (ESI+) [(M+H)+]: 345.2. |
| **70** | ***N*-[4-(5,7-difluoro-1,3-benzoxazol-2-yl)phenyl]tetrahydrofuran-3-carboxamide** 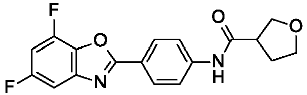 | **AMINE: Int-8** | ¹H NMR (DMSO-*d₆*, 400MHz) δ: 10.44 (s, 1 H), 8.13 - 8.19 (m, *J* = 8.8 Hz, 2 H), 7.83 - 7.89 (m, *J* = 8.8 Hz, 2H), 7.57 (dd, *J* = 8.3, 2.1 Hz, 1 H), 7.44 (td, *J* = 10.3, 2.3 Hz, 1 H), 3.92 - 4.00 (m, 1 H), 3.69 - 3.84 (m, 3 H), 3.21 (quin, *J* = 7.5 Hz, 1 H), 2.05 - 2.18 (m, 2 H). |
| | | **ACID:** tetrahydrofura n-3-carboxylic acid | |
| | | | MS obsd. (ESI+) [(M+H)+]: 345.1. |
| **71** | ***N*-[4-(5-methyloxazolo[5,4-b]pyridin-2-yl)phenyl]tetrahydrofuran-3-carboxamide** 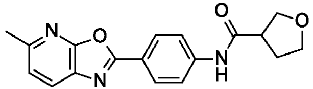 | **AMINE: Int-9** | ¹H NMR (DMSO-*d₆*, 400MHz) δ: 10.42 (s, 1 H), 8.13 (d, *J* = 8.0 Hz, 2 H), 8.08 (d, *J* = 7.9 Hz, 1 H), 7.85 (d, *J* = 8.7 Hz, 2 H), 7.33 (d, *J* = 7.9 Hz, 1 H), 3.96 (t, *J* = 8.2 Hz, 1 H), 3.68 - 3.85 (m, 3 H), 3.21 (quin, *J* = 7.5 Hz, 1 H), 2.58 (s, 3 H), 2.06 - 2.16 (m, 2 H). |
| | | **ACID:** tetrahydrofura n-3-carboxylic acid | |
| | | | MS obsd. (ESI+) [(M+H)+]: 324.1. |
| **72** | ***N*-[4-(5-methoxyoxazolo[5,4-b]pyridin-2-yl)phenyl]tetrahydrofuran-3-carboxamide** 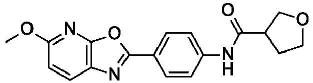 | **AMINE: Int-10** | ¹H NMR (DMSO-*d₆*, 400MHz) δ: 10.39 (s, 1 H), 8.11 (d, *J* = 8.6 Hz, 1 H), 8.07 (d, *J* = 8.8 Hz, 2 H), 7.82 (d, *J* = 8.8 Hz, 2 H), 6.88 (d, *J* = 8.6 Hz, 1 H), 3.94 - 3.99 (m, 1 H), 3.93 (s, 3 H), 3.69 - 3.84 (m, 3 H), 3.21 (quin, *J* = 7.5 Hz, 1 H), 2.06 - 2.16 (m, 2 H). |
| | | **ACID:** tetrahydrofura n-3-carboxylic acid | |
| | | | MS obsd. (ESI+) [(M+H)+]: 340.0. |
| **73** | ***N*-[4-(5-chloro-1,3-benzoxazol-2-yl)phenyl]oxazole-2-carboxamide** 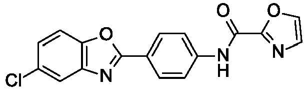 | **AMINE: Int-11** | ¹H NMR (DMSO-*d₆*, 400MHz) δ: 11.23 (s, 1 H), 8.46 (s, 1 H), 8.17 - 8.23 (m, 2 H), 8.07 - 8.13 (m, 2 H), 7.91 (d, *J* = 2.1 Hz, 1 H), 7.83 (d, *J* = 8.7 Hz, 1 H), 7.60 (s, 1 H), 7.46 (dd, *J* = 8.6, 2.1 Hz, 1 H). |
| | | **ACID:** oxazole-2-carboxylic acid | |
| | | | MS obsd. (ESI+) [(M+H)+]: 340.1. |
| **74** | ***N*-[4-(5-chloro-1,3-benzoxazol-2-yl)phenyl]pyridine-2-carboxamide** 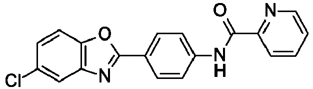 | **AMINE: Int-11** | ¹H NMR (DMSO-*d₆*, 400MHz) δ: 11.03 (s, 1 H), 8.78 (d, *J* = 4.3 Hz, 1 H), 8.18 - 8.23 (m, 5 H), 8.10 (td, *J* = 7.7, 1.6 Hz, 1 H), 7.91 (d, *J* = 2.0 Hz, 1 H), 7.83 (d, *J* = 8.7 Hz, 1 H), 7.72 (t, *J* = 6.1 Hz, 1 H), 7.46 (dd, *J* = 8.7, 2.1 Hz, 1 H). |
| | | **ACID:** pyridine-2-carboxylic acid | |
| | | | MS obsd. (ESI+) [(M+H)+]: 350.2. |
| **75** | ***N*-[4-(5-chloro-1,3-benzoxazol-2-yl)phenyl]tetrahydrofuran-3-carboxamide** 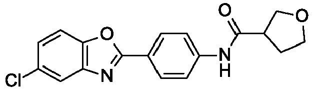 | **AMINE: Int-11** | ¹H NMR (DMSO-*d₆*, 400MHz) δ: 10.42 (s, 1 H), 8.15 (d, *J* = 8.1 Hz, 2 H), 7.79 - 7.89 (m, 4 H), 7.45 (dd, *J* = 8.7, 2.2 Hz, 1 H), 3.92 - 3.99 (m, 1 H), 3.69 - 3.83 (m, 3 H), 3.16 - 3.25 (m, 1 H), 2.05 - 2.17 (m, 2 H). |
| | | **ACID:** tetrahydrofura n-3-carboxylic acid | |
| | | | MS obsd. (ESI+) [(M+H)+]: 343.0. |
| **76** | ***N*-[4-(5-chloro-1,3-benzoxazol-2-yl)phenyl]bicyclo[1.1.1]penta ne-1-carboxamide** 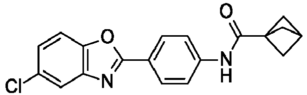 | **AMINE: Int-11** | ¹H NMR (DMSO-*d₆*, 400MHz) δ: 9.88 (s, 1 H), 8.15 (d, *J* = 7.4 Hz, 2 H), 7.87 - 7.95 (m, 3 H), 7.80 (d, *J* = 8.6 Hz, 1 H), 7.45 (dd, J = 8.7, 2.1 Hz, 1 H), 2.47 - 2.49 (m, 1 H), 2.11 (s, 6 H). |
| | | **ACID:** bicyclo[1.1.1]p entane-1-carboxylic acid | |
| | | | MS obsd. (ESI+) [(M+H)+]: 339.1. |
| **77** | ***N*-[4-(5-chloro-1,3-benzoxazol-2-yl)phenyl]-2-hydroxy-propanamide** 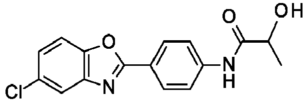 | **AMINE: Int-11** | ¹H NMR (DMSO-*d₆*, 400MHz) δ: 10.07 (s, 1 H), 8.11 - 8.17 (m, 2 H), 7.97 - 8.02 (m, 2 H), 7.79 - 7.91 (m, 2 H), 7.43 - 7.47 (m, 1 H), 5.83 (d, *J* = 5.0 Hz, 1 H), 4.15 - 4.24 (m, 1 H), 1.33 (d, *J* = 6.7 Hz, 3 H). |
| | | **ACID:** 2-hydroxypropan oic acid | |
| | | | MS obsd. (ESI+) [(M+H)+]: 316.9. |
| **78** | ***N*-[4-(6,7-difluoro-1,3-benzoxazol-2-yl)phenyl]-5-methyl-oxazole-2-carboxamide** 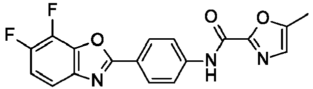 | **AMINE: Int-12** | ¹H NMR (DMSO-*d₆*, 400MHz) δ: 11.14 (s, 1 H), 8.16 - 8.25 (m, 2 H), 8.03 - 8.14 (m, 2 H), 7.66 (m, 1 H), 7.51 (m, 1 H), 7.23 (d, *J* = 1.1 Hz, 1 H), 2.45 (d, *J* = 1.0 Hz, 3 H). |
| | | **ACID:** 5-methyloxazole -2-carboxylic acid | |
| | | | MS obsd. (ESI+) [(M+H)+]: 355.9. |
| **79** | ***N*-[4-(6,7-difluoro-1,3-benzoxazol-2-yl)phenyl]-4-methyl-oxazole-2-carboxamide** 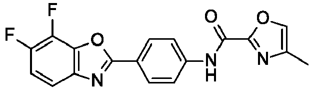 | **AMINE: Int-12** | ¹H NMR (DMSO-*d₆*, 400MHz) δ: 11.20 (s, 1 H), 8.08 - 8.19 (m, 5 H), 7.63 - 7.66 (m, 1 H), 7.49 - 7.52 (m, 1 H), 2.24 (s, 3 H). |
| | | **ACID:** 4-methyloxazole -2-carboxylic acid | |
| | | | MS obsd. (ESI+) [(M+H)+]: 356.0. |
| | | | |
| **80** | ***N*-[4-(6,7-difluoro-1,3-benzoxazol-2-yl)phenyl]-5-methyl-1,3,4-oxadiazole-2-carboxamide** 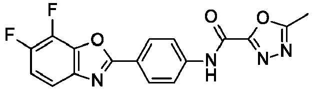 | **AMINE: Int-12** | ¹H NMR (DMSO-*d₆*, 400MHz) δ: 11.54 (br, s, 1 H), 8.06 - 8.21 (m, 4 H), 7.64 - 7.67 (m, 1 H), 7.48 - 7.51 (m, 1 H), 2.64 (s, 3 H). |
| | | **ACID:** 5-methyl-1,3,4-oxadiazole-2-carboxylic acid | |
| | | | MS obsd. (ESI+) [(M+H)+]: 357.0. |
| **81** | ***N*-[4-(6,7-difluoro-1,3-benzoxazol-2-yl)phenyl]-2-methyl-oxazole-5-carboxamide** 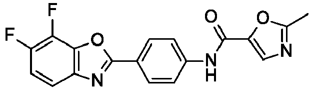 | **AMINE: Int-12** | ¹H NMR (DMSO-*d₆*, 400MHz) δ: 10.63 (s, 1 H), 8.18 (d, *J* = 8.8 Hz, 2 H), 7.99 (d, *J* = 8.8 Hz, 2 H), 7.91 (s, 1 H), 7.63 (dd, *J* = 3.4, 8.4 Hz, 1 H), 7.48 (m, 1 H), 2.55 (s, 3 H). |
| | | **ACID:** 2-methyloxazole -5-carboxylic acid | |
| | | | MS obsd. (ESI+) [(M+H)+]: 355.9. |
| **82** | ***N*-[4-(6,7-difluoro-1,3-benzoxazol-2-yl)phenyl]tetrahydrofuran-3-carboxamide** 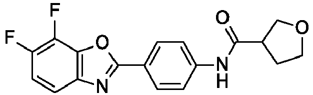 | **AMINE: Int-12** | ¹H NMR (DMSO-*d₆*, 400MHz) δ: 10.43 (s, 1 H), 8.12 - 8.20 (m, 2 H), 7.83 - 7.88 (m, 2 H), 7.63 (ddd, *J* = 8.8, 3.9, 1.3 Hz, 1 H), 7.41 - 7.54 (m, 1 H), 3.96 (t, *J* = 8.2 Hz, 1 H), 3.70 - 3.83 (m, 3 H), 3.21 (quin, *J* = 7.5 Hz, 1 H), 2.05 - 2.18 (m, 2 H). |
| | | **ACID:** tetrahydrofura n-3-carboxylic acid | |
| | | | MS obsd. (ESI+) [(M+H)+]: 345.1. |
| **83** | ***N*-[4-(6,7-difluoro-1,3-benzoxazol-2-yl)phenyl]-1,1-dioxo-thiolane-3-carboxamide** 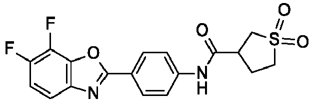 | **AMINE: Int-12** | ¹H NMR (DMSO-*d₆*, 400MHz) δ: 10.60 (s, 1 H), 8.15 - 8.20 (m, 2 H), 7.83 - 7.89 (m, 2 H), 7.64 (ddd, *J* = 8.8, 4.0, 1.3 Hz, 1 H), 7.50 (ddd, *J* = 11.6, 8.8, 7.3 Hz, 1 H), 3.38 - 3.51 (m, 2 H), 3.11 - 3.32 (m, 3 H), 2.38 - 2.48 (m, 1 H), 2.19 - 2.32 (m, 1 H). |
| | | **ACID:** 1,1-dioxothiolane-3-carboxylic acid | |
| | | | MS obsd. (ESI+) [(M+H)+]: 393.1. |
| **84** | ***N*-[4-(4,6-difluoro-1,3-benzoxazol-2-yl)phenyl]tetrahydrofuran-3-carboxamide** 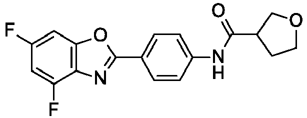 | **AMINE: Int-13** | ¹H NMR (DMSO-*d₆*, 400MHz) δ: 10.43 (s, 1 H), 8.10 - 8.16 (m, 2 H), 7.83 - 7.88 (m, 2 H), 7.71 (dd, *J* = 8.1, 1.7 Hz, 1 H), 7.38 (td, *J* = 10.5, 2.2 Hz, 1 H), 3.96 (t, *J* = 8.2 Hz, 1 H), 3.69 - 3.83 (m, 3 H), 3.16 - 3.26 (m, 1 H), 2.04 - 2.18 (m, 2 H). |
| | | **ACID:** tetrahydrofura n-3-carboxylic acid | |
| | | | MS obsd. (ESI+) [(M+H)+]: 345.2. |
| **85** | ***N*-[4-(7-chloro-1,3-benzoxazol-2-yl)phenyl]-1,1-dioxo-thiolane-3-carboxamide** 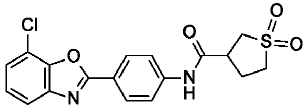 | **AMINE: Int-14** | ¹H NMR (DMSO-*d₆*, 400MHz) δ: 10.61 (s, 1 H), 8.16 - 8.21 (m, *J* = 8.8 Hz, 2 H), 7.84 - 7.90 (m, *J* = 8.8 Hz, 2H), 7.76 (dd, *J* = 7.8, 0.9 Hz, 1 H), 7.52 (dd, *J* = 8.0, 0.9 Hz, 1 H), 7.42 (t, *J* = 7.9 Hz, 1 H), 3.39 - 3.52 (m, 2 H), 3.12 - 3.3 1 (m, 3 H), 2.44 - 2.48 (m, 1 H), 2.19 - 2.29 (m, 1 H). |
| | | **ACID:** 1,1-dioxothiolane-3-carboxylic acid | |
| | | | MS obsd. (ESI+) [(M+H)+]: 391.1. |

### Example 86-a, Example 86-b

### (3R)-N-(4-oxazolo[5,4-b]pyridin-2-ylphenyl)-1,1-dioxo-thiolane-3-carboxamide

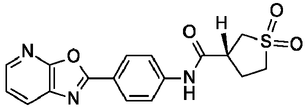

### (3S)-N-(4-oxazolo[5,4-b]pyridin-2-ylphenyl)-1,1-dioxo-thiolane-3-carboxamide

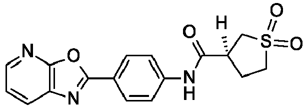

The two enantiomers **(Example 86-a, Example 86-b)** were obtained through SFC [Instrument: Thar 200 preparative SFC (SFC-7), Phenomenex Lux Cellulose-2, 300×50 mm I.D., 10 µm; Mobile phase: A for CO₂ and B for MEOH; Gradient: B 50%; Flow rate: 200 mL /min; Back pressure: 100 bar; Column temperature: 38 °C; elution order was **Example 86-a, Example 86-b]** chiral separation of *N*-(4-oxazolo[5,4-b]pyridin-2-ylphenyl)-1,1-dioxo-thiolane-3-carboxamide **(Example 63).**
**Example 86-a:** MS obsd. (ESI⁺) [(M+H)⁺]: 358.1. ¹H NMR (DMSO-*d₆*, 400MHz) δ: 10.60 (s, 1 H), 8.36 (d, *J* = 5.0 Hz, 1 H), 8.18 - 8.26 (m, 3 H), 7.87 (d, *J* = 8.7 Hz, 2 H), 7.47 - 7.52 (m, 1 H), 3.39 - 3.52 (m, 2 H), 3.12 - 3.33 (m, 3 H), 2.44 - 2.48 (m, 1 H), 2.19 - 2.31 (m, 1 H).
**Example 86-b:** MS obsd. (ESI⁺) [(M+H)⁺]: 358.1. ¹H NMR (DMSO-*d₆*, 400MHz) δ: 10.60 (s, 1 H), 8.36 (d, *J* = 5.0 Hz, 1 H), 8.18 - 8.26 (m, 3 H), 7.87 (d, *J* = 8.1 Hz, 2 H), 7.50 (dd, *J* = 7.8, 5.0 Hz, 1 H), 3.39 - 3.52 (m, 2 H), 3.12 - 3.32 (m, 3 H), 2.44 - 2.48 (m, 1 H), 2.19 - 2.31 (m, 1 H).

### Example 87-a, Example 87-b

### (3R)-N-[4-(5-chloro-1,3-benzoxazol-2-yl)phenyl]tetrahydrofuran-3-carboxamide

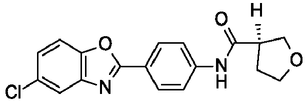

### (3S)-N-[4-(5-chloro-1,3-benzoxazol-2-yl)phenyl]tetrahydrofuran-3-carboxamide

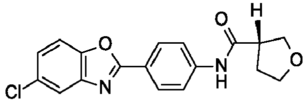

The two enantiomers **(Example 87-a, Example 87-b)** were obtained through SFC [Instrument: SFC 80, Column: ChiralCel OJ, 250×20mm I.D., 5µm; Mobile phase: A for CO₂ and B for Methanol (0.1% NH₄OH); Gradient: B 40%; Flow rate: 50 mL/min; Back pressure: 100 bar; Column temperature: 35 °C; elution order was **Example 87-a, Example 87-b]** chiral separation of *N*-[4-(5-chloro-1,3-benzoxazol-2-yl)phenyl]tetrahydrofuran-3-carboxamide **(Example 75).**
**Example 87-a:** MS obsd. (ESI⁺) [(M+H)⁺]: 343.2. ¹H NMR (DMSO-*d₆*, 400MHz) δ: 10.43 (s, 1 H), 8.15 (d, *J* = 8.1 Hz, 2 H), 7.79 - 7.90 (m, 4 H), 7.45 (d, *J* = 8.7 Hz, 1 H), 3.96 (t, *J* = 8.3 Hz, 1 H), 3.70 - 3.83 (m, 3 H), 3.21 (quin, *J* = 7.5 Hz, 1 H), 2.07 - 2.17 (m, 2 H).
**Example 87-b:** MS obsd. (ESI⁺) [(M+H)⁺]: 343.2. ¹H NMR (DMSO-*d₆*, 400MHz) δ: 10.43 (s, 1 H), 8.15 (d, *J* = 8.1 Hz, 2 H), 7.79 - 7.90 (m, 4 H), 7.45 (d, *J* = 8.7 Hz, 1 H), 3.96 (t, *J* = 8.2 Hz, 1 H), 3.69 - 3.83 (m, 3 H), 3.16 - 3.25 (m, 1 H), 2.05 - 2.17 (m, 2 H).

### Example 88

### PHH Natural Infection Assay

Detailed procedures regarding primary human hepatocyte (PHH) HBV natural infection assay are described as below. One tube of frozen PHH (10 million cells) is thawed in 37 °C water bath and then transferred to 20 mL of PHH thawing medium (Sigma, InVitroGRO HT Medium, Cat. S03319) with gently mixing. The cells were then centrifuged at 80 g/min for 5 min, the supernatant was discarded and the tube was refilled with 25 mL of PHH plating medium (Sigma , InVitroGRO CP Medium, Cat. S03317). The tube was shaken very gently to re-suspend all cells, and then 50 µL of cells were transferred to each well 384-well collagen I coated plate with appropriate liquid handling equipment, e.g. Integra VIAFLO384 or Agilent Bravo. The cells were then cultured for 24 hours in a cell incubator. For HBV infection, after PHH attachment on the culture plate, the plating medium was removed and replenished with PHH culture medium containing HBV virus. The PHH culture medium was prepared with Dulbecco's Modified Eagle Medium (DMEM)/F12 (1: 1 in volume ratio) containing 10% fetal bovine serum (Gibco, Cat.10099141), 5 ng/mL human epidermal growth factor (Gibco, Cat.PHG0311L), 20 ng/mL dexamethasone (Sigma, Cat.D4902-100mg), 250 ng/mL human recombinant insulin (Gibco, Cat.41400045) and 100 U/mL penicillin. HBV virus at 200 genome equivalent (GE) per cell with 4% PEG8000 (Sigma, Cat.P1458) containing culture medium were added to the PHH culture medium for infection. The cells were then cultured for 24 hours in cell incubator. Then the cell culture supernatant was removed. The HBV-infected PHH were cultured with sandwich culture method with PHH culture medium containing 1% DMSO and 0.25 mg/mL matrix gel for 72 hours. The supernatant was then refreshed with PHH culture medium containing different concentrations of testing compounds for two times with 72-hour interval. At the end of treatment, the supernatant was collected for viral markers measurements, including HBsAg, HBeAg, HBV DNA and cytotoxicity. HBsAg and HBeAg were detected using alphalisa method using their specific antibodies. For HBV DNA detection, HBV DNA Quantitative Fluorescence Diagnostic Kit (Sansure Biotech Inc.) was used following the manufacture's protocol. Cytotoxicity was determined using Cell Counting Kit-8 (CCK8, Dojindo Molecular Technologies, Inc.).

The compounds of the present invention were tested for their capacity to inhibit HBsAg and HBeAg as described herein. The Examples were tested in the above assay and found to have IC₅₀ below 10 µM. Results of PHH assay are given in Table 1.

**Table 1: Activity data of compounds of this invention**

| Example No. | HBsAg IC₅₀ (µM) | HBeAg IC₅₀ (µM) | CC₅₀ (µM) |
|---|---|---|---|
| Example 1 | 0.77 | 0.70 | >100 |
| Example 2 | 1.14 | 1.29 | >100 |
| Example 3 | 1.23 | 1.63 | >100 |
| Example 4 | 1.08 | 0.64 | >100 |
| Example 5 | 1.45 | 1.46 | >100 |
| Example 6 | 1.61 | 5.55 | >100 |
| Example 7 | 3.86 | 5.18 | >100 |
| Example 8 | 0.92 | 0.82 | >100 |
| Example 9 | 1.19 | 1.46 | >100 |
| Example 10 | 6.46 | 4.38 | >100 |
| Example 11 | 2.06 | 5.62 | >100 |
| Example 12 | 0.48 | 0.63 | >100 |
| Example 13 | 0.26 | 0.19 | >100 |
| Example 14 | 0.33 | 0.40 | >100 |
| Example 15 | 0.99 | 1.07 | >100 |
| Example 16 | 0.48 | 0.44 | >100 |
| Example 17 | 0.54 | 0.63 | >100 |
| Example 18 | 0.92 | 0.96 | >100 |
| Example 19 | 0.68 | 0.98 | >100 |
| Example 20 | 0.70 | 0.69 | >100 |
| Example 21 | 0.72 | 0.85 | >100 |
| Example 22 | 0.96 | 1.23 | >100 |
| Example 23 | 0.65 | 0.52 | >100 |
| Example 24 | 0.94 | 1.80 | >100 |
| Example 25 | 0.95 | 0.72 | >100 |
| Example 26 | 1.08 | 0.84 | 73.5 |
| Example 27 | 1.10 | 0.71 | >100 |
| Example 28 | 0.96 | 1.00 | >100 |
| Example 29 | 1.38 | 1.45 | >100 |
| Example 30 | 1.49 | 1.47 | >100 |
| Example 31 | 1.50 | 2.29 | >100 |
| Example 32 | 1.52 | 1.16 | >100 |
| Example 33 | 1.58 | 1.09 | >100 |
| Example 34 | 1.61 | 1.76 | >100 |
| Example 35 | 1.67 | 1.85 | >100 |
| Example 36 | 2.74 | 2.61 | >100 |
| Example 37 | 3.43 | 4.30 | >100 |
| Example 38 | 3.74 | 3.94 | >100 |
| Example 39 | 6.29 | 9.01 | >100 |
| Example 40 | 0.17 | 0.33 | >100 |
| Example 41 | 0.42 | 0.52 | >100 |
| Example 42 | 0.46 | 0.52 | >100 |
| Example 43 | 1.09 | 1.07 | >100 |
| Example 44 | 1.33 | 1.28 | >100 |
| Example 45 | 1.36 | 1.35 | >100 |
| Example 46 | 2.23 | 4.53 | >100 |
| Example 47 | 1.02 | 1.20 | >100 |
| Example 48 | 0.76 | 0.97 | >100 |
| Example 49 | 0.62 | 0.53 | >100 |
| Example 50 | 1.20 | 1.51 | >100 |
| Example 51 | 1.16 | 1.23 | >100 |
| Example 52 | 1.72 | 1.78 | >100 |
| Example 53 | 1.43 | 1.17 | >100 |
| Example 54 | 2.68 | 2.83 | >100 |
| Example 55 | 2.96 | 2.80 | >100 |
| Example 56 | 3.39 | 4.30 | >100 |
| Example 57 | 8.19 | 9.08 | >100 |
| Example 58 | 5.96 | 8.77 | >100 |
| Example 59 | 1.64 | 1.57 | >100 |
| Example 60 | 5.04 | 3.00 | >100 |
| Example 61 | 1.50 | 1.55 | >100 |
| Example 62 | 1.61 | 3.29 | >100 |
| Example 63 | 2.53 | 4.83 | >100 |
| Example 64 | 2.76 | 3.56 | >100 |
| Example 65 | 2.73 | 2.20 | >100 |
| Example 66 | 3.90 | 6.11 | >100 |
| Example 67 | 3.08 | 3.30 | >100 |
| Example 68 | 4.51 | 4.22 | >100 |
| Example 69 | 1.49 | 2.10 | >100 |
| Example 70 | 1.75 | 2.46 | >100 |
| Example 71 | 9.29 | 17.5 | >100 |
| Example 72 | 2.22 | 2.61 | >100 |
| Example 73 | 1.78 | 2.05 | >100 |
| Example 74 | 4.10 | 5.44 | >100 |
| Example 75 | 1.10 | 1.34 | >100 |
| Example 76 | 1.59 | 2.11 | >100 |
| Example 77 | 2.85 | 2.54 | >100 |
| Example 78 | 0.81 | 1.11 | >100 |
| Example 79 | 0.82 | 1.03 | >100 |
| Example 80 | 1.34 | 2.11 | >100 |
| Example 81 | 1.43 | 2.06 | >100 |
| Example 82 | 0.52 | 0.55 | >100 |
| Example 83 | 1.79 | 2.54 | >100 |
| Example 84 | 1.40 | 2.01 | >100 |
| Example 85 | 2.68 | 1.72 | >100 |
| Example 86-a | 4.42 | 7.32 | >100 |
| Example 86-b | 1.95 | 1.87 | >100 |
| Example 87-a | 4.09 | 4.05 | >100 |
| Example 87-b | 3.96 | 3.80 | >100 |

## Claims

1. A compound of the formula (I), 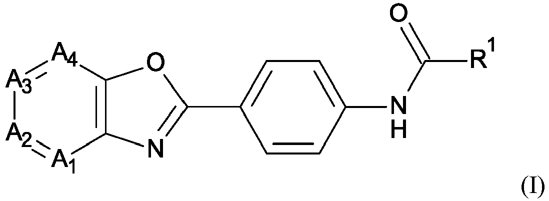 wherein
R¹ is tetrahydrofuran-3-yl, 1-hydroxyethyl, tert-butyl, tetrahydrothiopyran-4-yl, 2-methyltetrahydrofuran-2-yl, propyl, 1,1-dioxothiolan-3-yl, 3-furyl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothian-4-yl, cyclopropyl, 3-methyltetrahydrofuran-3-yl, 8-oxabicyclo[3.2.1]octan-3-yl, 1,4-dioxan-2-yl, cyclobutyl, 4-acetoxytetrahydropyran-4-yl, 2,2-difluorocyclopropyl, tetrahydropyran-4-yl, oxetan-3-yl, 1,1-dioxothietan-3-yl, 1-methylcyclopropyl, 3-oxabicyclo[3.1.0]hexan-6-yl, 1-methylcyclobutyl, 1-acetoxycyclopropyl, tetrahydrofuran-3-yloxy, 3-methoxycarbonyl-1-bicyclo[1.1.1]pentanyl, 3-ethyloxetan-3-yl, oxazol-2-yl, 5-methyloxazol-2-yl, 2-pyridyl, 4-methyloxazol-2-yl, thiazol-2-yl, 3-pyridyl, 3-methyloxetan-3-yl, 1-methyl-3-oxo-cyclobutyl, methoxy, ethoxy, isopropoxy, 1-methoxyethyl or 5-methyl-1,3,4-oxadiazol-2-yl;
A₁ is N or CR²; wherein R² is H or halogen; wherein with the proviso that when R¹ is 3-pyridyl, A₁ is CF;
A₂ is N or CR³; wherein R³ is H or halogen;
A₃ is N or CR⁴; wherein R⁴ is H, halogen, C₁₋₆alkyl, or C₁₋₆alkoxy;
A₄ is N or CR⁵; wherein R⁵ is H or halogen; wherein with the proviso that when R⁴ is C₁₋₆alkyl or C₁₋₆alkoxy, A₄ is N;
wherein with the proviso that A₁, A₂, A₃ and A₄ are not CH simultaneously;
or a pharmaceutically acceptable salt thereof.

2. A compound according to claim 1, wherein
R¹ is tetrahydrofuran-3-yl, 1-hydroxyethyl, tert-butyl, tetrahydrothiopyran-4-yl, 2-methyltetrahydrofuran-2-yl, propyl, 1,1-dioxothiolan-3-yl, 3-furyl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothian-4-yl, cyclopropyl, 3-methyltetrahydrofuran-3-yl, 8-oxabicyclo[3.2.1]octan-3-yl, 1,4-dioxan-2-yl, cyclobutyl, 4-acetoxytetrahydropyran-4-yl, 2,2-difluorocyclopropyl, tetrahydropyran-4-yl, oxetan-3-yl, 1,1-dioxothietan-3-yl, 1-methylcyclopropyl, 3-oxabicyclo[3.1.0]hexan-6-yl, 1-methylcyclobutyl, 1-acetoxycyclopropyl, tetrahydrofuran-3-yloxy, 3-methoxycarbonyl-1-bicyclo[1.1.1]pentanyl, 3-ethyloxetan-3-yl, oxazol-2-yl, 5-methyloxazol-2-yl, 2-pyridyl, 4-methyloxazol-2-yl, thiazol-2-yl, 3-pyridyl, 3-methyloxetan-3-yl, 1-methyl-3-oxo-cyclobutyl, methoxy, ethoxy, isopropoxy, 1-methoxyethyl or 5-methyl-1,3,4-oxadiazol-2-yl;
A₁ is CR²; wherein R² is H or F; wherein with the proviso that when R¹ is 3-pyridyl, A₁ is CF;
A₂ is CR³; wherein R³ is H, F or Cl;
A₃ is CR⁴; wherein R⁴ is H, F, Cl, methyl or methoxy;
A₄ is N or CR⁵; wherein R⁵ is H, F or Cl; wherein with the proviso that when R⁴ is methyl or methoxy, A₄ is N;
wherein with the proviso that A₁, A₂, A₃ and A₄ are not CH simultaneously;
or a pharmaceutically acceptable salt thereof.

3. A compound according to claim 1 or 2, or a pharmaceutically acceptable salt thereof, wherein A₂ is CH.

4. A compound according to claim 1 or 3, or a pharmaceutically acceptable salt thereof, wherein A₃ is CR⁴; wherein R⁴ is H or halogen.

5. A compound according to claim 4, or a pharmaceutically acceptable salt thereof, wherein A₃ is CR⁴; wherein R⁴ is H, F or Cl.

6. A compound according to any one of claims 1, 3 and 4, or a pharmaceutically acceptable salt thereof, wherein A₄ is CR⁵; wherein R⁵ is H or halogen.

7. A compound according to any one of claims 6, or a pharmaceutically acceptable salt thereof, wherein A₄ is CR⁵; wherein R⁵ is H or F.

8. A compound according to any one of claims 1, 3, 4 and 6, or a pharmaceutically acceptable salt thereof, wherein R¹ is tetrahydrofuran-3-yl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothiolan-3-yl, 1,1-dioxothian-4-yl, tert-butyl, cyclopropyl, 3-methyltetrahydrofuran-3-yl, 8-oxabicyclo[3.2.1]octan-3-yl, 1,4-dioxan-2-yl, 1-hydroxyethyl, cyclobutyl, tetrahydropyran-4-yl, oxazol-2-yl, 5-methyloxazol-2-yl, 2-pyridyl or 4-methyloxazol-2-yl.

9. A compound according to claim 1, wherein
R¹ is tetrahydrofuran-3-yl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothiolan-3-yl, 1,1-dioxothian-4-yl, tert-butyl, cyclopropyl, 3-methyltetrahydrofuran-3-yl, 8-oxabicyclo[3.2.1]octan-3-yl, 1,4-dioxan-2-yl, 1-hydroxyethyl, cyclobutyl, tetrahydropyran-4-yl, oxazol-2-yl, 5-methyloxazol-2-yl, 2-pyridyl or 4-methyloxazol-2-yl;
A₁ is CR²; wherein R² is H or halogen;
A₂ is CH;
A₃ is CR⁴; wherein R⁴ is H or halogen;
A₄ is CR⁵; wherein R⁵ is H or halogen;
wherein with the proviso that A₁, A₂, A₃ and A₄ are not CH simultaneously;
or a pharmaceutically acceptable salt thereof.

10. A compound according to claim 9, wherein
R¹ is tetrahydrofuran-3-yl, 3-methyloxetan-3-yl, 1-hydroxy-1-methyl-ethyl, 1-bicyclo[1.1.1]pentanyl, 3-fluoro-1-bicyclo[1.1.1]pentanyl, 1,1-dioxothiolan-3-yl, 1,1-dioxothian-4-yl, tert-butyl, cyclopropyl, 3-methyltetrahydrofuran-3-yl, 8-oxabicyclo[3.2.1]octan-3-yl, 1,4-dioxan-2-yl, 1-hydroxyethyl, cyclobutyl, tetrahydropyran-4-yl, oxazol-2-yl, 5-methyloxazol-2-yl, 2-pyridyl or 4-methyloxazol-2-yl;
A₁ is CH or CF;
A₂ is CH;
A₃ is CH, CF or CCl;
A₄ is CH or CF;
wherein with the proviso that A₁, A₂, A₃ and A₄ are not CH simultaneously;
or a pharmaceutically acceptable salt thereof.

11. A compound according to any one of claims 1 to 2, selected from
N-[4-(6-fluoro-1,3-benzoxazol-2-yl)phenyl]tetrahydrofuran-3-carboxamide;
N-[4-(6-fluoro-1,3-benzoxazol-2-yl)phenyl]-2-hydroxy-propanamide;
N-[4-(6-fluoro-1,3-benzoxazol-2-yl)phenyl]-2,2-dimethyl-propanamide;
N-[4-(6-fluoro-1,3-benzoxazol-2-yl)phenyl]tetrahydrothiopyran-4-carboxamide;
N-[4-(6-fluoro-1,3-benzoxazol-2-yl)phenyl]-2-methyl-tetrahydrofuran-2-carboxamide;
N-[4-(5-fluoro-1,3-benzoxazol-2-yl)phenyl]-2,2-dimethyl-propanamide;
N-[4-(5-fluoro-1,3-benzoxazol-2-yl)phenyl]tetrahydrothiopyran-4-carboxamide;
N-[4-(6-chloro-1,3-benzoxazol-2-yl)phenyl]tetrahydrofuran-3-carboxamide;
N-[4-(6-chloro-1,3-benzoxazol-2-yl)phenyl]butanamide;
N-[4-(6-chloro-1,3-benzoxazol-2-yl)phenyl]-1,1-dioxo-thiolane-3-carboxamide;
N-[4-(7-fluoro-1,3-benzoxazol-2-yl)phenyl]furan-3-carboxamide;
N-[4-(7-fluoro-1,3-benzoxazol-2-yl)phenyl]-3-methyl-oxetane-3-carboxamide;
N-[4-(7-fluoro-1,3-benzoxazol-2-yl)phenyl]-2-hydroxy-2-methyl-propanamide;
N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]tetrahydrofuran-3-carboxamide;
3-fluoro-N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]-1,1-dioxo-thiolane-3-carboxamide;
N-[4-(7-fluoro-1,3-benzoxazol-2-yl)phenyl]-1,1-dioxo-thiane-4-carboxamide;
N-[4-(7-fluoro-1,3-benzoxazol-2-yl)phenyl]-2,2-dimethyl-propanamide;
N-[4-(7-fluoro-1,3-benzoxazol-2-yl)phenyl]cyclopropanecarboxamide;
N-[4-(7-fluoro-1,3-benzoxazol-2-yl)phenyl]-3-methyl-tetrahydrofuran-3-carboxamide;
N-[4-(7-fluoro-1,3-benzoxazol-2-yl)phenyl]-8-oxabicyclo[3.2.1]octane-3-carboxamide;
N-[4-(7-fluoro-1,3-benzoxazol-2-yl)phenyl]-1,4-dioxane-2-carboxamide;
N-[4-(7-fluoro-1,3-benzoxazol-2-yl)phenyl]-2-hydroxy-propanamide;
N-[4-(7-fluoro-1,3-benzoxazol-2-yl)phenyl]cyclobutanecarboxamide;
[4-[[4-(7-fluoro-1,3-benzoxazol-2-yl)phenyl]carbamoyl]tetrahydropyran-4-yl] acetate;
2,2-difluoro-N-[4-(7-fluoro-1,3-benzoxazol-2-yl)phenyl]cyclopropanecarboxamide;
N-[4-(7-fluoro-1,3-benzoxazol-2-yl)phenyl]tetrahydropyran-4-carboxamide;
N-[4-(7-fluoro-1,3-benzoxazol-2-yl)phenyl]oxetane-3-carboxamide;
N-[4-(7-fluoro-1,3-benzoxazol-2-yl)phenyl]tetrahydrothiopyran-4-carboxamide;
N-[4-(7-fluoro-1,3-benzoxazol-2-yl)phenyl]-1,1-dioxo-thietane-3-carboxamide;
N-[4-(7-fluoro-1,3-benzoxazol-2-yl)phenyl]-1-methyl-cyclopropanecarboxamide;
N-[4-(7-fluoro-1,3-benzoxazol-2-yl)phenyl]-3-oxabicyclo[3.1.0]hexane-6-carboxamide;
N-[4-(7-fluoro-1,3-benzoxazol-2-yl)phenyl]-1-methyl-cyclobutanecarboxamide;
N-[4-(7-fluoro-1,3-benzoxazol-2-yl)phenyl]-2-methyl-tetrahydrofuran-2-carboxamide;
[1-[[4-(7-fluoro-1,3-benzoxazol-2-yl)phenyl]carbamoyl]cyclopropyl] acetate;
tetrahydrofuran-3-yl N-[4-(7-fluoro-1,3-benzoxazol-2-yl)phenyl]carbamate;
methyl 3-[[4-(7-fluoro-1,3-benzoxazol-2-yl)phenyl]carbamoyl]bicyclo[1.1.1]pentane-1-carboxylate;
3-ethyl-N-[4-(7-fluoro-1,3-benzoxazol-2-yl)phenyl]oxetane-3-carboxamide;
N-[4-(4-fluoro-1,3-benzoxazol-2-yl)phenyl]oxazole-2-carboxamide;
N-[4-(4-fluoro-1,3-benzoxazol-2-yl)phenyl]-5-methyl-oxazole-2-carboxamide;
N-[4-(4-fluoro-1,3-benzoxazol-2-yl)phenyl]pyridine-2-carboxamide;
N-[4-(4-fluoro-1,3-benzoxazol-2-yl)phenyl]-4-methyl-oxazole-2-carboxamide;
N-[4-(4-fluoro-1,3-benzoxazol-2-yl)phenyl]thiazole-2-carboxamide;
N-[4-(4-fluoro-1,3-benzoxazol-2-yl)phenyl]-2-methyl-oxazole-5-carboxamide;
N-[4-(4-fluoro-1,3-benzoxazol-2-yl)phenyl]pyridine-3-carboxamide;
N-[4-(4-fluoro-1,3-benzoxazol-2-yl)phenyl]bicyclo[1.1.1]pentane-1-carboxamide;
3-fluoro-N-[4-(4-fluoro-1,3-benzoxazol-2-yl)phenyl]bicyclo[1.1.1]pentane-1-carboxamide;
N-[4-(4-fluoro-1,3-benzoxazol-2-yl)phenyl]-2-hydroxy-2-methyl-propanamide;
N-[4-(4-fluoro-1,3-benzoxazol-2-yl)phenyl]-1,1-dioxo-thiolane-3-carboxamide;
N-[4-(4-fluoro-1,3-benzoxazol-2-yl)phenyl]-3-methyl-oxetane-3-carboxamide;
N-[4-(4-fluoro-1,3-benzoxazol-2-yl)phenyl]-2-hydroxy-propanamide;
N-[4-(4-fluoro-1,3-benzoxazol-2-yl)phenyl]-2,2-dimethyl-propanamide;
N-[4-(4-fluoro-1,3-benzoxazol-2-yl)phenyl]tetrahydrofuran-3-carboxamide;
N-[4-(4-fluoro-1,3-benzoxazol-2-yl)phenyl]-1,1-dioxo-thiane-4-carboxamide;
N-[4-(4-fluoro-1,3-benzoxazol-2-yl)phenyl]-1-methyl-3-oxo-cyclobutanecarboxamide;
N-[4-(4-fluoro-1,3-benzoxazol-2-yl)phenyl]-1,1-dioxo-thietane-3-carboxamide;
methyl N-[4-(4-fluoro-1,3-benzoxazol-2-yl)phenyl]carbamate;
ethyl N-[4-(4-fluoro-1,3-benzoxazol-2-yl)phenyl]carbamate;
isopropyl N-[4-(4-fluoro-1,3-benzoxazol-2-yl)phenyl]carbamate;
2,2-dimethyl-N-(4-oxazolo[5,4-b]pyridin-2-ylphenyl)propanamide;
2-methoxy-N-(4-oxazolo[5,4-b]pyridin-2-ylphenyl)propanamide;
N-(4-oxazolo[5,4-b]pyridin-2-ylphenyl)-1,1-dioxo-thiolane-3-carboxamide;
3-methyl-N-(4-oxazolo[5,4-b]pyridin-2-ylphenyl)tetrahydrofuran-3-carboxamide;
3-methyl-N-(4-oxazolo[5,4-b]pyridin-2-ylphenyl)oxetane-3-carboxamide;
N-(4-oxazolo[5,4-b]pyridin-2-ylphenyl)-1,1-dioxo-thiane-4-carboxamide;
N-(4-oxazolo[5,4-b]pyridin-2-ylphenyl)tetrahydrofuran-3-carboxamide;
N-(4-oxazolo[5,4-b]pyridin-2-ylphenyl)oxetane-3-carboxamide;
N-[4-(4,5-difluoro-1,3-benzoxazol-2-yl)phenyl]tetrahydrofuran-3-carboxamide;
N-[4-(5,7-difluoro-1,3-benzoxazol-2-yl)phenyl]tetrahydrofuran-3-carboxamide;
N-[4-(5-methyloxazolo[5,4-b]pyridin-2-yl)phenyl]tetrahydrofuran-3-carboxamide;
N-[4-(5-methoxyoxazolo[5,4-b]pyridin-2-yl)phenyl]tetrahydrofuran-3-carboxamide;
N-[4-(5-chloro-1,3-benzoxazol-2-yl)phenyl]oxazole-2-carboxamide;
N-[4-(5-chloro-1,3-benzoxazol-2-yl)phenyl]pyridine-2-carboxamide;
N-[4-(5-chloro-1,3-benzoxazol-2-yl)phenyl]tetrahydrofuran-3-carboxamide;
N-[4-(5-chloro-1,3-benzoxazol-2-yl)phenyl]bicyclo[1.1.1]pentane-1-carboxamide;
N-[4-(5-chloro-1,3-benzoxazol-2-yl)phenyl]-2-hydroxy-propanamide;
N-[4-(6,7-difluoro-1,3-benzoxazol-2-yl)phenyl]-5-methyl-oxazole-2-carboxamide;
N-[4-(6,7-difluoro-1,3-benzoxazol-2-yl)phenyl]-4-methyl-oxazole-2-carboxamide;
N-[4-(6,7-difluoro-1,3-benzoxazol-2-yl)phenyl]-5-methyl-1,3,4-oxadiazole-2-carboxamide;
N-[4-(6,7-difluoro-1,3-benzoxazol-2-yl)phenyl]-2-methyl-oxazole-5-carboxamide;
N-[4-(6,7-difluoro-1,3-benzoxazol-2-yl)phenyl]tetrahydrofuran-3-carboxamide;
N-[4-(6,7-difluoro-1,3 -benzoxazol-2-yl)phenyl]-1,1 -dioxo-thiolane-3 -carboxamide;
N-[4-(4,6-difluoro-1,3-benzoxazol-2-yl)phenyl]tetrahydrofuran-3-carboxamide;
N-[4-(7-chloro-1,3-benzoxazol-2-yl)phenyl]-1,1-dioxo-thiolane-3-carboxamide;
(3R)-N-(4-oxazolo[5,4-b]pyridin-2-ylphenyl)-1,1-dioxo-thiolane-3-carboxamide;
(3 S)-N-(4-oxazolo[5,4-b]pyridin-2-ylphenyl)-1,1-dioxo-thiolane-3-carboxamide;
(3R)-N-[4-(5-chloro-1,3-benzoxazol-2-yl)phenyl]tetrahydrofuran-3-carboxamide; and
(3S)-N-[4-(5-chloro-1,3-benzoxazol-2-yl)phenyl]tetrahydrofuran-3-carboxamide;
or a pharmaceutically acceptable salt thereof.

12. A compound according to any one of claims 1 to 10, selected from
N-[4-(6-fluoro-1,3-benzoxazol-2-yl)phenyl]tetrahydrofuran-3-carboxamide;
N-[4-(6-chloro-1,3-benzoxazol-2-yl)phenyl]tetrahydrofuran-3-carboxamide;
N-[4-(7-fluoro-1,3-benzoxazol-2-yl)phenyl]-3-methyl-oxetane-3-carboxamide;
N-[4-(7-fluoro-1,3-benzoxazol-2-yl)phenyl]-2-hydroxy-2-methyl-propanamide;
N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]tetrahydrofuran-3-carboxamide;
3-fluoro-N-[4-(7-fluoro-1,3-benzoxazol-2-yl)phenyl]bicyclo[1.1.1]pentane-1-carboxamide;
N-[4-(7-fluoro-1,3-benzoxazol-2-yl)phenyl]-1,1-dioxo-thiolane-3-carboxamide;
N-[4-(7-fluoro-1,3-benzoxazol-2-yl)phenyl]-1,1-dioxo-thiane-4-carboxamide;
N-[4-(7-fluoro-1,3-benzoxazol-2-yl)phenyl]-2,2-dimethyl-propanamide;
N-[4-(7-fluoro-1,3-benzoxazol-2-yl)phenyl]cyclopropanecarboxamide;
N-[4-(7-fluoro-1,3-benzoxazol-2-yl)phenyl]-3-methyl-tetrahydrofuran-3-carboxamide;
N-[4-(7-fluoro-1,3-benzoxazol-2-yl)phenyl]-8-oxabicyclo[3.2.1]octane-3-carboxamide;
N-[4-(7-fluoro-1,3-benzoxazol-2-yl)phenyl]-1,4-dioxane-2-carboxamide;
N-[4-(7-fluoro-1,3-benzoxazol-2-yl)phenyl]-2-hydroxy-propanamide;
N-[4-(7-fluoro-1,3-benzoxazol-2-yl)phenyl]cyclobutanecarboxamide;
N-[4-(7-fluoro-1,3-benzoxazol-2-yl)phenyl]tetrahydropyran-4-carboxamide;
N-[4-(4-fluoro-1,3-benzoxazol-2-yl)phenyl]oxazole-2-carboxamide;
N-[4-(4-fluoro-1,3-benzoxazol-2-yl)phenyl]-5-methyl-oxazole-2-carboxamide;
N-[4-(4-fluoro-1,3-benzoxazol-2-yl)phenyl]pyridine-2-carboxamide;
3-fluoro-N-[4-(4-fluoro-1,3-benzoxazol-2-yl)phenyl]bicyclo[1.1.1]pentane-1-carboxamide;
N-[4-(4-fluoro-1,3-benzoxazol-2-yl)phenyl]-2-hydroxy-2-methyl-propanamide;
N-[4-(6,7-difluoro-1,3-benzoxazol-2-yl)phenyl]-5-methyl-oxazole-2-carboxamide;
N-[4-(6,7-difluoro-1,3-benzoxazol-2-yl)phenyl]-4-methyl-oxazole-2-carboxamide; and
N-[4-(6,7-difluoro-1,3-benzoxazol-2-yl)phenyl]tetrahydrofuran-3-carboxamide;
or a pharmaceutically acceptable salt thereof.

13. A process for the preparation of a compound according to any one of claims 1 to 12 comprising at least one of the following steps,
(a) reaction of a compound of formula (IX), 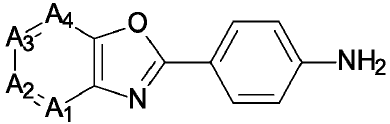 (IX), with a compound of formula (X), 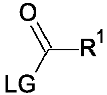 (X), in the presence of a base and optionally in the presence of a coupling reagent;
(b) reaction of a compound of formula (IX) with a compound of formula (X) by heating; wherein LG is OH or halogen.

14. A compound according to any one of claims 1 to 12 for use as therapeutically active substance.

15. A pharmaceutical composition comprising a compound in accordance with any one of claims 1 to 12 and a therapeutically inert carrier.

16. A compound according to any one of claims 1 to 12 for use in the treatment or prophylaxis of HBV infection.

## Patentansprüche

1. Verbindung der Formel (I) 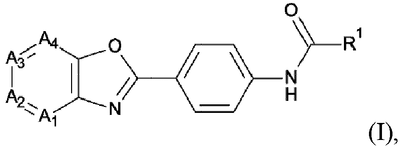 wobei
R¹ Tetrahydrofuran-3-yl, 1-Hydroxyethyl, tert-Butyl, Tetrahydrothiopyran-4-yl, 2-Methyltetrahydrofuran-2-yl, Propyl, 1,1-Dioxothiolan-3-yl, 3-Furyl, 3-Methyloxetan-3-yl, 1-Hydroxy-1-methylethyl, 1-Bicyclo[1.1.1]pentanyl, 3-Fluor-1-bicyclo[1.1.1]pentanyl, 1,1-Dioxothian-4-yl, Cyclopropyl, 3-Methyltetrahydrofuran-3-yl, 8-Oxabicyclo[3.2.1]octan-3-yl, 1,4-Dioxan-2-yl, Cyclobutyl, 4-Acetoxytetrahydropyran-4-yl, 2,2-Difluorcyclopropyl, Tetrahydropyran-4-yl, Oxetan-3-yl, 1,1-Dioxothietan-3-yl, 1-Methylcyclopropyl, 3-Oxabicyclo[3.1.0]hexan-6-yl, 1-Methylcyclobutyl, 1-Acetoxycyclopropyl, Tetrahydrofuran-3-yloxy, 3-Methoxycarbonyl-1-bicyclo[1.1.1]pentanyl, 3-Ethyloxetan-3-yl, Oxazol-2-yl, 5-Methyloxazol-2-yl, 2-Pyridyl, 4-Methyloxazol-2-yl, Thiazol-2-yl, 3-Pyridyl, 3-Methyloxetan-3-yl, 1-Methyl-3-oxocyclobutyl, Methoxy, Ethoxy, Isopropoxy, 1-Methoxyethyl oder 5-Methyl-1,3,4-oxadiazol-2-yl ist;
A₁ N oder CR² ist; wobei R² H oder Halogen ist; mit der Maßgabe, dass, wenn R¹ 3-Pyridyl ist, A₁ CF ist;
A₂ N oder CR³ ist; wobei R³ H oder Halogen ist;
A₃ N oder CR⁴ ist; wobei R⁴ H, Halogen, C₁₋₆-Alkyl oder C₁₋₆-Alkoxy ist;
A₄ N oder CR⁵ ist; wobei R⁵ H oder Halogen ist; mit der Maßgabe, dass, wenn R⁴ C₁₋₆-Alkyl oder C₁₋₆-Alkoxy ist, A₄ N ist;
mit der Maßgabe, dass A₁, A₂, A₃ und A₄ nicht gleichzeitig CH sind;
oder ein pharmazeutisch unbedenkliches Salz davon.

2. Verbindung nach Anspruch 1, wobei
R¹ Tetrahydrofuran-3-yl, 1-Hydroxyethyl, tert-Butyl, Tetrahydrothiopyran-4-yl, 2-Methyltetrahydrofuran-2-yl, Propyl, 1,1-Dioxothiolan-3-yl, 3-Furyl, 3-Methyloxetan-3-yl, 1-Hydroxy-1-methylethyl, 1-Bicyclo[1.1.1]pentanyl, 3-Fluor-1-bicyclo[1.1.1]pentanyl, 1,1-Dioxothian-4-yl, Cyclopropyl, 3-Methyltetrahydrofuran-3-yl, 8-Oxabicyclo[3.2.1]octan-3-yl, 1,4-Dioxan-2-yl, Cyclobutyl, 4-Acetoxytetrahydropyran-4-yl, 2,2-Difluorcyclopropyl, Tetrahydropyran-4-yl, Oxetan-3-yl, 1,1-Dioxothietan-3-yl, 1-Methylcyclopropyl, 3-Oxabicyclo[3.1.0]hexan-6-yl, 1-Methylcyclobutyl, 1-Acetoxycyclopropyl, Tetrahydrofuran-3-yloxy, 3-Methoxycarbonyl-1-bicyclo[1.1.1]pentanyl, 3-Ethyloxetan-3-yl, Oxazol-2-yl, 5-Methyloxazol-2-yl, 2-Pyridyl, 4-Methyloxazol-2-yl, Thiazol-2-yl, 3-Pyridyl, 3-Methyloxetan-3-yl, 1-Methyl-3-oxocyclobutyl, Methoxy, Ethoxy, Isopropoxy, 1-Methoxyethyl oder 5-Methyl-1,3,4-oxadiazol-2-yl ist;
A₁ CR² ist; wobei R² H oder F ist; mit der Maßgabe, dass, wenn R¹ 3-Pyridyl ist, A₁ CF ist;
A₂ CR³ ist; wobei R³ H, F oder Cl ist;
A₃ CR⁴ ist; wobei R⁴ H, F, Cl, Methyl oder Methoxy ist;
A₄ N oder CR⁵ ist; wobei R⁵ H, F oder Cl ist; mit der Maßgabe, dass, wenn R⁴ Methyl oder Methoxy ist, A₄ N ist;
mit der Maßgabe, dass A₁, A₂, A₃ und A₄ nicht gleichzeitig CH sind;
oder ein pharmazeutisch unbedenkliches Salz davon.

3. Verbindung nach Anspruch 1 oder 2 oder ein pharmazeutisch unbedenkliches Salz davon, wobei A₂ CH ist.

4. Verbindung nach Anspruch 1 oder 3 oder ein pharmazeutisch unbedenkliches Salz davon, wobei A₃ CR⁴ ist; wobei R⁴ H oder Halogen ist.

5. Verbindung nach Anspruch 4 oder ein pharmazeutisch unbedenkliches Salz davon, wobei A₃ CR⁴ ist; wobei R⁴ H, F oder Cl ist.

6. Verbindung nach einem der Ansprüche 1, 3 und 4 oder ein pharmazeutisch unbedenkliches Salz davon, wobei A₄ CR⁵ ist; wobei R⁵ H oder Halogen ist.

7. Verbindung nach Anspruch 6 oder ein pharmazeutisch unbedenkliches Salz davon, wobei A₄ CR⁵ ist; wobei R⁵ H oder F ist.

8. Verbindung nach einem der Ansprüche 1, 3, 4 und 6 oder ein pharmazeutisch unbedenkliches Salz davon, wobei R¹ Tetrahydrofuran-3-yl, 3-Methyloxetan-3-yl, 1-Hydroxy-1-methylethyl, 1-Bicyclo[1.1.1]pentanyl, 3-Fluor-1-bicyclo[1.1.1]pentanyl, 1,1-Dioxothiolan-3-yl, 1,1-Dioxothian-4-yl, tert-Butyl, Cyclopropyl, 3-Methyltetrahydrofuran-3-yl, 8-Oxabicyclo[3.2.1]octan-3-yl, 1,4-Dioxan-2-yl, 1-Hydroxyethyl, Cyclobutyl, Tetrahydropyran-4-yl, Oxazol-2-yl, 5-Methyloxazol-2-yl, 2-Pyridyl oder 4-Methyloxazol-2-yl ist.

9. Verbindung nach Anspruch 1, wobei
R¹ Tetrahydrofuran-3-yl, 3-Methyloxetan-3-yl, 1-Hydroxy-1-methylethyl, 1-Bicyclo[1.1.1]pentanyl, 3-Fluor-1-bicyclo[1.1.1]pentanyl, 1,1-Dioxothiolan-3-yl, 1,1-Dioxothian-4-yl, tert-Butyl, Cyclopropyl, 3-Methyltetrahydrofuran-3-yl, 8-Oxabicyclo[3.2.1]octan-3-yl, 1,4-Dioxan-2-yl, 1-Hydroxyethyl, Cyclobutyl, Tetrahydropyran-4-yl, Oxazol-2-yl, 5-Methyloxazol-2-yl, 2-Pyridyl oder 4-Methyloxazol-2-yl ist;
A₁ CR² ist; wobei R² H oder Halogen ist;
A₂ CH ist;
A₃ CR⁴ ist; wobei R⁴ H oder Halogen ist;
A₄ CR⁵ ist; wobei R⁵ H oder Halogen ist;
mit der Maßgabe, dass A₁, A₂, A₃ und A₄ nicht gleichzeitig CH sind;
oder ein pharmazeutisch unbedenkliches Salz davon.

10. Verbindung nach Anspruch 9, wobei
R¹ Tetrahydrofuran-3-yl, 3-Methyloxetan-3-yl, 1-Hydroxy-1-methylethyl, 1-Bicyclo[1.1.1]pentanyl, 3-Fluor-1-bicyclo[1.1.1]pentanyl, 1,1-Dioxothiolan-3-yl, 1,1-Dioxothian-4-yl, tert-Butyl, Cyclopropyl, 3-Methyltetrahydrofuran-3-yl, 8-Oxabicyclo[3.2.1]octan-3-yl, 1,4-Dioxan-2-yl, 1-Hydroxyethyl, Cyclobutyl, Tetrahydropyran-4-yl, Oxazol-2-yl, 5-Methyloxazol-2-yl, 2-Pyridyl oder 4-Methyloxazol-2-yl ist;
A₁ CH oder CF ist;
A₂ CH ist;
A₃ CH, CF oder CCl ist;
A₄ CH oder CF ist;
mit der Maßgabe, dass A₁, A₂, A₃ und A₄ nicht gleichzeitig CH sind;
oder ein pharmazeutisch unbedenkliches Salz davon.

11. Verbindung nach einem der Ansprüche 1 bis 2, ausgewählt aus
N-[4-(6-Fluor-1,3-benzoxazol-2-yl)phenyl]tetrahydrofuran-3-carboxamid;
N-[4-(6-Fluor-1,3-benzoxazol-2-yl)phenyl]-2-hydroxypropanamid;
N-[4-(6-Fluor-1,3-benzoxazol-2-yl)phenyl]-2,2-dimethylpropanamid;
N-[4-(6-Fluor-1,3-benzoxazol-2-yl)phenyl]tetrahydrothiopyran-4-carboxamid;
N-[4-(6-Fluor-1,3-benzoxazol-2-yl)phenyl]-2-methyltetrahydrofuran-2-carboxamid;
N-[4-(5-Fluor-1,3-benzoxazol-2-yl)phenyl]-2,2-dimethylpropanamid;
N-[4-(5-Fluor-1,3-benzoxazol-2-yl)phenyl]tetrahydrothiopyran-4-carboxamid;
N-[4-(6-Chlor-1,3-benzoxazol-2-yl)phenyl]tetrahydrofuran-3-carboxamid;
N-[4-(6-Chlor-1,3-benzoxazol-2-yl)phenyl]butanamid;
N-[4-(6-Chlor-1,3-benzoxazol-2-yl)phenyl]-1,1-dioxothiolan-3-carboxamid;
N-[4-(7-Fluor-1,3-benzoxazol-2-yl)phenyl]furan-3-carboxamid;
N-[4-(7-Fluor-1,3-benzoxazol-2-yl)phenyl]-3-methyloxetan-3-carboxamid;
N-[4-(7-Fluor-1,3-benzoxazol-2-yl)phenyl]-2-hydroxy-2-methylpropanamid;
N-[4-(7-Fluor-1,3-benzoxazol-2-yl)phenyl]bicyclo[1.1.1]pentan-1-carboxamid;
N-[4-(7-Fluor-1,3-benzoxazol-2-yl)phenyl]tetrahydrofuran-3-carboxamid;
3-Fluor-N-[4-(7-fluor-1,3-benzoxazol-2-yl)phenyl]bicyclo[1.1.1]pentan-1-carboxamid;
N-[4-(7-Fluor-1,3-benzoxazol-2-yl)phenyl]-1,1-dioxothiolan-3-carboxamid;
N-[4-(7-Fluor-1,3-benzoxazol-2-yl)phenyl]-1,1-dioxothian-4-carboxamid;
N-[4-(7-Fluor-1,3-benzoxazol-2-yl)phenyl]-2,2-dimethylpropanamid;
N-[4-(7-Ffluor-1,3-benzoxazol-2-yl)phenyl]cyclopropancarboxamid,
N-[4-(7-Fluor-1,3-benzoxazol-2-yl)phenyl]-3-methyltetrahydrofuran-3-carboxamid;
N-[4-(7-Fluor-1,3-benzoxazol-2-yl)phenyl]-8-oxabicyclo[3.2.1]octan-3-carboxamid;
N-[4-(7-Fluor-1,3-benzoxazol-2-yl)phenyl]-1,4-dioxan-2-carboxamid;
N-[4-(7-Fluor-1,3-benzoxazol-2-yl)phenyl]-2-hydroxypropanamid;
N-[4-(7-Fluor-1,3-benzoxazol-2-yl)phenyl]cyclobutancarboxamid;
[4-[[4-(7-Fluor-1,3-benzoxazol-2-yl)phenyl]carbamoyl]tetrahydropyran-4-yl]acetat;
2,2-Difluor-N-[4-(7-fluor-1,3-benzoxazol-2-yl)phenyl]cyclopropancarboxamid,
N-[4-(7-Fluor-1,3-benzoxazol-2-yl)phenyl]tetrahydropyran-4-carboxamid;
N-[4-(7-Fluor-1,3-benzoxazol-2-yl)phenyl]oxetan-3-carboxamid;
N-[4-(7-Fluor-1,3-benzoxazol-2-yl)phenyl]tetrahydrothiopyran-4-carboxamid;
N-[4-(7-Fluor-1,3-benzoxazol-2-yl)phenyl]-1,1-dioxothietan-3-carboxamid;
N-[4-(7-Fluor-1,3-benzoxazol-2-yl)phenyl]-1-methylcyclopropancarboxamid;
N-[4-(7-Fluor-1,3-benzoxazol-2-yl)phenyl]-3-oxabicyclo[3.1.0]hexan-6-carboxamid;
N-[4-(7-Fluor-1,3-benzoxazol-2-yl)phenyl]-1-methylcyclobutancarboxamid;
N-[4-(7-Fluor-1,3-benzoxazol-2-yl)phenyl]-2-methyltetrahydrofuran-2-carboxamid;
[1-[[4-(7-fluor-1,3-benzoxazol-2-yl)phenyl]carbamoyl]cyclopropyl]acetat;
Tetrahydrofuran-3-yl-N-[4-(7-fluor-1,3-benzoxazol-2-yl)phenyl]carbamat;
Methyl-3-[[4-(7-fluor-1,3-benzoxazol-2-yl)phenyl]carbamoyl]bicyclo[1.1.1]pentan-1-carboxylat;
3-Ethyl-N-[4-(7-fluor-1,3-benzoxazol-2-yl)phenyl]oxetan-3-carboxamid;
N-[4-(4-Fluor-1,3-benzoxazol-2-yl)phenyl]oxazol-2-carboxamid;
N-[4-(4-Fluor-1,3-benzoxazol-2-yl)phenyl]-5-methyloxazol-2-carboxamid;
N-[4-(4-Fluor-1,3-benzoxazol-2-yl)phenyl]pyridin-2-carboxamid;
N-[4-(4-Fluor-1,3-benzoxazol-2-yl)phenyl]-4-methyloxazol-2-carboxamid;
N-[4-(4-Fluor-1,3-benzoxazol-2-yl)phenyl]thiazol-2-carboxamid;
N-[4-(4-Fluor-1,3-benzoxazol-2-yl)phenyl]-2-methyloxazol-5-carboxamid;
N-[4-(4-Fluor-1,3-benzoxazol-2-yl)phenyl]pyridin-3-carboxamid;
N-[4-(4-Fluor-1,3-benzoxazol-2-yl)phenyl]bicyclo[1.1.1]pentan-1 -carboxamid;
3-Fluor-N-[4-(4-fluor-1,3-benzoxazol-2-yl)phenyl]bicyclo[1.1.1]pentan-1-carboxamid;
N-[4-(4-Fluor-1,3-benzoxazol-2-yl)phenyl]-2-hydroxy-2-methylpropanamid;
N-[4-(4-Fluor-1,3-benzoxazol-2-yl)phenyl]-1,1-dioxothiolan-3-carboxamid;
N-[4-(4-Fluor-1,3-benzoxazol-2-yl)phenyl]-3-methyloxetan-3-carboxamid;
N-[4-(4-Fluor-1,3-benzoxazol-2-yl)phenyl]-2-hydroxypropanamid;
N-[4-(4-Fluor-1,3-benzoxazol-2-yl)phenyl]-2,2-dimethylpropanamid;
N-[4-(4-Fluor-1,3-benzoxazol-2-yl)phenyl]tetrahydrofuran-3-carboxamid;
N-[4-(4-Fluor-1,3-benzoxazol-2-yl)phenyl]-1,1-dioxothian-4-carboxamid;
N-[4-(4-Fluor-1,3-benzoxazol-2-yl)phenyl]-1-methyl-3-oxocyclobutancarboxamid;
N-[4-(4-Fluor-1,3-benzoxazol-2-yl)phenyl]-1,1-dioxothietan-3-carboxamid;
Methyl-N-[4-(4-fluor-1,3-benzoxazol-2-yl)phenyl]carbamat;
Ethyl-N-[4-(4-fluor-1,3-benzoxazol-2-yl)phenyl]carbamat;
Isopropyl-N-[4-(4-fluor-1,3-benzoxazol-2-yl)phenyl]carbamat;
2,2-Dimethyl-N-(4-oxazolo[5,4-b]pyridin-2-ylphenyl)propanamid;
2-Methoxy-N-(4-oxazolo[5,4-b]pyridin-2-ylphenyl)propanamid;
N-(4-Oxazolo[5,4-b]pyridin-2-ylphenyl)-1,1-dioxothiolan-3-carboxamid;
3-Methyl-N-(4-oxazolo[5,4-b]pyridin-2-ylphenyl)tetrahydrofuran-3-carboxamid;
3-Methyl-N-(4-oxazolo[5,4-b]pyridin-2-ylphenyl)oxetan-3-carboxamid;
N-(4-Oxazolo[5,4-b]pyridin-2-ylphenyl)-1,1-dioxothian-4-carboxamid;
N-(4-Oxazolo[5,4-b]pyridin-2-ylphenyl)tetrahydrofuran-3-carboxamid;
N-(4-Oxazolo[5,4-b]pyridin-2-ylphenyl)oxetan-3-carboxamid;
N-[4-(4,5-Difluor-1,3-benzoxazol-2-yl)phenyl]tetrahydrofuran-3-carboxamid;
N-[4-(5,7-Difluor-1,3-benzoxazol-2-yl)phenyl]tetrahydrofuran-3-carboxamid;
N-[4-(5-Methyloxazolo[5,4-b]pyridin-2-yl)phenyl]tetrahydrofuran-3-carboxamid;
N-[4-(5-Methoxyoxazolo[5,4-b]pyridin-2-yl)phenyl]tetrahydrofuran-3-carboxamid;
N-[4-(5-Chlor-1,3-benzoxazol-2-yl)phenyl]oxazol-2-carboxamid;
N-[4-(5-Chlor-1,3-benzoxazol-2-yl)phenyl]pyridin-2-carboxamid,
N-[4-(5-Chlor-1,3-benzoxazol-2-yl)phenyl]tetrahydrofuran-3-carboxamid,
N-[4-(5-Chlor-1,3-benzoxazol-2-yl)phenyl]bicyclo[1.1.1]pentan-1-carboxamid;
N-[4-(5-Chlor-1,3-benzoxazol-2-yl)phenyl]-2-hydroxypropanamid,
N-[4-(6,7-Difluor-1,3-benzoxazol-2-yl)phenyl]-5-methyloxazol-2-carboxamid;
N-[4-(6,7-Difluor-1,3-benzoxazol-2-yl)phenyl]-4-methyloxazol-2-carboxamid;
N-[4-(6,7-Difluor-1,3-benzoxazol-2-yl)phenyl]-5-methyl-1,3,4-oxadiazol-2-carboxamid;
N-[4-(6,7-Difluor-1,3-benzoxazol-2-yl)phenyl]-2-methyloxazol-5-carboxamid;
N-[4-(6,7-Difluor-1,3-benzoxazol-2-yl)phenyl]tetrahydrofuran-3-carboxamid;
N-[4-(6,7-Difluor-1,3-benzoxazol-2-yl)phenyl]-1,1-dioxothiolan-3-carboxamid,
N-[4-(4,6-Difluor-1,3-benzoxazol-2-yl)phenyl]tetrahydrofuran-3-carboxamid;
N-[4-(7-Chlor-1,3 -benzoxazol-2-yl)phenyl]-1,1 -dioxothiolan-3 -carboxamid;
(3R)-N-(4-Oxazolo[5,4-b]pyridin-2-ylphenyl)-1,1-dioxothiolan-3-carboxamid;
(3S)-N-(4-Oxazolo[5,4-b]pyridin-2-ylphenyl)-1,1-dioxothiolan-3-carboxamid;
(3R)-N-[4-(5-Chlor-1,3-benzoxazol-2-yl)phenyl]tetrahydrofuran-3-carboxamid; und
(3S)-N-[4-(5-Chlor-1,3-benzoxazol-2-yl)phenyl]tetrahydrofuran-3-carboxamid;
oder ein pharmazeutisch unbedenkliches Salz davon.

12. Verbindung nach einem der Ansprüche 1 bis 10, ausgewählt aus
N-[4-(6-Fluor-1,3-benzoxazol-2-yl)phenyl]tetrahydrofuran-3-carboxamid;
N-[4-(6-Chlor-1,3-benzoxazol-2-yl)phenyl]tetrahydrofuran-3-carboxamid;
N-[4-(7-Fluor-1,3-benzoxazol-2-yl)phenyl]-3-methyloxetan-3-carboxamid;
N-[4-(7-Fluor-1,3-benzoxazol-2-yl)phenyl]-2-hydroxy-2-methylpropanamid;
N-[4-(7-Fluor-1,3-benzoxazol-2-yl)phenyl]bicyclo[1.1.1]pentan-1-carboxamid;
N-[4-(7-Fluor-1,3-benzoxazol-2-yl)phenyl]tetrahydrofuran-3-carboxamid;
3-Fluor-N-[4-(7-fluor-1,3-benzoxazol-2-yl)phenyl]bicyclo[1.1.1]pentan-1-carboxamid;
N-[4-(7-Fluor-1,3-benzoxazol-2-yl)phenyl]-1,1-dioxothiolan-3-carboxamid;
N-[4-(7-Fluor-1,3-benzoxazol-2-yl)phenyl]-1,1-dioxothian-4-carboxamid;
N-[4-(7-Fluor-1,3-benzoxazol-2-yl)phenyl]-2,2-dimethylpropanamid;
N-[4-(7-Fluor-1,3-benzoxazol-2-yl)phenyl]cyclopropancarboxamid;
N-[4-(7-Fluor-1,3-benzoxazol-2-yl)phenyl]-3-methyltetrahydrofuran-3-carboxamid;
N-[4-(7-Fluor-1,3-benzoxazol-2-yl)phenyl]-8-oxabicyclo[3.2.1]octan-3-carboxamid;
N-[4-(7-Fluor-1,3-benzoxazol-2-yl)phenyl]-1,4-dioxan-2-carboxamid;
N-[4-(7-Fluor-1,3-benzoxazol-2-yl)phenyl]-2-hydroxypropanamid;
N-[4-(7-Fluor-1,3-benzoxazol-2-yl)phenyl]cyclobutancarboxamid;
N-[4-(7-Fluor-1,3-benzoxazol-2-yl)phenyl]tetrahydropyran-4-carboxamid;
N-[4-(4-Fluor-1,3-benzoxazol-2-yl)phenyl]oxazol-2-carboxamid;
N-[4-(4-Fluor-1,3-benzoxazol-2-yl)phenyl]-5-methyloxazol-2-carboxamid;
N-[4-(4-Fluor-1,3-benzoxazol-2-yl)phenyl]pyridin-2-carboxamid;
3-Fluor-N-[4-(4-fluor-1,3-benzoxazol-2-yl)phenyl]bicyclo[1.1.1]pentan-1-carboxamid;
N-[4-(4-Fluor-1,3-benzoxazol-2-yl)phenyl]-2-hydroxy-2-methylpropanamid;
N-[4-(6,7-Difluor-1,3-benzoxazol-2-yl)phenyl]-5-methyloxazol-2-carboxamid;
N-[4-(6,7-Difluor-1,3-benzoxazol-2-yl)phenyl]-4-methyloxazol-2-carboxamid; und
N-[4-(6,7-Difluor-1,3-benzoxazol-2-yl)phenyl]tetrahydrofuran-3-carboxamid;
oder ein pharmazeutisch unbedenkliches Salz davon.

13. Verfahren zur Herstellung einer Verbindung nach einem der Ansprüche 1 bis 12, umfassend mindestens einen der folgenden Schritte,
(a) Reaktion einer Verbindung der Formel (IX), 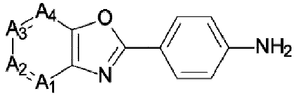 (IX), mit einer Verbindung der Formel (X), 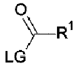 (X), in der Gegenwart einer Base und gegebenenfalls in der Gegenwart eines Kopplungsmittels;
(b) Reaktion einer Verbindung der Formel (IX) mit einer Verbindung der Formel (X) durch Erwärmen; wobei LG OH oder Halogen ist.

14. Verbindung nach einem der Ansprüche 1 bis 12 zur Verwendung als therapeutisch wirksame Substanz.

15. Pharmazeutische Zusammensetzung, umfassend eine Verbindung nach einem der Ansprüche 1 bis 12 und einen therapeutisch inerten Träger.

16. Verbindung nach einem der Ansprüche 1 bis 12 zur Verwendung bei der Behandlung oder Prophylaxe einer HBV-Infektion.

## Revendications

1. Composé de formule (I), 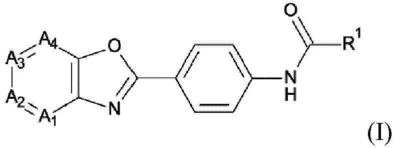 dans lequel
R¹ représente un tétrahydrofuran-3-yle, un 1-hydroxyéthyle, un tert-butyle, un tétrahydrothiopyran-4-yle, un 2-méthyltétrahydrofuran-2-yle, un propyle, un 1,1-dioxothiolan-3-yle, un 3-furyle, un 3-méthyloxétan-3-yle, un 1-hydroxy-1-méthyl-éthyle, un 1-bicyclo[1.1.1]pentanyle, un 3-fluoro-1-bicyclo[1.1.1]pentanyle, un 1,1-dioxothian-4-yle, un cyclopropyle, un 3-méthyltétrahydrofuran-3-yle, un 8-oxabicyclo[3.2.1]octan-3-yle, un 1,4-dioxan-2-yle, un cyclobutyle, un 4-acétoxytétrahydropyran-4-yle, un 2,2-difluorocyclopropyle, un tétrahydropyran-4-yle, un oxétan-3-yle, un 1,1-dioxothiétan-3-yle, un 1-méthylcyclopropyle, un 3-oxabicyclo[3.1.0]hexan-6-yle, un 1-méthylcyclobutyle, un 1-acétoxycyclopropyle, un tétrahydrofuran-3-yloxy, un 3-méthoxycarbonyl-1-bicyclo[1.1.1]pentanyle, un 3-éthyloxétan-3-yle, un oxazol-2-yle, un 5-méthyloxazol-2-yle, un 2-pyridyle, un 4-méthyloxazol-2-yle, un thiazol-2-yle, un 3-pyridyle, un 3-méthyloxétan-3-yle, un 1-méthyl-3-oxo-cyclobutyle, un méthoxy, un éthoxy, un isopropoxy, un 1-méthoxyéthyle ou un 5-méthyl-1,3,4-oxadiazol-2-yle ;
A₁ représente N ou CR² ; dans lequel R² représente H ou un halogène ; dans lequel à condition que lorsque R¹ représente un 3-pyridyle, A₁ représente CF ;
A₂ représente N ou CR³ ; dans lequel R³ représente H ou un halogène ;
A₃ représente N ou CR⁴ ; dans lequel R⁴ représente H, un halogène, un alkyle en C₁₋₆ ou un alcoxy en C₁₋₆ ;
A₄ représente N ou CR⁵ ; dans lequel R⁵ représente H ou un halogène ; dans lequel à condition que lorsque R⁴ représente un alkyle en C₁₋₆ ou un alcoxy en C₁₋₆, A₄ représente N ;
dans lequel à condition que A₁, A₂, A₃ et A₄ ne représentent pas CH simultanément ;
ou un sel pharmaceutiquement acceptable de celui-ci.

2. Composé selon la revendication 1, dans lequel
R¹ représente un tétrahydrofuran-3-yle, un 1-hydroxyéthyle, un tert-butyle, un tétrahydrothiopyran-4-yle, un 2-méthyltétrahydrofuran-2-yle, un propyle, un 1,1-dioxothiolan-3-yle, un 3-furyle, un 3-méthyloxétan-3-yle, un 1-hydroxy-1-méthyl-éthyle, un 1-bicyclo[1.1.1]pentanyle, un 3-fluoro-1-bicyclo[1.1.1]pentanyle, un 1,1-dioxothian-4-yle, un cyclopropyle, un 3-méthyltétrahydrofuran-3-yle, un 8-oxabicyclo[3.2.1]octan-3-yle, un 1,4-dioxan-2-yle, un cyclobutyle, un 4-acétoxytétrahydropyran-4-yle, un 2,2-difluorocyclopropyle, un tétrahydropyran-4-yle, un oxétan-3-yle, un 1,1-dioxothiétan-3-yle, un 1-méthylcyclopropyle, un 3-oxabicyclo[3.1.0]hexan-6-yle, un 1-méthylcyclobutyle, un 1-acétoxycyclopropyle, un tétrahydrofuran-3-yloxy, un 3-méthoxycarbonyl-1-bicyclo[1.1.1]pentanyle, un 3-éthyloxétan-3-yle, un oxazol-2-yle, un 5-méthyloxazol-2-yle, un 2-pyridyle, un 4-méthyloxazol-2-yle, un thiazol-2-yle, un 3-pyridyle, un 3-méthyloxétan-3-yle, un 1-méthyl-3-oxo-cyclobutyle, un méthoxy, un éthoxy, un isopropoxy, un 1-méthoxyéthyle ou un 5-méthyl-1,3,4-oxadiazol-2-yle ;
A₁ représente CR² ; dans lequel R² représente H ou F ; dans lequel à condition que lorsque R¹ représente un 3-pyridyle, A₁ représente CF ;
A₂ représente CR³ ; dans lequel R³ représente H, F ou Cl ;
A₃ représente CR⁴ ; dans lequel R⁴ représente H, F, Cl, un méthyle ou un méthoxy ;
A₄ représente N ou CR⁵ ; dans lequel R⁵ représente H, F ou Cl ; dans lequel à condition que lorsque R⁴ représente un méthyle ou un méthoxy, A₄ représente N ;
dans lequel à condition que A₁, A₂, A₃ et A₄ ne représentent pas CH simultanément ;
ou un sel pharmaceutiquement acceptable de celui-ci.

3. Composé selon la revendication 1 ou 2, ou un sel pharmaceutiquement acceptable de celui-ci, dans lequel A₂ représente CH.

4. Composé selon la revendication 1 ou 3, ou un sel pharmaceutiquement acceptable de celui-ci, dans lequel A₃ représente CR⁴ ; dans lequel R⁴ représente H ou un halogène.

5. Composé selon la revendication 4, ou un sel pharmaceutiquement acceptable de celui-ci, dans lequel A₃ représente CR⁴ ; dans lequel R⁴ représente H, F ou Cl.

6. Composé selon l'une quelconque des revendications 1, 3 et 4, ou un sel pharmaceutiquement acceptable de celui-ci, dans lequel A₄ représente CR⁵ ; dans lequel R⁵ représente H ou un halogène.

7. Composé selon l'une quelconque des revendications 6, ou un sel pharmaceutiquement acceptable de celui-ci, dans lequel A₄ représente CR⁵ ; dans lequel R⁵ représente H ou F.

8. Composé selon l'une quelconque des revendications 1, 3, 4 et 6, ou un sel pharmaceutiquement acceptable de celui-ci, dans lequel R¹ représente un tétrahydrofuran-3-yle, un 3-méthyloxétan-3-yle, un 1-hydroxy-1-méthyl-éthyle, un 1-bicyclo[1.1.1]pentanyle, un 3-fluoro-1-bicyclo[1.1.1]pentanyle, un 1,1-dioxothiolan-3-yle, un 1,1-dioxothian-4-yle, un tert-butyle, un cyclopropyle, un 3-méthyltétrahydrofuran-3-yle, un 8-oxabicyclo[3.2.1]octan-3-yle, un 1,4-dioxan-2-yle, un 1-hydroxyéthyle, un cyclobutyle, un tétrahydropyran-4-yle, un oxazol-2-yle, un 5-méthyloxazol-2-yle, un 2-pyridyle ou un 4-méthyloxazol-2-yle.

9. Composé selon la revendication 1, dans lequel
R¹ représente un tétrahydrofuran-3-yle, un 3-méthyloxétan-3-yle, un 1-hydroxy-1-méthyl-éthyle, un 1-bicyclo[1.1.1]pentanyle, un 3-fluoro-1-bicyclo[1.1.1]pentanyle, un 1,1-dioxothiolan-3-yle, un 1,1-dioxothian-4-yle, un tert-butyle, un cyclopropyle, un 3-méthyltétrahydrofuran-3-yle, un 8-oxabicyclo[3.2.1]octan-3-yle, un 1,4-dioxan-2-yle, un 1-hydroxyéthyle, un cyclobutyle, un tétrahydropyran-4-yle, un oxazol-2-yle, un 5-méthyloxazol-2-yle, un 2-pyridyle ou un 4-méthyloxazol-2-yle ;
A₁ représente CR² ; dans lequel R² représente H ou un halogène ;
A₂ représente CH ;
A₃ représente CR⁴ ; dans lequel R⁴ représente H ou un halogène ;
A₄ représente CR⁵ ; dans lequel R⁵ représente H ou un halogène ;
dans lequel à condition que A₁, A₂, A₃ et A₄ ne représentent pas CH simultanément ;
ou un sel pharmaceutiquement acceptable de celui-ci.

10. Composé selon la revendication 9, dans lequel
R¹ représente un tétrahydrofuran-3-yle, un 3-méthyloxétan-3-yle, un 1-hydroxy-1-méthyl-éthyle, un 1-bicyclo[1.1.1]pentanyle, un 3-fluoro-1-bicyclo[1.1.1]pentanyle, un 1,1-dioxothiolan-3-yle, un 1,1-dioxothian-4-yle, un tert-butyle, un cyclopropyle, un 3-méthyltétrahydrofuran-3-yle, un 8-oxabicyclo[3.2.1]octan-3-yle, un 1,4-dioxan-2-yle, un 1-hydroxyéthyle, un cyclobutyle, un tétrahydropyran-4-yle, un oxazol-2-yle, un 5-méthyloxazol-2-yle, un 2-pyridyle ou un 4-méthyloxazol-2-yle ;
A₁ représente CH ou CF ;
A₂ représente CH ;
A₃ représente CH, CF ou CCl ;
A₄ représente CH ou CF ;
dans lequel à condition que A₁, A₂, A₃ et A₄ ne représentent pas CH simultanément ;
ou un sel pharmaceutiquement acceptable de celui-ci.

11. Composé selon l'une quelconque des revendications 1 à 2, choisi parmi
le N-[4-(6-fluoro-1,3-benzoxazol-2-yl)phényl]tétrahydrofurane-3-carboxamide ;
le N-[4-(6-fluoro-1,3-benzoxazol-2-yl)phényl]-2-hydroxy-propanamide ;
le N-[4-(6-fluoro-1,3-benzoxazol-2-yl)phényl]-2,2-diméthyl-propanamide ;
le N-[4-(6-fluoro-1,3-benzoxazol-2-yl)phényl]tétrahydrothiopyrane-4-carboxamide ;
le N-[4-(6-fluoro-1,3-benzoxazol-2-yl)phényl]-2-méthyl-tétrahydrofurane-2-carboxamide ;
le N-[4-(5-fluoro-1,3-benzoxazol-2-yl)phényl]-2,2-diméthyl-propanamide ;
le N-[4-(5-fluoro-1,3-benzoxazol-2-yl)phényl]tétrahydrothiopyrane-4-carboxamide ;
le N-[4-(6-chloro-1,3-benzoxazol-2-yl)phényl]tétrahydrofurane-3-carboxamide ;
le N-[4-(6-chloro-1,3-benzoxazol-2-yl)phényl]butanamide ;
le N-[4-(6-chloro-1,3-benzoxazol-2-yl)phényl]-1,1-dioxo-thiolane-3-carboxamide ;
le N-[4-(7-fluoro-1,3-benzoxazol-2-yl)phényl]furane-3-carboxamide ;
le N-[4-(7-fluoro-1,3-benzoxazol-2-yl)phényl]-3-méthyl-oxétane-3-carboxamide ;
le N-[4-(7-fluoro-1,3-benzoxazol-2-yl)phényl]-2-hydroxy-2-méthyl-propanamide ;
le N-[4-(7-fluoro-1,3-benzoxazol-2-yl)phényl]bicyclo[1.1.1]pentane-1-carboxamide ;
le N-[4-(7-fluoro-1,3-benzoxazol-2-yl)phényl]tétrahydrofurane-3-carboxamide ;
le 3-fluoro-N-[4-(7-fluoro-1,3-benzoxazol-2-yl)phényl]bicyclo[1.1.1]pentane-1-carboxamide ;
le N-[4-(7-fluoro-1,3-benzoxazol-2-yl)phényl]-1,1-dioxo-thiolane-3-carboxamide ;
le N-[4-(7-fluoro-1,3-benzoxazol-2-yl)phényl]-1,1-dioxo-thiane-4-carboxamide ;
le N-[4-(7-fluoro-1,3-benzoxazol-2-yl)phényl]-2,2-diméthyl-propanamide ;
le N-[4-(7-fluoro-1,3-benzoxazol-2-yl)phényl]cyclopropanecarboxamide ;
le N-[4-(7-fluoro-1,3-benzoxazol-2-yl)phényl]-3-méthyl-tétrahydrofurane-3-carboxamide ;
le N-[4-(7-fluoro-1,3-benzoxazol-2-yl)phényl]-8-oxabicyclo[3.2.1]octane-3-carboxamide ;
le N-[4-(7-fluoro-1,3-benzoxazol-2-yl)phényl]-1,4-dioxane-2-carboxamide ;
le N-[4-(7-fluoro-1,3-benzoxazol-2-yl)phényl]-2-hydroxy-propanamide ;
le N-[4-(7-fluoro-1,3-benzoxazol-2-yl)phényl]cyclobutanecarboxamide ;
l'acétate de [4-[[4-(7-fluoro-1,3-benzoxazol-2-yl)phényl]carbamoyl]tétrahydropyran-4-yle] ;
le 2,2-difluoro-N-[4-(7-fluoro-1,3-benzoxazol-2-yl)phényl]cyclopropanecarboxamide ;
le N-[4-(7-fluoro-1,3-benzoxazol-2-yl)phényl]tétrahydropyrane-4-carboxamide ;
le N-[4-(7-fluoro-1,3-benzoxazol-2-yl)phényl]oxétane-3-carboxamide ;
le N-[4-(7-fluoro-1,3-benzoxazol-2-yl)phényl]tétrahydrothiopyrane-4-carboxamide ;
le N-[4-(7-fluoro-1,3-benzoxazol-2-yl)phényl]-1,1-dioxo-thiétane-3-carboxamide ;
le N-[4-(7-fluoro-1,3-benzoxazol-2-yl)phényl]-1-méthyl-cyclopropanecarboxamide ;
le N-[4-(7-fluoro-1,3-benzoxazol-2-yl)phényl]-3-oxabicyclo[3.1.0]hexane-6-carboxamide ;
le N-[4-(7-fluoro-1,3-benzoxazol-2-yl)phényl]-1-méthyl-cyclobutanecarboxamide ;
le N-[4-(7-fluoro-1,3-benzoxazol-2-yl)phényl]-2-méthyl-tétrahydrofurane-2-carboxamide ;
l'acétate de [1-[[4-(7-fluoro-1,3-benzoxazol-2-yl)phényl]carbamoyl]cyclopropyle] ;
le N-[4-(7-fluoro-1,3-benzoxazol-2-yl)phényl]carbamate de tétrahydrofuran-3-yle ;
le 3-[[4-(7-fluoro-1,3-benzoxazol-2-yl)phényl]carbamoyl]bicyclo[1.1.1]pentane-1-carboxylate de méthyle ;
le 3-éthyl-N-[4-(7-fluoro-1,3-benzoxazol-2-yl)phényl]oxétane-3-carboxamide ;
le N-[4-(4-fluoro-1,3-benzoxazol-2-yl)phényl]oxazole-2-carboxamide ;
le N-[4-(4-fluoro-1,3-benzoxazol-2-yl)phényl]-5-méthyl-oxazole-2-carboxamide ;
le N-[4-(4-fluoro-1,3-benzoxazol-2-yl)phényl]pyridine-2-carboxamide ;
le N-[4-(4-fluoro-1,3-benzoxazol-2-yl)phényl]-4-méthyl-oxazole-2-carboxamide ;
le N-[4-(4-fluoro-1,3-benzoxazol-2-yl)phényl]thiazole-2-carboxamide ;
le N-[4-(4-fluoro-1,3-benzoxazol-2-yl)phényl]-2-méthyl-oxazole-5-carboxamide ;
le N-[4-(4-fluoro-1,3-benzoxazol-2-yl)phényl]pyridine-3-carboxamide ;
le N-[4-(4-fluoro-1,3-benzoxazol-2-yl)phényl]bicyclo[1.1.1]pentane-1-carboxamide ;
le 3-fluoro-N-[4-(4-fluoro-1,3-benzoxazol-2-yl)phényl]bicyclo[1.1.1]pentane-1-carboxamide ;
le N-[4-(4-fluoro-1,3-benzoxazol-2-yl)phényl]-2-hydroxy-2-méthyl-propanamide ;
le N-[4-(4-fluoro-1,3-benzoxazol-2-yl)phényl]-1,1-dioxo-thiolane-3-carboxamide ;
le N-[4-(4-fluoro-1,3-benzoxazol-2-yl)phényl]-3-méthyl-oxétane-3-carboxamide ;
le N-[4-(4-fluoro-1,3-benzoxazol-2-yl)phényl]-2-hydroxy-propanamide ;
le N-[4-(4-fluoro-1,3-benzoxazol-2-yl)phényl]-2,2-diméthyl-propanamide ;
le N-[4-(4-fluoro-1,3-benzoxazol-2-yl)phényl]tétrahydrofurane-3-carboxamide ;
le N-[4-(4-fluoro-1,3-benzoxazol-2-yl)phényl]-1,1-dioxo-thiane-4-carboxamide ;
le N-[4-(4-fluoro-1,3-benzoxazol-2-yl)phényl]-1-méthyl-3-oxo-cyclobutanecarboxamide ;
le N-[4-(4-fluoro-1,3-benzoxazol-2-yl)phényl]-1,1-dioxo-thiétane-3-carboxamide ;
le N-[4-(4-fluoro-1,3-benzoxazol-2-yl)phényl]carbamate de méthyle ;
le N-[4-(4-fluoro-1,3-benzoxazol-2-yl)phényl]carbamate d'éthyle ;
le N-[4-(4-fluoro-1,3-benzoxazol-2-yl)phényl]carbamate d'isopropyle ;
le 2,2-diméthyl-N-(4-oxazolo[5,4-b]pyridin-2-ylphényl)propanamide ;
le 2-méthoxy-N-(4-oxazolo[5,4-b]pyridin-2-ylphényl)propanamide ;
le N-(4-oxazolo[5,4-b]pyridin-2-ylphényl)-1,1-dioxo-thiolane-3-carboxamide ;
le 3-méthyl-N-(4-oxazolo[5,4-b]pyridin-2-ylphényl)tétrahydrofurane-3-carboxamide ;
le 3-méthyl-N-(4-oxazolo[5,4-b]pyridin-2-ylphényl)oxétane-3-carboxamide ;
le N-(4-oxazolo[5,4-b]pyridin-2-ylphényl)-1,1-dioxo-thiane-4-carboxamide ;
le N-(4-oxazolo[5,4-b]pyridin-2-ylphényl)tétrahydrofurane-3-carboxamide ;
le N-(4-oxazolo[5,4-b]pyridin-2-ylphényl)oxétane-3-carboxamide ;
le N-[4-(4,5-difluoro-1,3-benzoxazol-2-yl)phényl]tétrahydrofurane-3-carboxamide ;
le N-[4-(5,7-difluoro-1,3-benzoxazol-2-yl)phényl]tétrahydrofurane-3-carboxamide ;
le N-[4-(5-méthyloxazolo[5,4-b]pyridin-2-yl)phényl]tétrahydrofurane-3-carboxamide ;
le N-[4-(5-méthoxyoxazolo[5,4-b]pyridin-2-yl)phényl]tétrahydrofurane-3-carboxamide ;
le N-[4-(5-chloro-1,3-benzoxazol-2-yl)phényl]oxazole-2-carboxamide ;
le N-[4-(5-chloro-1,3-benzoxazol-2-yl)phényl]pyridine-2-carboxamide ;
le N-[4-(5-chloro-1,3-benzoxazol-2-yl)phényl]tétrahydrofurane-3-carboxamide ;
le N-[4-(5-chloro-1,3-benzoxazol-2-yl)phényl]bicyclo[1.1.1]pentane-1-carboxamide ;
le N-[4-(5-chloro-1,3-benzoxazol-2-yl)phényl]-2-hydroxy-propanamide ;
le N-[4-(6,7-difluoro-1,3-benzoxazol-2-yl)phényl]-5-méthyl-oxazole-2-carboxamide ;
le N-[4-(6,7-difluoro-1,3-benzoxazol-2-yl)phényl]-4-méthyl-oxazole-2-carboxamide ;
le N-[4-(6,7-difluoro-1,3-benzoxazol-2-yl)phényl]-5-méthyl-1,3,4-oxadiazole-2-carboxamide ;
le N-[4-(6,7-difluoro-1,3-benzoxazol-2-yl)phényl]-2-méthyl-oxazole-5-carboxamide ;
le N-[4-(6,7-difluoro-1,3-benzoxazol-2-yl)phényl]tétrahydrofurane-3-carboxamide ;
le N-[4-(6,7-difluoro-1,3-benzoxazol-2-yl)phényl]-1,1-dioxo-thiolane-3-carboxamide ;
le N-[4-(4,6-difluoro-1,3-benzoxazol-2-yl)phényl]tétrahydrofurane-3-carboxamide ;
le N-[4-(7-chloro-1,3-benzoxazol-2-yl)phényl]-1,1-dioxo-thiolane-3-carboxamide ;
le (3R)-N-(4-oxazolo[5,4-b]pyridin-2-ylphényl)-1,1-dioxo-thiolane-3-carboxamide ;
le (3S)-N-(4-oxazolo[5,4-b]pyridin-2-ylphényl)-1,1-dioxo-thiolane-3-carboxamide ;
le (3R)-N-[4-(5-chloro-1,3-benzoxazol-2-yl)phényl]tétrahydrofurane-3-carboxamide ; et
le (3S)-N-[4-(5-chloro-1,3-benzoxazol-2-yl)phényl]tétrahydrofurane-3-carboxamide ;
ou un sel pharmaceutiquement acceptable de ceux-ci.

12. Composé selon l'une quelconque des revendications 1 à 10, choisi parmi
le N-[4-(6-fluoro-1,3-benzoxazol-2-yl)phényl]tétrahydrofurane-3-carboxamide ;
le N-[4-(6-chloro-1,3-benzoxazol-2-yl)phényl]tétrahydrofurane-3-carboxamide ;
le N-[4-(7-fluoro-1,3-benzoxazol-2-yl)phényl]-3-méthyl-oxétane-3-carboxamide ;
le N-[4-(7-fluoro-1,3-benzoxazol-2-yl)phényl]-2-hydroxy-2-méthyl-propanamide ;
le N-[4-(7-fluoro-1,3-benzoxazol-2-yl)phényl]bicyclo[1.1.1]pentane-1-carboxamide ;
le N-[4-(7-fluoro-1,3-benzoxazol-2-yl)phényl]tétrahydrofurane-3-carboxamide ;
le 3-fluoro-N-[4-(7-fluoro-1,3-benzoxazol-2-yl)phényl]bicyclo[1.1.1]pentane-1-carboxamide ;
le N-[4-(7-fluoro-1,3-benzoxazol-2-yl)phényl]-1,1-dioxo-thiolane-3-carboxamide ;
le N-[4-(7-fluoro-1,3-benzoxazol-2-yl)phényl]-1,1-dioxo-thiane-4-carboxamide ;
le N-[4-(7-fluoro-1,3-benzoxazol-2-yl)phényl]-2,2-diméthyl-propanamide ;
le N-[4-(7-fluoro-1,3-benzoxazol-2-yl)phényl]cyclopropanecarboxamide ;
le N-[4-(7-fluoro-1,3-benzoxazol-2-yl)phényl]-3-méthyl-tétrahydrofurane-3-carboxamide ;
le N-[4-(7-fluoro-1,3-benzoxazol-2-yl)phényl]-8-oxabicyclo[3.2.1]octane-3-carboxamide ;
le N-[4-(7-fluoro-1,3-benzoxazol-2-yl)phényl]-1,4-dioxane-2-carboxamide ;
le N-[4-(7-fluoro-1,3-benzoxazol-2-yl)phényl]-2-hydroxy-propanamide ;
le N-[4-(7-fluoro-1,3-benzoxazol-2-yl)phényl]cyclobutanecarboxamide ;
le N-[4-(7-fluoro-1,3-benzoxazol-2-yl)phényl]tétrahydropyrane-4-carboxamide ;
le N-[4-(4-fluoro-1,3-benzoxazol-2-yl)phényl]oxazole-2-carboxamide ;
le N-[4-(4-fluoro-1,3-benzoxazol-2-yl)phényl]-5-méthyl-oxazole-2-carboxamide ;
le N-[4-(4-fluoro-1,3-benzoxazol-2-yl)phényl]pyridine-2-carboxamide ;
le 3-fluoro-N-[4-(4-fluoro-1,3-benzoxazol-2-yl)phényl]bicyclo[1.1.1]pentane-1-carboxamide ;
le N-[4-(4-fluoro-1,3-benzoxazol-2-yl)phényl]-2-hydroxy-2-méthyl-propanamide ;
le N-[4-(6,7-difluoro-1,3-benzoxazol-2-yl)phényl]-5-méthyl-oxazole-2-carboxamide ;
le N-[4-(6,7-difluoro-1,3-benzoxazol-2-yl)phényl]-4-méthyl-oxazole-2-carboxamide ; et
le N-[4-(6,7-difluoro-1,3-benzoxazol-2-yl)phényl]tétrahydrofurane-3-carboxamide ;
ou un sel pharmaceutiquement acceptable de ceux-ci.

13. Procédé de préparation d'un composé selon l'une quelconque des revendications 1 à 12 comprenant au moins l'une des étapes suivantes,
(a) réaction d'un composé de formule (IX), 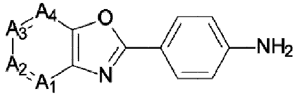 (IX) avec un composé de formule (X), 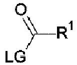 (X), en présence d'une base et éventuellement en présence d'un réactif de couplage ;
(b) réaction d'un composé de formule (IX) avec un composé de formule (X) par chauffage ; dans lequel LG représente OH ou un halogène.

14. Composé selon l'une quelconque des revendications 1 à 12 pour une utilisation comme substance thérapeutiquement active.

15. Composition pharmaceutique comprenant un composé selon l'une quelconque des revendications 1 à 12 et un véhicule thérapeutiquement inerte.

16. Composé selon l'une quelconque des revendications 1 à 12 pour une utilisation dans le traitement ou la prophylaxie d'une infection par le VHB.
